# EUROPEAN PATENT APPLICATION

(11) **EP 4 560 020 A1**
(43) Date of publication of application: **28.05.2025**
(21) Application number: 23383192.4
(22) Date of filing: 22.11.2023
(51) Int. Cl.: C12N 15/113, A61P 27/02

(54) **SIRNA AND COMPOSITIONS FOR PROPHYLACTIC AND THERAPEUTIC TREATMENT OF OCULAR RETINAL CONDITIONS**

(71) Applicant: Sylentis S.A.U., 28003 Madrid (ES)
(72) Inventor: JIMÉNEZ, Ana Isabel, E-28760 Tres Cantos, Madrid (ES); MARTíNEZ, Tamara, E-28760 Tres Cantos, Madrid (ES); RAMOS, Facundo Nehuén, E-28760 Tres Cantos, Madrid (ES)
(74) Representative: Marks & Clerk LLP

(57) **Abstract**

RNA interfering oligonucleotides targeting TSC2 are described for the treatment of retinal dystrophies. The RNAi oligonucleotides may be siRNAs, and may further comprise siRNAs targeting TSC1. Branched oligonucleotides comprising two or more siRNAs and product combinations of two or more different siRNAs are described, which in embodiments may target either or both of TSC1 and/or TSC2. The invention also relates to methods and compositions comprising such products.

## Description

### FIELD OF THE INVENTION

The present invention provides RNAi products, compositions and methods for modulating the expression of either or both of the genes encoding for TSC Complex Subunit 1 (TSC1) and TSC Complex Subunit 2 (TSC2), which can be useful in reducing the expression of these two genes and managing diseases related to reactive gliosis and the death and/or functional impairment of photoreceptor cells (rod and/or cones), including retinal dystrophies that involve, for example, retinitis pigmentosa, Leber's congenital amaurosis or Alström syndrome. In particular, this invention relates to RNAi oligonucleotide products, for example small interfering RNA (siRNA) compounds or molecules, branched oligonucleotides comprising two or more siRNA molecules and product combinations of two or more different siRNA compounds, which in embodiments hybridize with nucleic acid molecules encoding either or both of TSC1 and/or TSC2, two proteins which seem to be involved in the cellular mechanisms of cell survival and cell death. Such oligonucleotide products are shown herein to reduce the expression of either or both of TSC1 and/or TSC2 in cells, which can be useful for treating retinal dystrophy, but also for preventing or ameliorating the death of retinal cells and photoreceptors. The invention also relates to methods and compositions comprising such products.

### BACKGROUND OF THE INVENTION

Retinal dystrophies (RD) are a group of degenerative disorders of the retina with clinical and genetic heterogeneity which are estimated to affect as many as 1 in 4,000 individuals. Vision impairment may vary from poor peripheral or night vision to complete blindness, and severity usually increases with age (Nash et al. 2015).

Multiple causative gene defects have been identified, and to date, more than 270 genes are known to be associated with different phenotypes of retinal dystrophies. Most of these dystrophies affect children and young adults of the working-age group, adding to the socioeconomic burden. There are a number of different retinal dystrophies, which may appear only in the eye or may be associated with syndromes affecting several organs. Some of the main ones include achromatopsia, Leber congenital amaurosis (LCA), choroideremia, cone dystrophy, cone-rod dystrophy, Best disease, Stargardt disease, retinitis pigmentosa (RP), retinoschisis, Alström Syndrome, Stickler Syndrome, Usher Syndrome, and Familial exudative vitreoretinopathy.

Rod and cone photoreceptors are the main cellular units responsible for visual phototransduction, a process by which light signals are converted into action potential within the retina and facilitate the brain's perception of an image. RD occurs due to abnormalities in photoreceptors as well as defects in phototransduction.

Depending on the type of photoreceptors affected, retinal dystrophies can be subdivided into rod-dominated diseases, cone-dominated diseases, and generalized forms, which involve both rods and cones. Cases may be syndromic or non-syndromic, sporadic, or familial. In familial cases, inheritance patterns could be autosomal dominant, autosomal recessive or X linked. Syndromic cases have clinical features that extend to multisystemic involvement rather than non-syndromic varieties, which affect only the retina (Chawla and Vohra, 2023).

Rod dominated dystrophies include rod and rod-cone dystrophies, where either rod photoreceptors are predominantly affected, or rod photoreceptors are the first affected. This includes Retinitis pigmentosa (RP) and Congenital stational night blindness (CSNB).

Retinitis pigmentosa is the most seen retinal dystrophy, and causes severe vision impairment due to the progressive degeneration of photoreceptor cells in the retina. RP is a progressive rod-cone disease with rods affected first and has a high level of clinical and genetic heterogeneity. The age of presentation and the prognosis depend on the type of inheritance. Like other forms of RD, it may be sporadic or inherited in an autosomal dominant (AD), autosomal recessive (AR), or X- linked recessive (XLR) pattern. AD is the most common form, and XLR is the least common but most severe form. Non-syndromic and syndromic forms are reported. Nyctalopia is a constant feature, although it is not pathognomic of RP. Most cases do not report night vision problems until the ocular disease is in an advanced stage. Patients also notice an insidious progressive loss of peripheral field of vision. In most cases, the inferior retina is affected first. Hence superior field losses are commonly seen. In typical RP, the rate of progression of visual field loss is usually slow, and usually the patient does not notice these changes until it reaches the stage of tunnel vision when the patient becomes acutely aware of the changes. Central vision can be affected earlier due to secondary changes such as cystoid macular edema, epiretinal membrane, or development of retinal pigment epithelial defects (RPE) in macular or fovea. Color vision remains unaffected until the later stages of the disease in most cases. Fundus appearance of RP classically includes a triad of retinal vessel attenuation, waxy pallor of the optic disc, and bone spicule intraretinal pigmentation. Patients with very early RP without fundus pigmentary abnormalities are termed as RP sine pigmento. This is no longer considered as a subtype of RP as it is a stage of RP through which some patients pass. Fine dust-like pigment cells are noted in the vitreous cavity; these are released from the degeneration of RPE. Phenotypic variants, based on retinal involvement, are sectoral retinitis pigmentosa, pericentral RP, and unilateral or extremely asymmetric RP. Sectoral RP is characterized by pigmentary changes limited to one or two quadrants with limited visual field changes, good electroretinogram (ERG) responses, and minimal progression with time. True sector RP can be autosomal dominant or recessive. Sporadic cases are, however, common and may possibly result from non-genetic causes of retinal degeneration. The pericentral variant shows field loss between 5 and 15 degrees from fixation. The areas of field deficit enlarge and coalesce over time and encroach the central field of vision early, causing greater disability. Unilateral RP is typically an acquired condition and is commonly referred to as diffuse unilateral neuroretinitis. However, extremely asymmetric RP is an entity with a genetic association.

Congenital Stationary Night Blindness (CSNB) is a non-progressive form of night blindness. Various inheritance patterns (autosomal dominant, autosomal recessive, or X-linked) are now recognized. CSNB is further categorized as CSNB with normal fundus and abnormal fundus. Some cases of CSNB with normal fundi present with reduced vision (20/50) and no history of night blindness. CSNB with abnormal fundi includes two entities: Oguchi disease and fundus albipunctatus. In Oguchi disease, the Mizuo Nakamura phenomenon is classically observed. In this variant, golden sheen over the retina is noted with an unusually dark macula on exposure to light. However, the retina appears normal after prolonged dark adaptation. Color vision and visual acuity are normal in these cases. Histopathological studies suggest the presence of an abnormal layer between outer segments of photoreceptors and RPE. Fundus albipunctatus is another type of CSNB with yellow-white dots over the fundus. In most cases, these dots are found incidentally on routine eye checkups. Color vision and visual acuity are usually normal.

Cone dominated diseases can be further subdivided into diseases with early-onset with no progression and late-onset forms that are usually progressive. The progressive forms include cone dominated dystrophies and cone dystrophies. The stationary forms include achromatopsia and blue cone monochromatism.

Achromatopsia is an autosomal recessive condition with patients presenting with poor visual acuity since birth, photosensitivity, and poor color discrimination. Photosensitivity is due to poor visual acuity in bright lights, instead of actual light intolerance. It has two subtypes: complete and incomplete achromatopsia. Cases with complete achromatopsia, also known as rod monochromats, usually have visual acuity less than 20/200. Incomplete or atypical forms retain a visual acuity between 20/80- 20/200. Color vision is completely absent in complete achromatopsia cases. Pendular nystagmus may be present but usually improves with age. Fundus examination is usually normal in these cases; however, some cases may have a granular appearance of the macula or temporal optic disc pallor. Visual field testing may reveal central scotoma; however, peripheral fields are usually normal or mildly constricted. Characteristically, these are non-progressive changes.

Cone Monochromatism is an X linked recessive congenital disorder where two of the three cone systems are absent or significantly affected. The most common variety is blue cone monochromatism, in which both red and green cone systems are completely absent. Visual acuity in affected individuals ranges from 20/80 to 20/200. Clinical signs and symptoms resemble achromatopsia cases. They can perceive blue color.

Cone rod dystrophy (CRD) is usually misdiagnosed as RP with more involvement of cones than rods. Patients present with early loss of vision and color vision abnormalities with subsequent peripheral field constriction. The fundus examination reveals macular pigmentation and atrophy in the early stages, followed by peripheral bone spicule pigmentation in advanced cases. Often mid periphery is affected later in the course of the disease. The diagnosis of CRD is essentially based on ERG changes, which show cone affliction more than rods.

Generalized retinal dystrophies include disorders like Leber congenital amaurosis. Leber congenital amaurosis (LCA) is a group of disorders due to a mutation in at least 16 different genes, all presenting with severe visual impairment or blindness from infancy and extinguished ERG. Most patients show either a normal fundus appearance or subtle RPE changes and retinal vascular attenuation. Eye rubbing, also known as the oculo-digital sign, is a common association. Keratoconus is seen in 29% of cases. This association is not just a consequence of eye rubbing but may be due to other genetic factors. Complicated cases have systemic features. Cases with deafness, renal anomalies, hepatic dysfunction, or skeletal abnormalities have been reported. Mental retardation is reported in around 20% of cases. The most common inheritance pattern is autosomal recessive. Patients with RPE65 or CRB1 gene mutations have progressive vision loss with age, with the prognosis being slightly better in those with onset after infancy.

For most RD cases, although there is no cure, treatments are available for managing some aspects of its clinical manifestations. However, advances in stem cell and genome editing technologies are the catalysing factors in the development of gene-based therapies and treatment options in RD. Various treatment strategies investigating the applications of gene-based technologies, cell based therapies and retinal implant or transplantation are actively being sought in the RDs. Indeed, preclinical studies and phase I/II/III gene therapy trials are ongoing for several RD subtypes, and a first retinal gene therapy has been already approved by the US Food and Drug Administration (FDA) and the European Medicine Agency (EMA) for RPE65-associated RDs: Luxturna^{®} voretigene neparvovec-rzyl (Talib and Boon, 2020).

But the diversity of clinical features and gene mutations makes it difficult to develop effective treatments for retinal dystrophies. Finding therapies that would preserve photoreceptors and/or visual function in people with retinitis pigmentosa or any other retinal dystrophy regardless of their specific genetic mutation would help many more patients.

This first approved gene therapy Luxturna was designated an 'orphan medicine' (a medicine used in rare diseases) for two forms of the disease, retinitis pigmentosa and Leber's congenital amaurosis, and is useful for patients with an inherited retinal dystrophy caused by a mutation in the RPE65 gene. Because of this mutation, the retina doesn't respond properly to light. A single injection of Luxturna delivers a healthy copy of the RPE65 gene directly to the retina. This restores the retina's ability to respond to light. If patients receive the treatment early enough after diagnosis, Luxturna can improve night vision and help patients better navigate in low-light conditions.

Several new treatments on the horizon, which are still being tested, aim to benefit people with retinitis pigmentosa. However, it will likely be several years before they become available to patients. Further, some of them are based on gene therapies intended for restoring the normal function of an impaired or mutated gene related to the retinal disease, meaning that genetic diagnosis may be required before treatment.

There is a current need for effective treatments that can be useful to ameliorate or manage the signs and symptoms of such retinal dystrophies in patients, and finding therapies that would preserve photoreceptors and/or visual function in people with retinitis pigmentosa or any other retinal dystrophy regardless of their specific genetic mutation would help many more patients.

Promoting anabolic pathways in rods has been proposed as a therapeutic approach to improve the photoreceptor resistance to degeneration in a non-gene-specific manner. This can be achieved for example by activation or upregulation of a key regulator of anabolism such as mTOR (mechanistic target of rapamycin).

The aim of obtaining a neuroprotective effect in the photoreceptor survival could be also approached by regulation of autophagy. Many studies indicate that autophagy has a neuroprotective effect in the photoreceptor survival, although this role remains a topic of debate since some authors have proposed that its inhibition may be beneficial under certain conditions. Nevertheless, proper autophagy in retinal dystrophies can be considered essential in general terms to maintain vision. Indeed, deficient autophagy seems to affect both the structure and function of rods, while its overactivation can be counterproductive in certain cases, exacerbating photoreceptor degeneration despite the fact autophagy is essential for the normal recycling of visual cycle proteins such as rhodopsin and transducin. In addition, autophagy seems to be also necessary to maintain photopic vision, since in most models in which rod autophagy is specifically altered, cone neurodegeneration is also observed (Villarejo-Zori et al. 2021).

TSC2 and TSC1 proteins are known inhibitors of the mTOR signalling pathway. TSC1/2 protein complex plays a major role in controlling mTOR, which exists in two distinct complexes: mTORC1 and mTORC2. The TSC1/2 complex negatively regulates the mammalian target of rapamycin complex 1 (mTORC1), a master regulator of protein synthesis and cell growth but which also acts as an inhibitor of autophagy (Di Nardo et al. 2014).

Although upregulating the mTOR pathway can be seen as an starting point for the development of new therapeutic agents for preventing photoreceptor degeneration in retinal degenerative diseases, such as retinitis pigmentosa and age related macular degeneration (AMD), as far as we know, no treatment is being currently developed based on the local administration to the eye of particular siRNA molecules targeting either or both of the genes TSC2 (tuberous sclerosis complex 2) or TSC1 (tuberous sclerosis complex 1).

Therefore, inhibiting or reducing the mRNA expression levels of both TSC1 and TSC2 genes, or the expression and/or activity of their related encoded proteins hamartin (TSC1) and tuberin (TSC2), could be a useful approach for managing the prevention ortreatment of retinal dystrophies orto prevent or ameliorate their associated signs and symptoms, and finding compounds with an appropriate profile for this and good therapeutic performance is needed.

Among the technologies available to address these types of molecular targets, a good alternative to TSC1 and TSC2 inhibitor drugs for the treatment of such diseases are RNA interference (RNAi) based drugs. Since RNAi therapies target RNA, these are very advantageous over conventional small molecule drugs used for therapy. This may require the identification and provision of optimized and efficacious RNAi oligonucleotides and siRNAs against both or either of these two genes which are able to produce a high silencing effect, in particular in ocular cells. However, hamartin and tuberin interact within the cell to form the TSC protein complex together with TBC1D7, and changes in the expression of one of these TSC genes may have some impact on the expression profile of the other TSC gene. Besides, co-transfection of two or more siRNAs may reduce the efficacy of any individual siRNA.

RNAi is a naturally occurring post-transcriptional regulatory mechanism present in most eukaryotic cells that uses small double stranded RNA (dsRNA) molecules to direct homology-dependent gene silencing. Its discovery by Fire and Mello in the worm *C*. *elegans* (Fire et al 1998) was awarded the Nobel Prize in 2006. Shortly after its first description, RNAi was also shown to occur in mammalian cells by means of double-stranded small interfering RNAs (siRNAs) 21 nucleotides long (Elbashir et al 2001).

The process of RNA interference is thought to be an evolutionarily conserved cellular defence mechanism used to prevent the expression of foreign genes and is commonly shared by diverse phyla and flora, where it is called post-transcriptional gene silencing. Since the discovery of the RNAi mechanism there has been an explosion of research to uncover new compounds that can selectively alter gene expression as a new way to treat human disease by addressing targets that are otherwise "undruggable" with traditional pharmaceutical approaches involving small molecules or proteins.

According to current knowledge, the mechanism of RNAi is initiated when long double stranded RNAs are processed by an RNase III-like protein known as Dicer. The protein Dicer typically contains an N-terminal RNA helicase domain, an RNA-binding so-called Piwi/Argonaute/Zwille (PAZ) domain, two RNase III domains and a double-stranded RNA binding domain (dsRBD) (Collins et al 2005) and its activity leads to the processing of the long double stranded RNAs into 21-24 nucleotide double stranded siRNAs with 2 base 3' overhangs and a 5' phosphate and 3' hydroxyl group. The resulting siRNA duplexes are then incorporated into the effector complex known as RNA-induced silencing complex (RISC), where the antisense or guide strand of the siRNA guides RISC to recognize and cleave target mRNA sequences (Elbashir et al 2001) upon adenosine-triphosphate (ATP)-dependent unwinding of the double-stranded siRNA molecule through an RNA helicase activity (Nykanen et al 2001). The catalytic activity of RISC, which leads to mRNA degradation, is mediated by the endonuclease Argonaute 2 (AGO2) (Liu et al 2004; Song et al 2004). AGO2 belongs to the highly conserved Argonaute family of proteins. Argonaute proteins are ^{~}100 KDa highly basic proteins that contain two common domains, namely PIWI and PAZ domains (Cerutti et al 2000). The PIWI domain is crucial for the interaction with Dicer and contains the nuclease activity responsible for the cleavage of mRNAs. AGO2 uses one strand of the siRNA duplex as a guide to find messenger RNAs containing complementary sequences and cleaves the phosphodiester backbone between bases 10 and 11 relative to the guide strand's 5' end (Elbashir et al 2001). An important step during the activation of RISC is the cleavage of the sense or passenger strand by AGO2, removing this strand from the complex (Rand et al 2005). Crystallography studies analyzing the interaction between the siRNA guide strand and the PIWI domain reveal that it is only nucleotides 2 to 8 that constitute a "seed sequence" that directs target mRNA recognition by RISC, and that a mismatch of a single nucleotide in this sequence may drastically affect silencing capability of the molecule (Ma et al 2005; Doench et al 2004; Lewis et al 2003). Once the mRNA has been cleaved, due to the presence of unprotected RNA ends in the fragments the mRNA is further cleaved and degraded by intracellular nucleases and will no longer be translated into proteins (Orban et al 2005) while RISC will be recycled for subsequent rounds (Hutvagner et al 2002). This constitutes a catalytic process leading to the selective reduction of specific mRNA molecules and the corresponding proteins. It is possible to exploit this native mechanism for gene silencing with the purpose of regulating any gene(s) of choice by directly delivering siRNA effectors into the cells or tissues, where they will activate RISC and produce a potent and specific silencing of the targeted mRNA. RNAi has been applied in biomedical research such as treatment for HIV, viral hepatitis, cardiovascular and cerebrovascular diseases, metabolic disease, neurodegenerative disorders, and cancer (Angaji SA et al 2010) and it has also emerged as a viable therapy with several RNAi based therapies being approved in recent years (Godinho and Khvorova, 2019). In 2018, the first formulation-based RNAi therapeutic, Patisiran (Onpattro^{™}), was approved by the Food and Drug Administration (FDA) and the European Medicines Agency (EMA), and approximately a year later, another breakthrough was achieved with the approval of Givosiran (GIVLAARI^{™}), the first siRNA using the conjugate-mediated approach for targeted delivery to hepatocytes. Later, three additional therapies based on siRNA conjugates for hepatic delivery were authorised, and currently, five siRNA therapies are being commercialized.

Many studies have been published describing the ideal features a siRNA should have to achieve maximum effectiveness, regarding length, structure, chemical composition, and sequence. Initial parameters for siRNA design were set out by Tuschl and co-workers in WO02/44321, although many subsequent studies, algorithms and/or improvements have been published since then. siRNA selection approaches have become more sophisticated as mechanistic details have emerged; in addition, further analysis of existing and new data can provide additional insights into further refinement of these approaches (Walton SP et al 2010). Alternatively, several studies reported the design and analysis of novel RNAi-triggering structures distinct from the classical 19+2 siRNA structure and which do not conform to the key features of classical siRNA in terms of overhang, length, or symmetry, discussing the flexibility of the RNAi machinery in mammalian cells (Chang CI et al 2011).

Also, a lot of efforts have been put into enhancing siRNA stability as this is perceived as one of the potential obstacles for therapy based on siRNA, given the ubiquitous nature of RNAses in biological fluids. Another inherent problem of siRNA molecules is their immunogenicity, whereby siRNAs have been found to induce unspecific activation of the innate immune system. The knockdown of unintended genes (mRNAs) is a well-known side effect of siRNA-mediated gene silencing. It is caused because of partial complementarity between the siRNA and mRNAs other than the intended target and causes off-target effects (OTEs) from genes having sequence complementarity to either siRNA strand. One of the main strategies followed for stability enhancement and OTE reduction has been the use of modified nucleotides such as 2'-O-methyl nucleotides, 2'-amino nucleotides, or nucleotides containing 2'-O or 4'-C methylene bridges. Also, the modification of the ribonucleotide backbone connecting adjacent nucleotides has been described, mainly by the introduction of phosphorothioate modified nucleotides. It seems that enhanced stability and/or reduction of immunogenicity are often inversely proportional to efficacy (Parrish, 2000), and only a certain number, positions and/or combinations of modified nucleotides may result in a stable and non-immunogenic silencing compound. As this is an important hurdle for siRNA-based treatments, different studies have been published which describe certain modification patterns showing good results; examples of such include EP1527176, WO2008/050329, WO2008/104978 or WO2009/044392, although many more may be found in the literature (Sanghvi YS. 2011; Deleavey et al 2012).

Other important hurdles for RNAi therapies include the requirement for the siRNA drugs to be delivered to the intended targeted cells and tissues, and to be internalized into the specific cells, where the compound further will need to be released from the endosomal compartment. These problems are not yet completely resolved. Indeed, many efforts have been also made to find optimal delivery strategies for in vivo applications of RNAi drugs, including formulation and conjugation, two approaches widely used and demonstrated to be useful in the clinic, as demonstrated by the five RNAi therapeutic products currently approved for use. Formulations can be employed with or without modifications to the siRNA molecule itself, whereas the conjugation approach relies heavily on chemical modifications to protect the oligonucleotide from nuclease attack. An overview of the strategies for delivery with formulation and conjugation of RNAi compounds, together with some examples of materials used as non-viral vectors, nanocarriers or in formulated nanosystems, as well as some ligands used for conjugation can be found in Godinho and Khvorova 2019 and in references included therein. Regarding formulations, examples of biomaterials that enhance biodistribution and promote cellular uptake of nucleic acids include cationic lipids (e.g. D-Lin-MC3-DMA), polymers (e.g. cyclodextrin-based polymers and biocollagen), polypeptides, and exosomes. These materials usually interact with nucleic acids and self-assemble into nanoparticles that protect the cargo from enzymatic degradation and using cationic vectors (including for example cationic lipids and/or cationic nanoparticles) can confer a positive surface charge to the complex that facilitates the interaction of the positively charged complex surface with negatively-charged cellular membrane and endocytosis. Endosomolytic moieties (e.g. melittin-like peptides) or fusogenic lipids (e.g. DOPE) can also be included in nanosystems engineered to promote release from the endosomal compartment and enable immediate release of siRNAs to the cytoplasm, which can reduce dose requirements for *in vivo* administration. Also useful can be materials that can mask positive charges of the nanosystem, such as polyethyleneglycol (PEG)-lipid layer, and reduce *in vivo* aggregation. Regarding conjugation of ligands to improve bioavailability of RNAi drugs, ligands have been successfully attached to the 5'- and/or 3'-end of the sense strand without affecting RISC loading. Examples of available ligands that have been investigated for such purposes include lipids (for example, cholesterol, docosahexanoic acid), carbohydrates (for example, N-acetylgalactosamine (GaINAc)), glycoproteins, and peptides or peptide derivatives (for example, transferrin, tat peptide, GLP1), folate, and vitamin E. Broad functional delivery to many different tissues can be achieved with lipid bioconjugates such as cholesterol and docosahexaenoic acid (DHA), which preferably are selected depending on their specific distribution profile and the desired tissue intended to be targeted.

The eye is a relatively isolated tissue compartment, which provides advantages for utilization of siRNA-based drugs for treating retinal diseases. Feasibility of using siRNA for treatment of CNV has been demonstrated using siRNAs administered by intravitreal injection directed against VEGF or VEGF receptor 1 (VEGFR1) {Campochiaro PA. 2006}. Delivery of siRNAs to the posterior segment by topical instillation is truly challenging, because of the relatively large distance that the siRNAs must go through the vitreous body before they reach the retina {Guzman-Aranguez A. et al 2013}. In addition, pharmaceutical treatment of retinal diseases affecting the posterior segment of the eye is also made challenging by restrictive blood ocular barriers such as the blood aqueous barrier (BAB) and the BRB, which separate the eye from systemic circulation. Furthermore, the compartmentalized structure of the eye limits the passage of siRNAs from the anterior chamber to the posterior segment of the eye. Finally, once siRNAs successfully enter the back of the eye, effective clearance mechanisms act to rapidly clear the delivered molecules {Del Amo EM et al 2008}. Thus, direct injection into the vitreous cavity has become the most efficient means to deliver siRNA-based therapeutics into the posterior segment of the eye {Edelhauser HF et al 2010}. Intravitreous injection of siRNAs achieves high concentrations of siRNAs that are locally available to the retinal tissues while limiting systemic exposure. However, the concentration of siRNAs is rapidly depleted from the posterior segment due to degradation by vitreous endonucleases and/or via permeation across the BRB and by diffusion across the vitreous to the anterior chamber. Thus, multiple intravitreal injections are required to maintain optimal siRNA concentrations within the posterior segment of the eye. The main disadvantage of this administration mode is that multiple intravitreal injections are associated with raised intraocular pressure, vitreous or retinal hemorrhage, retinal detachment, retinal tears, endophthalmitis, cataracts, floaters, and transient blurry vision {Edelhauser HF et al 2010}. Therefore, while intravitreal injections ensure delivering a high concentration of siRNA to the retina, this method of administration also comes with its own set of particular risks. Consequently, topical administration of siRNAs could reduce risks and entail a more patient-friendly method of administration.

Naked siRNAs have shown to reach certain regions following topical applications, but access to deeper regions such as the innermost layer of the retina and effective cellular uptake require the development of strategies that ensure sufficient concentration of the compound reaching the cytoplasm of cells located in the target area and provoke a desired physiologic or therapeutic response. Physical approaches to deliver siRNAs across the stratum corneum barrier include microneedles (Chong, Gonzalez-Gonzalez et al., 2013), intradermal injection (Leachman, Hickerson et al., 2010), electroporation (Nakai, Kishida et al., 2007), and iontophoresis (Kigasawa, Kajimoto et al., 2010) among others. Modifications of the molecule and/or formulation can also enable the molecule to penetrate the required region and improve cellular uptake.

Topical administration of siRNA-based therapeutics for the treatment of retinal diseases has been described; for instance, US20130123330 discloses the treatment of diabetic retinopathy and other ocular neovascularization diseases by administering at least a siRNA duplex binding to mRNA molecules encoding VEGF or VEGFR2, or a cocktail combining siRNA duplexes targeting both genes VEGF and VEGFR2. This patent application described that the siRNA duplexes may be administered to the eye topically, subconjunctivally, or intravitreally. However, the specification only includes examples of compounds administered intravitreally or subconjunctivally.

WO2010048352 (Quark Pharmaceuticals) discloses the use of chemically modified siRNA compounds for the treatment of ocular diseases, disorders and injuries associated with degeneration or death of retinal ganglion cells, including retinitis pigmentosa (RP), diabetic retinopathy (DR), diabetic macular edema (DME) and age-related macular degeneration (AMD). Although the topical delivery to retinal tissue has been demonstrated for siRNA compounds which down-regulate the expression of target genes associated with loss of these cells, such as CASP2, RTP801, TIGASEII and p53 genes, only siRNA compounds targeting Caspase 2 have been proven to provide an ocular neuroprotective effect by increasing the survival of the retinal ganglion cells.

WO2017042238 (Sylentis) also discloses the use of siRNA compounds targeting the mRNA of the NRARP gene for the treatment of ocular diseases related with neovascularization in the retina, such as age-related macular degeneration (AMD), diabetic retinopathy (DR) or diabetic macular edema (DME). This published patent document shows that topical delivery onto the ocular surface of a chemically modified NRARP siRNA is able to reduce lesion size and neo-vessel formation in an animal model of laser-induced choroidal neovascularization (CNV) in rats.

Particular difficulties have been associated with non-viral gene transfer into the retina in vivo. One of the challenges is to overcome the inner limiting membrane, which impedes the transfection of the retina. The efficient entry into ocular tissues and efficient internalization into cells of the ocular system have been achieved for example by conjugation of lipophilic monomers or moieties to the RNAi agent.

WO2021092145 discloses some lipophilic moieties that can be conjugated to the oligonucleotide strand of the RNAi agent, optionally via a linker or carrier, to provide optimal hydrophobicity for an enhanced in vivo delivery to ocular cells. These lipophilic moieties can be aliphatic, cyclic such as alicyclic, or polycyclic such as a polyalicyclic compound, such as a steroid (e.g., sterol) or a linear or branched aliphatic hydrocarbon. The lipophilic moiety may generally comprise a hydrocarbon chain, which may be cyclic or acyclic, and may comprise various substituents and/or one or more heteroatoms, such as an oxygen or nitrogen atom. Such lipophilic aliphatic moieties include, without limitation, saturated or unsaturated C₄-C₃₀ hydrocarbon (e.g., C₆-C₁₈ hydrocarbon), saturated or unsaturated fatty acids, waxes (e.g., monohydric alcohol esters of fatty acids and fatty diamides), terpenes (e.g., C₁₀ terpenes, C₁₅ sesquiterpenes, C₂₀ diterpenes, C₃₀ triterpenes, and C₄₀ tetraterpenes), and other polyalicyclic hydrocarbons. For instance, the lipophilic moiety may contain a C₄-C₃₀ hydrocarbon chain (e.g., C₄-C₃₀ alkyl or alkenyl). In some embodiment the lipophilic moiety contains a saturated or unsaturated C₆-C₁₈ hydrocarbon chain (e.g., a linear C₆-C₁₈ alkyl or alkenyl). In one embodiment, the lipophilic moiety contains a saturated or unsaturated C₁₆ hydrocarbon chain (e.g., a linear C₁₆ alkyl or alkenyl). Suitable lipophilic moieties include lipid, cholesterol, retinoic acid, cholic acid, adamantane acetic acid, 1-pyrene butyric acid, dihydrotestosterone, 1,3-bis-O(hexadecyl)glycerol, geranyloxyhexyanol, hexadecylglycerol, borneol, menthol, 1,3-propanediol, heptadecyl group, palmitic acid, myristic acid, O3-(oleoyl)lithocholic acid, O3-(oleoyl)cholenic acid, ibuprofen, naproxen, dimethoxytrityl, or phenoxazine. The lipophilic moiety may be conjugated to the RNAi agent via a direct attachment to the ribosugar of the RNAi agent (for example, in position 2'-O), or alternatively, conjugated via a linker containing an ether, thioether, urea, carbonate, amine, amide, maleimide-thioether, disulfide, phosphodiester, sulfonamide linkage, or a product of a click reaction, or via a carrier that replaces one or more nucleotides which include sugar replacement-based monomers cyclic (e.g. 3-hydroxyproline) or acyclic. Examples of useful linkers and details about their structure and properties are described in WO2021092145, and include cleavable linkers with groups that are cleaved upon reduction or oxidation reactions (redox cleavable linker, e.g. disulphide group), groups that are cleaved under acidic conditions (acid cleavable linkers, e.g. hydrazones, ketals, acetals, esters, and esters of amino acids, which can have the general formula -C=NN-, C(O)O, or -OC(O)), groups that are cleaved by enzymes such as esterases and amidases (ester-based linkers or linking groups, e.g. esters of alkylene, alkenylene and alkynylene groups, which have the general formula - C(O)O-, or -OC(O)-), groups that are cleaved by agents that degrade or hydrolyze the phosphate group (phosphate-based cleavable linkers, e.g. a phosphate group -O- P(O)(OH)-O-), groups that are cleaved by enzymes such as peptidases and proteases in cells (peptide-based linkers, e.g. peptide bonds formed between amino acids to yield oligopeptides and polypeptides, which have the general formula -NHCHR¹C(O)NHCHR²C(O)-, where R¹ and R² are the R groups of the two adjacent amino acids), and biocleavable linkers that are nucleotide and non-nucleotide linkers or combinations thereof that connect two parts of a molecule, for example, one or both strands of two individual siRNA molecule to generate a bis(siRNA) (e.g. such linker can be a mere electrostatic or stacking interaction between two individual siRNAs, or a group consisting of DNA, RNA, disulfide, amide, functionalized monosaccharides or oligosaccharides of galactosamine, glucosamine, glucose, galactose, and mannose, and combinations thereof). Examples of carriers that replaces one or more nucleotide are also described in WO2021092145, which include cyclic groups such as pyrrolidinyl, pyrazolinyl, pyrazolidinyl, imidazolinyl, imidazolidinyl, piperidinyl, piperazinyl, 1,3-dioxolane, oxazolidinyl, isoxazolidinyl, morpholinyl, thiazolidinyl, isothiazolidinyl, quinoxalinyl, pyridazinonyl, tetrahydrofuryl, or decalin; and also acyclic groups such as moieties based on a serinol backbone or a diethanolamine backbone.

Accordingly, there is a need in the art for effective and optimized alternative or new treatments for subjects having retinal dystrophies, e.g., in subjects with retinitis pigmentosa, Leber's congenital amaurosis or Alström syndrome.

### SUMMARY OF THE INVENTION

The present invention provides improved products, in particular RNAi oligonucleotides and siRNA molecules, which are targeted to TSC2 gene which modulate or reduce the expression and/or activity of TSC2, and optionally also modulate or reduce the TSC1 expression. These products are particularly useful for managing or treating inherited retinal dystrophies and retinal dystrophies characterized by photoreceptor death. One of the advantages of treating a retinal dystrophy with RNAi could be increasing potency by using a combination of different siRNAs to treat the condition; this could be achieved by combining siRNAs or RNAi oligonucleotides targeting TSC2 expression with other modulators of TSC2 and/or other molecular mediators involved in the pathogenic mechanisms underlying the clinical manifestations of these diseases or in the photoreceptor death observed in subjects suffering from it, such as the TSC1 gene. Another advantage is that treatments based on siRNA or RNAi will have a longer-lasting effect. This feature is due to the fact that siRNAs or RNAi oligonucleotides block the synthesis of the target protein. When treatment is suspended the cell will have to synthesise new target proteins from scratch; whereas treatments based on small molecules would leave the target protein intact, ready to be active again once the inhibitor is no longer present. The mechanism of action of siRNAs or RNAi entails that once the active molecule reaches the cytoplasm the same molecule can be used to mediate the degradation of many target mRNA molecules, this is not the case with antibodies, which require a 1:1 stoichiometry. In addition, using siRNA products for managing such disease versus traditional TSC2 and/or TSC1 inhibitors is that they are highly specific for their target and are optimized for minimum off-target effect. Therefore, it is anticipated that lower doses of the compounds will be needed to achieve the same clinical efficacy thus potentially reducing side effects.

In some embodiments, the RNAi oligonucleotides and siRNA molecules of the invention comprise two complementary oligonucleotide strands, wherein the antisense strand preferably has from 19 to 21 nucleobases and is complementary to at least 18 consecutive nucleotides, and preferably to at least 19 consecutive nucleotides, of the TSC2 mRNA, and the sense strand has from 14 to 21 nucleobases with at least 14 consecutive nucleotides from the 5'-end or the 3'-end being complementary to at least 14 consecutive nucleotides of the antisense strand, preferably from 14 to about 19 nucleobases, and more preferably 15, 16, 17, 18 or 19 nucleobases. In some embodiments, the RNAi oligonucleotide or siRNA molecule comprises at least one modified nucleobase, modified sugar moiety or modified internucleoside linkage. In some embodiments, the RNAi oligonucleotide or siRNA molecule comprises at least one modification selected from: 2'-O-methyl modification, 2'-fluoro modification, 2'-O-methyl-5'-vinylphosphonate modification, 5'-O-methyl modification, substitution of uracil with 5'-methyluridine, substitution of cytosine with 5-methylcytosine, substitution of uridine or cytosine with 2'-deoxyinosine, substitution of uracyl ribose nucleotide with deoxythymidine nucleotide, substitution of ribose nucleotide with deoxyribose nucleotide, substitution of ribose nucleotide with unlocked nucleic acid (UNA) monomer, substitution of ribose nucleotide with glycol nucleic acid (GNA) monomer, substitution of ribose nucleotide with inverted abasic deoxyribose, introduction of phosphorothioate modified nucleotides, and combinations thereof. In other embodiments, the oligonucleotide or siRNA molecule comprises at least one 2'-O-methoxy modification and/or at least one 2'-fluoro modification and/or at least one phosphorothioate linkage.

In some embodiments, the siRNA is blunt ended; that is, each strand is the same length, and there are no overhangs. Other embodiments may also include 3' mononucleotide or dinucleotide overhangs (for example, U, UU, dT or dTdT).

In some other embodiments, the RNAi oligonucleotide or siRNA molecule may also include additional abasic deoxyribose monomers at the 5'-end or the 3'-end, or at both 5'- and 3'-ends, of any one of its two complementary strands, and preferably in one or both of these ends of its sense strand.

In other embodiments the siRNA compounds or RNAi oligonucleotide may be short hairpin oligonucleotides, being a single strand which forms a hairpin such that a double stranded region is formed wherein a first portion of the oligonucleotide has from 19 to 21 nucleobases and is complementary to at least 19 consecutive nucleotides of the TSC2 mRNA, and a second portion of the oligonucleotide has from 14 to 21 nucleobases with at least 14 consecutive nucleotides from the 5'-end or the 3'-end being complementary to at least 14 consecutive nucleotides of the first portion, preferably from 14 to about 19 nucleobases, and more preferably 15, 16, 17, 18, or 19 nucleobases; and said double stranded region comprises at least 14 consecutive nucleobases of said second portion.

Also provided are kits, compositions, including pharmaceutical compositions, and medicaments comprising compounds of the invention.

Also provided are RNAi oligonucleotides or siRNA molecules, which are targeted to a nucleic acid encoding TSC2, and which modulate the expression of TSC2, or both TSC2 and TSC1, for use in preventing or treating a retinal dystrophy. These compounds can be useful against retinal dystrophies such as retinitis pigmentosa, Leber's congenital amaurosis (LCA) or in Alström syndrome. These compounds can be also useful for ameliorating or reducing the signs or symptoms of the disease, including the loss of photoreceptor cells, retinal disorganization or other signs as visual impairment.

Also provided is a pharmaceutical composition comprising an RNAi oligonucleotide having at least an siRNA molecule targeted to a nucleic acid encoding TSC2 and an acceptable carrier, and optionally comprising also an RNAi oligonucleotide or siRNA molecule targeted to a nucleic acid encoding TSC1.

Also provided is the use of an RNAi oligonucleotide or siRNA molecule in the manufacture of a medicament for preventing or treating a retinal dystrophy, including retinitis pigmentosa, Leber's congenital amaurosis (LCA) or Alström syndrome, wherein the RNAi oligonucleotide or siRNA molecule is targeted to a nucleic acid encoding TSC2 and reduces the expression and/or activity of TSC2 gene or the levels of TSC2 protein, or reduces the expression and/or activity of TSC2 and TSC1 genes or the levels of TSC2 and TSC1 proteins. Also provided is the use in the manufacture of such medicaments of an RNAi oligonucleotide comprising an siRNA molecule targeted to a nucleic acid encoding TSC2, and an RNAi oligonucleotide comprising an siRNA molecule targeted to a nucleic acid encoding TSC1, which reduces the expression and/or activity of TSC2 and TSC1 genes or the levels of TSC2 and TSC1 proteins. Furthermore, the present disclosure relates to the use in the manufacture of these medicaments of an RNAi oligonucleotide comprising an siRNA compound targeted to a nucleic acid encoding TSC1 which reduces the expression and/or activity of TSC1 gene or the levels of the TSC1 protein, which are particularly useful for therapeutic purposes as it reduces also the expression and/or activity of TSC2 gene or the levels of the TSC2 protein, or it does not have a significant impact on the reduction in the expression levels of the TSC2 when used in combination with an RNAi oligonucleotide or siRNA molecule targeting the TSC2 gene.

Further provided are methods of modulating the expression of TSC2 in one or more cells or tissues, comprising contacting the cell(s) or tissue(s) with one or more RNAi oligonucleotides, siRNA molecules or compositions of the invention. The method may be in vivo, ex vivo, or in vitro. Methods of treating a subject, particularly a human, suspected of having or being prone to a disease or condition associated with expression of TSC2, preferably TSC2 and/or TSC1, or in need of treatment therefor, are also set forth herein; as are methods of treating a subject, particularly a human, suspected of having or being prone to a retinal dystrophy. Such methods comprise, for example, administering a therapeutically or prophylactically effective amount of one or more of the RNAi oligonucleotides, siRNA molecules or compositions of the invention to the subject being treated. In some embodiments, the subject to be treated has been diagnosed with having a retinal dystrophy, such as a retinitis pigmentosa, LCA or Alström syndrome.

The present invention also provides methods of treating a subject, particularly a human, having a retinal dystrophy (including retinitis pigmentosa, LCA or Alström syndrome) or a disease or condition associated with photoreceptor death, or in need of treatment therefor, comprising administering to the subject a therapeutically or prophylactically effective amount of a oligonucleotide or siRNA described herein so that expression of TSC2, and optionally TSC1, is inhibited. Such methods comprise administering a therapeutically or prophylactically effective amount of one or more of the compounds or compositions of the invention to the subject being treated, which can be administered in a combined treatment with a TSC1 inhibitor or TSC1 siRNA, to improve the therapeutic effect. The components of a combined treatment may be administered simultaneously or sequentially. In some embodiments, the animal or person being treated has been diagnosed with having the retinal distrophy.

### DESCRIPTION OF THE DRAWINGS

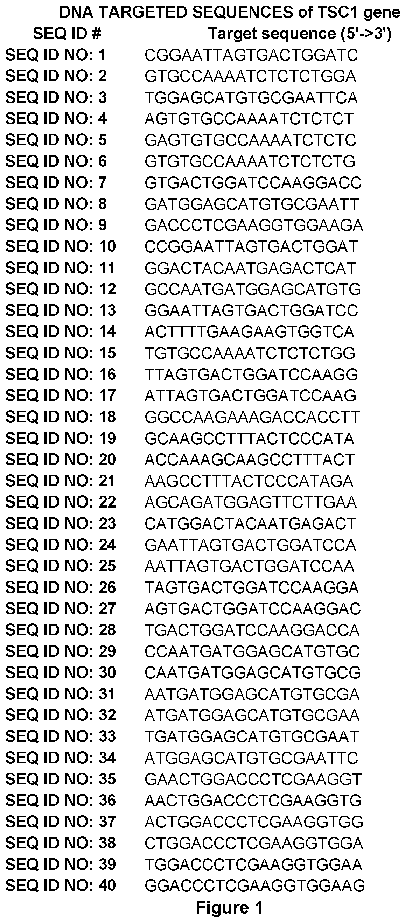

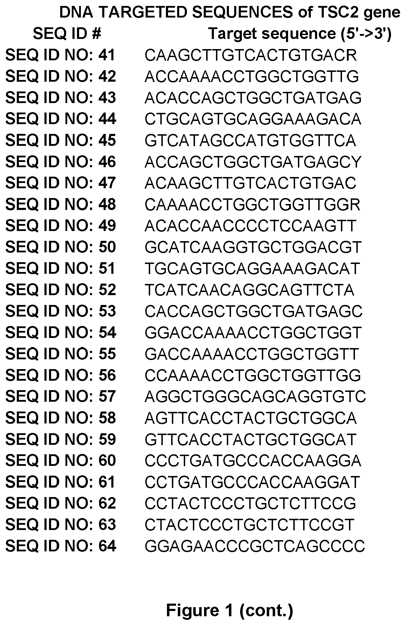

**FIG.** 1: shows the short fragments of the target gene sequences of TSC1 and TSC2 genes chosen as the target sequences for the siRNAs of the present invention. Nucleotide sequences SEQ ID NO: 1 to SEQ ID NO: 40 correspond to fragments of the gene sequences of TSC1 gene. Nucleotide sequences SEQ ID NO: 41 to SEQ ID NO: 64 correspond to fragments of the gene sequences of TSC2 gene. R can be A or G, and preferably is A; Y can be T or C, and preferably is T.

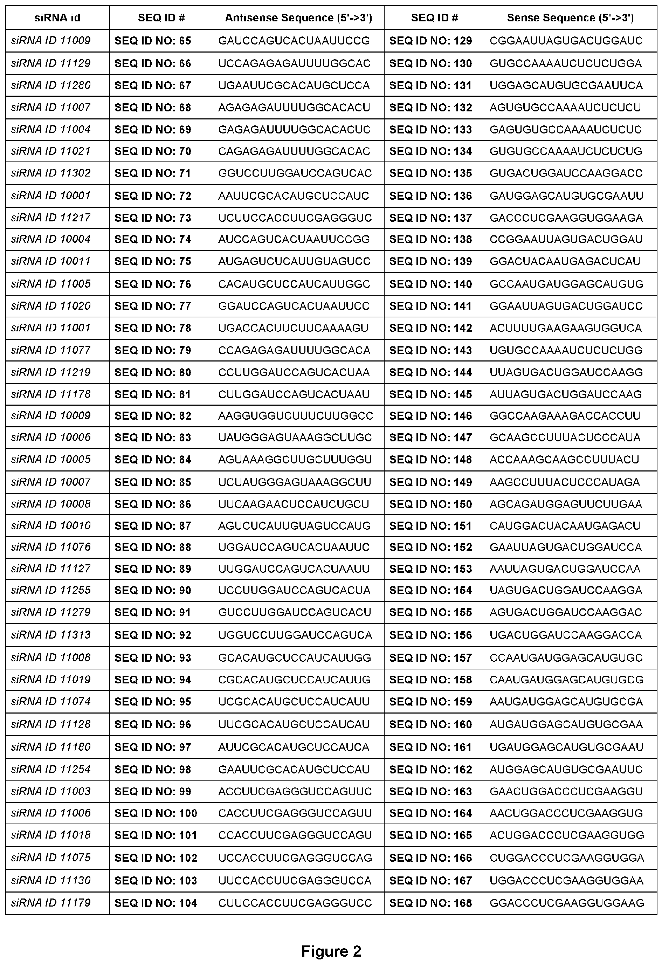

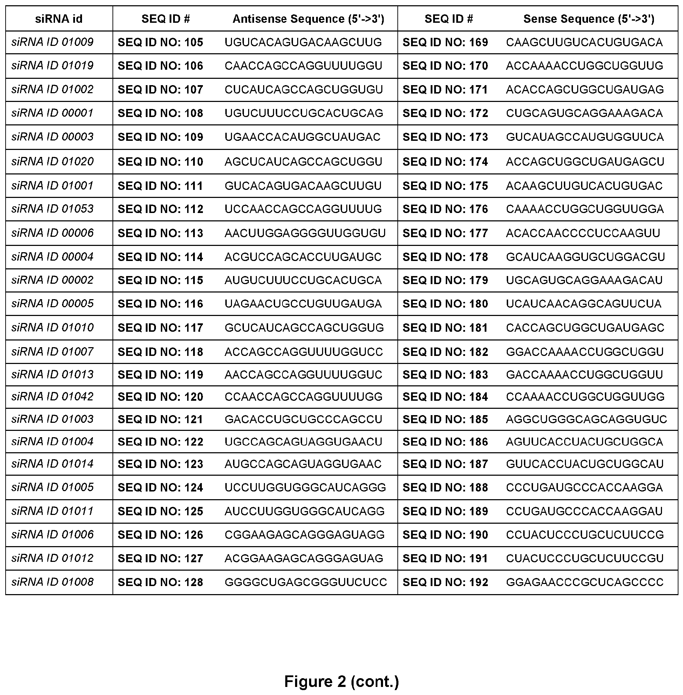

**FIG.** 2: shows the oligonucleotide sequences for the blunt ended siRNA molecules of the present invention targeting TSC2 and TSC1 encompassed by the present invention. The SEQ ID NOs given in the Figure refer to the oligonucleotide sequence of the antisense and sense (5' -> 3') strands; typically, siRNAs will be administered as dsRNAs, so siRNAs will include both the antisense strand and its complementary sense strand. SEQ ID NO. 65 to SEQ ID NO. 128 are antisense strands of siRNAs targeting SEQ ID NO. 1 to SEQ ID NO. 64, respectively. SEQ ID NO. 129 to SEQ ID NO. 192 correspond respectively to the complementary sense strands of siRNAs having antisense strands SEQ ID NO. 65 to SEQ ID NO. 128. Generally, a siRNA or the dsRNA will include the antisense and sense strands and may also include 3' mononucleotide or dinucleotide overhangs (for example, U, UU, dT or dTdT). However, this is not essential.

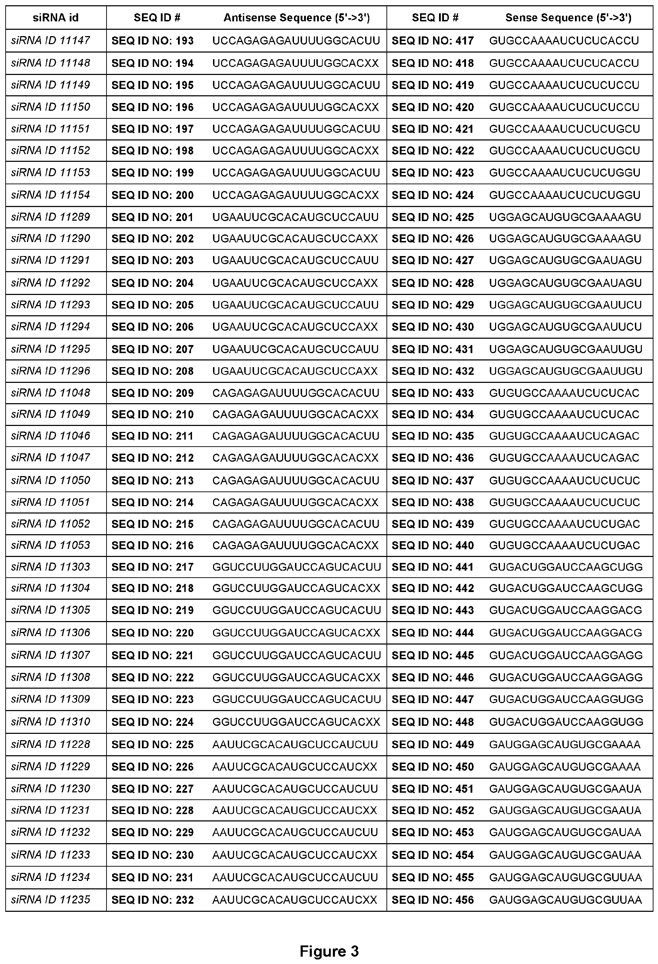

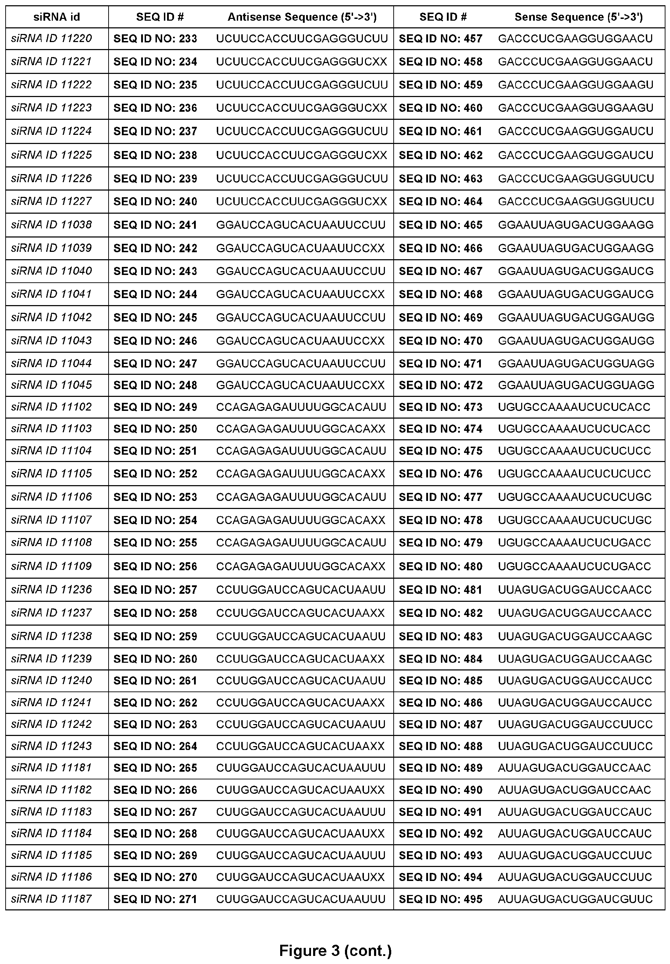

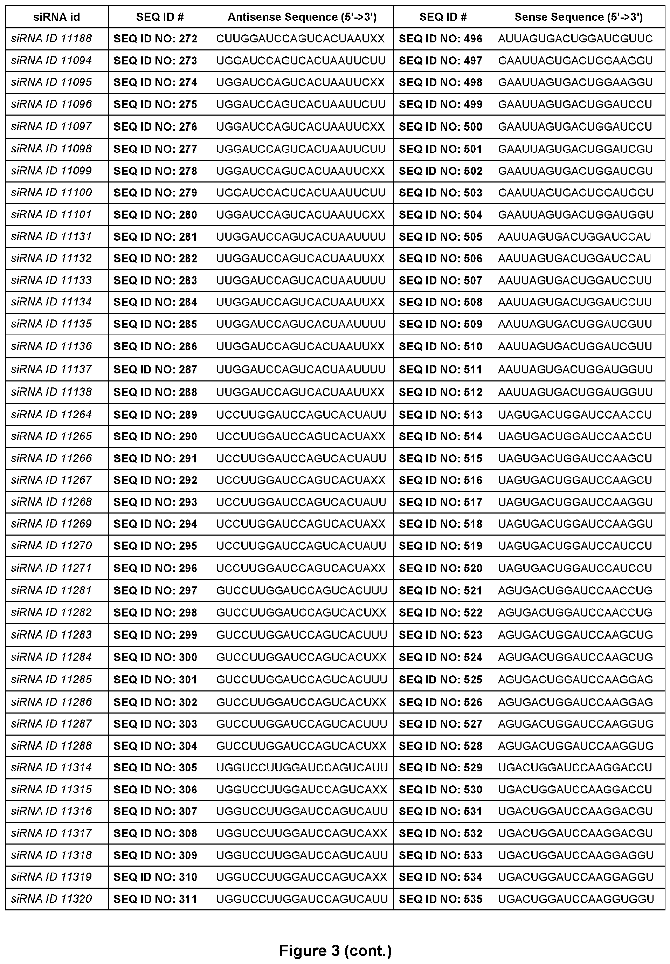

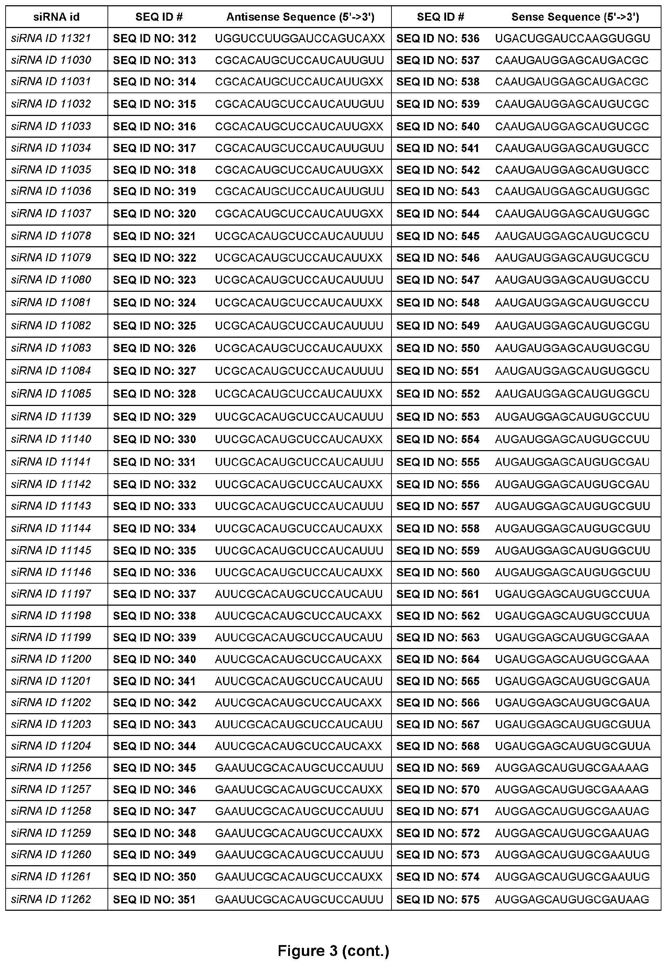

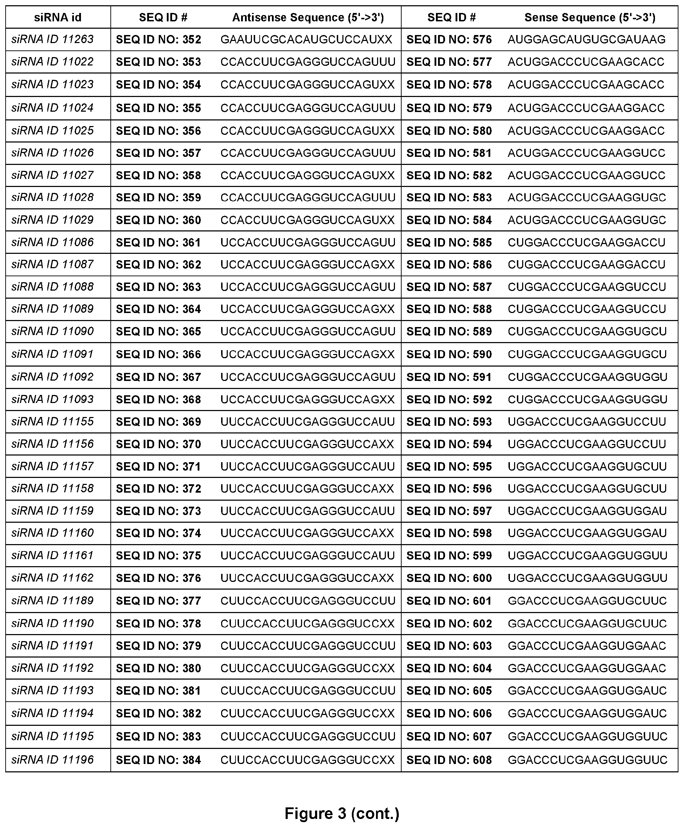

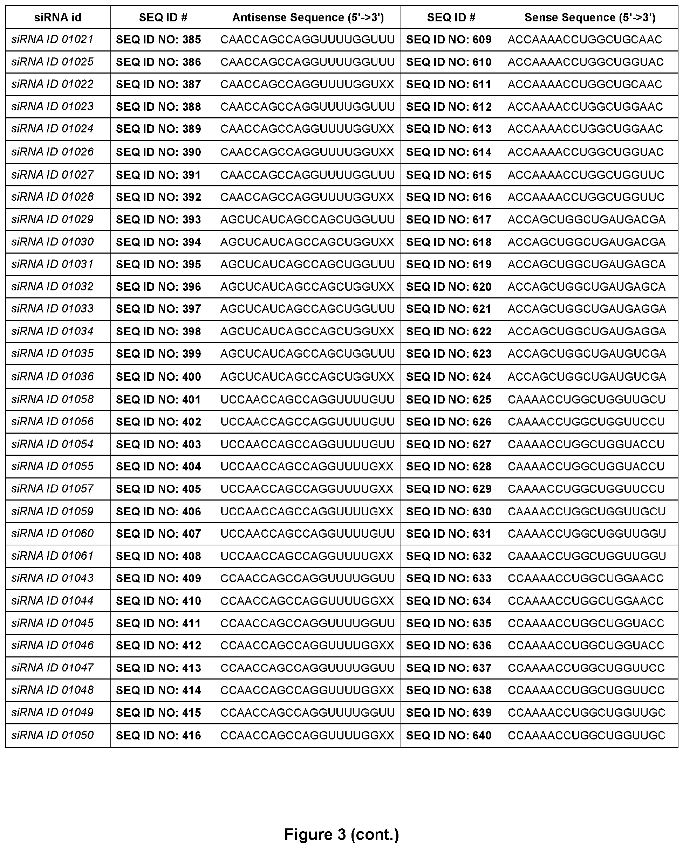

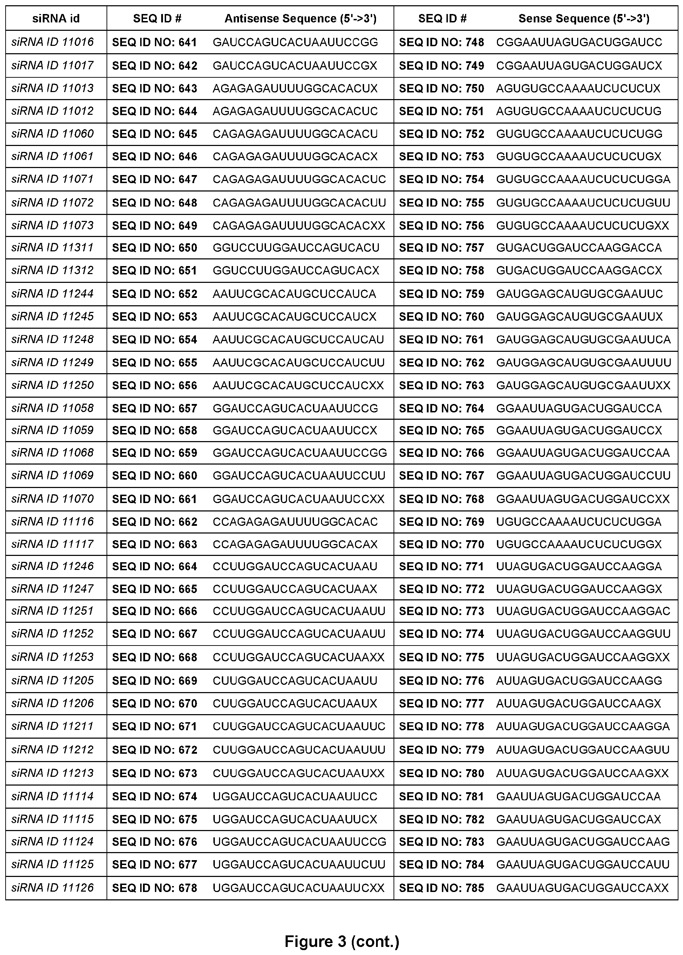

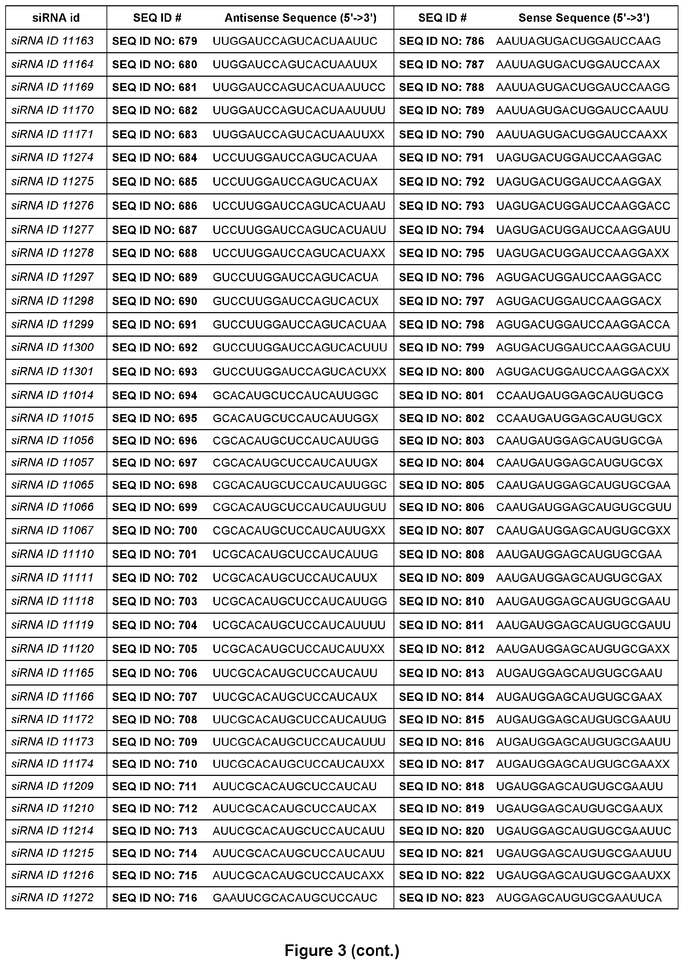

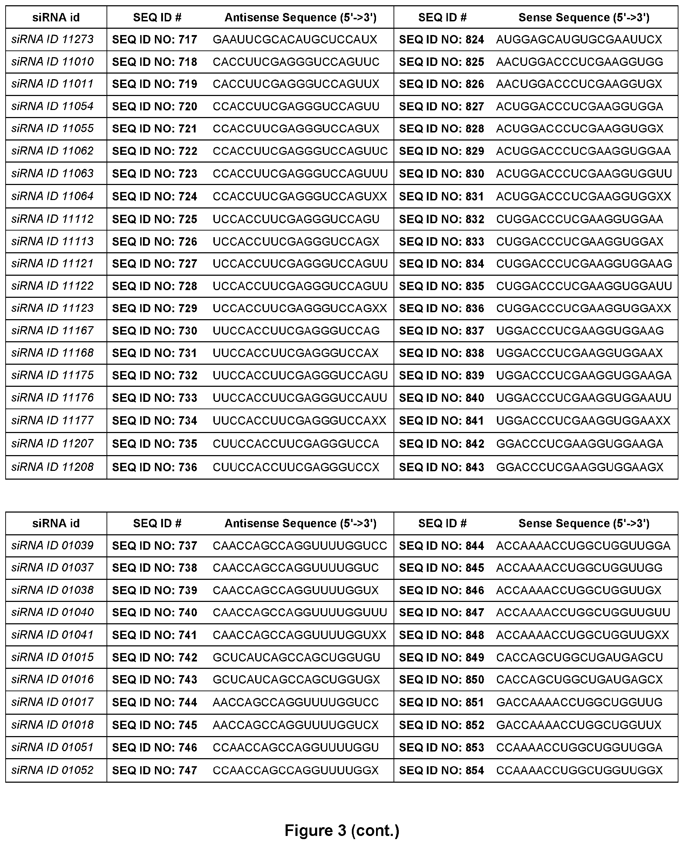

**FIG.** 3: shows the oligonucleotide sequences of the siRNA molecules targeting TSC1 or TSC2 with at least one 3'-overhang in any of their strands encompassed by the present invention. The SEQ ID NOs given in the Figure refer to the oligonucleotide sequence of the antisense and sense (5' -> 3') strands; typically, siRNAs will be administered as dsRNAs, so siRNAs will include both the antisense strand and its complementary sense strand. SEQ ID NO. 193 to SEQ ID NO. 416 and SEQ ID NO. 641 to SEQ ID NO. 747 are antisense strands of siRNAs targeting any of sequences SEQ ID NO. 1 to SEQ ID NO. 64, and SEQ ID NO. 417 to SEQ ID NO. 640 and SEQ ID NO. 748 to SEQ ID NO. 854 respectively correspond to complementary sense strands of siRNAs having antisense strands SEQ ID NO. 193 to SEQ ID NO. 416 and SEQ ID NO. 641 to SEQ ID NO. 747. X is dT (deoxythymidine).

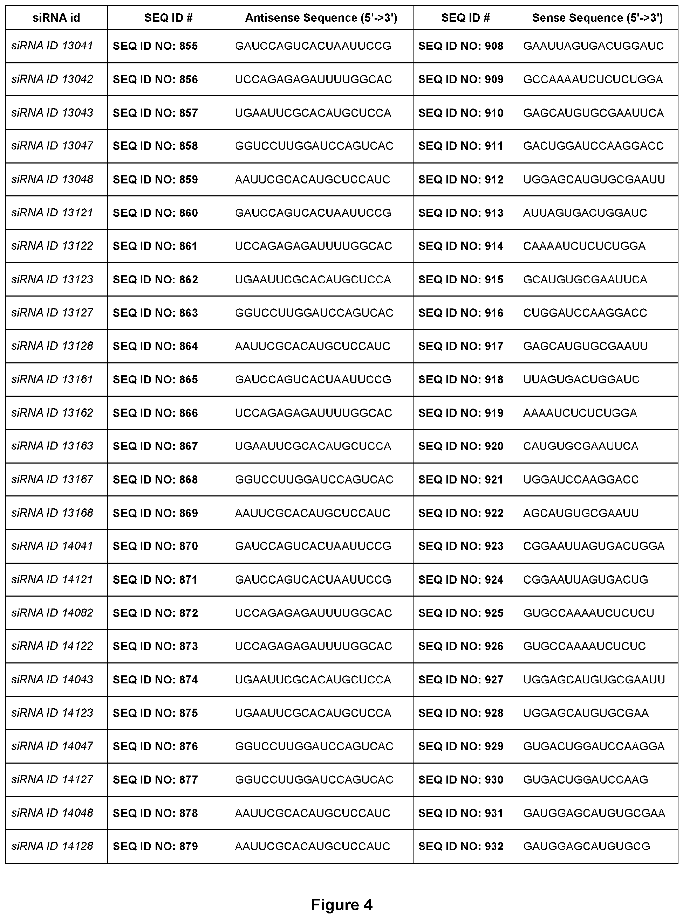

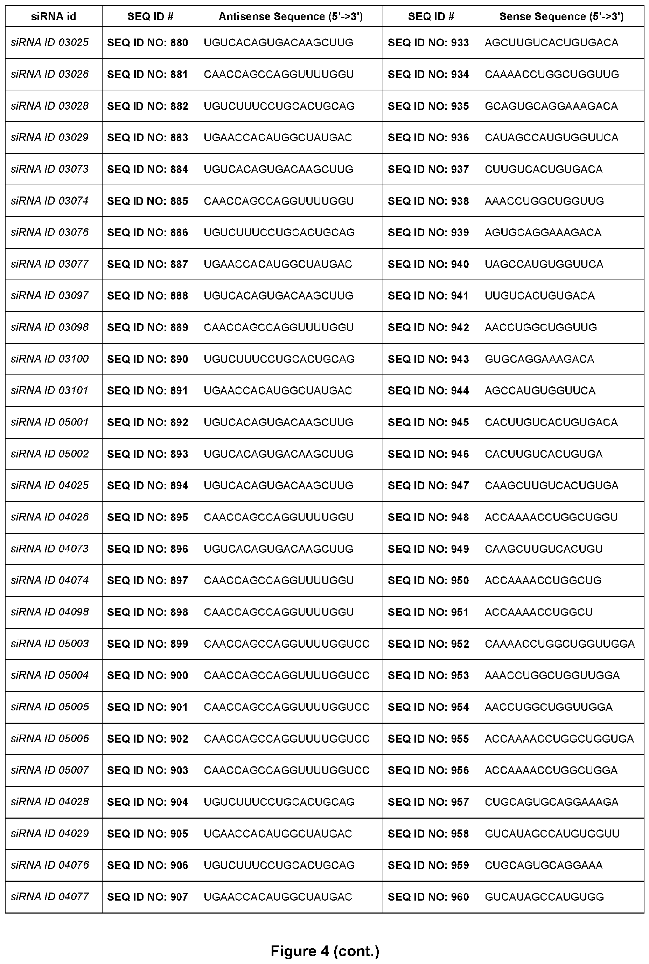

**FIG.** 4: shows the oligonucleotide sequences of asymmetric siRNA molecules targeting TSC1 or TSC2 encompassed by the present invention having sense strands shorter than their complementary antisense strands composing a dsRNA region of at least 14 nucleotides. The SEQ ID NOs given in the Figure refer to the oligonucleotide sequence of the antisense and sense (5' -> 3') strands; typically, siRNAs will be administered as dsRNAs, so siRNAs will include both the antisense strand and its complementary sense strand. SEQ ID NO. 855 to SEQ ID NO. 907 are antisense strands of siRNAs targeting any of sequences SEQ ID NO. 1 to SEQ ID NO. 64, and SEQ ID NO. 908 to SEQ ID NO. 960 respectively correspond to complementary sense strands of siRNAs having antisense strands SEQ ID NO. 855 to SEQ ID NO. 907. X is dT (deoxythymidine).

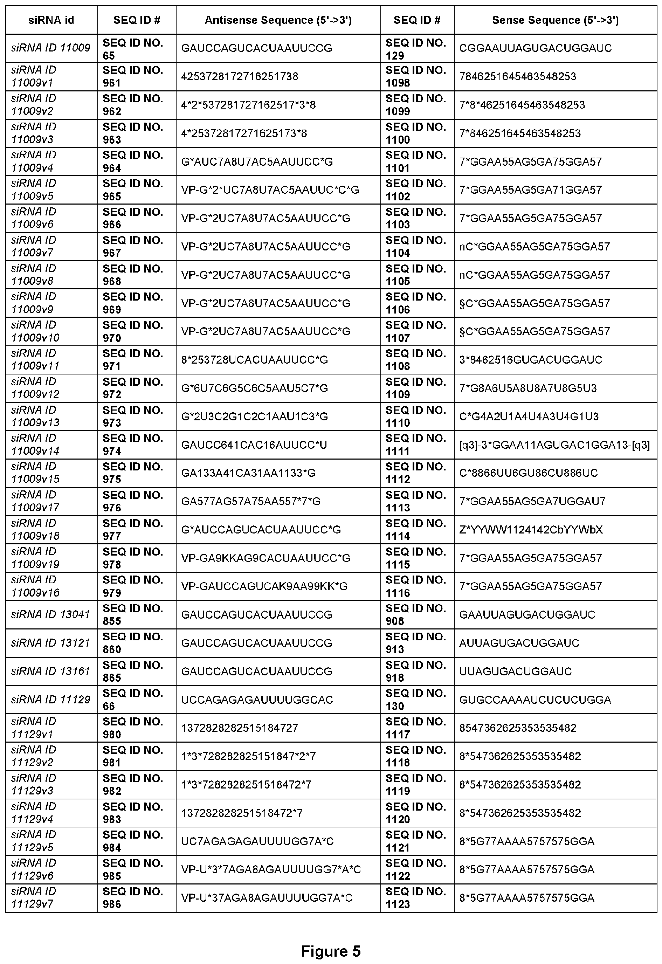

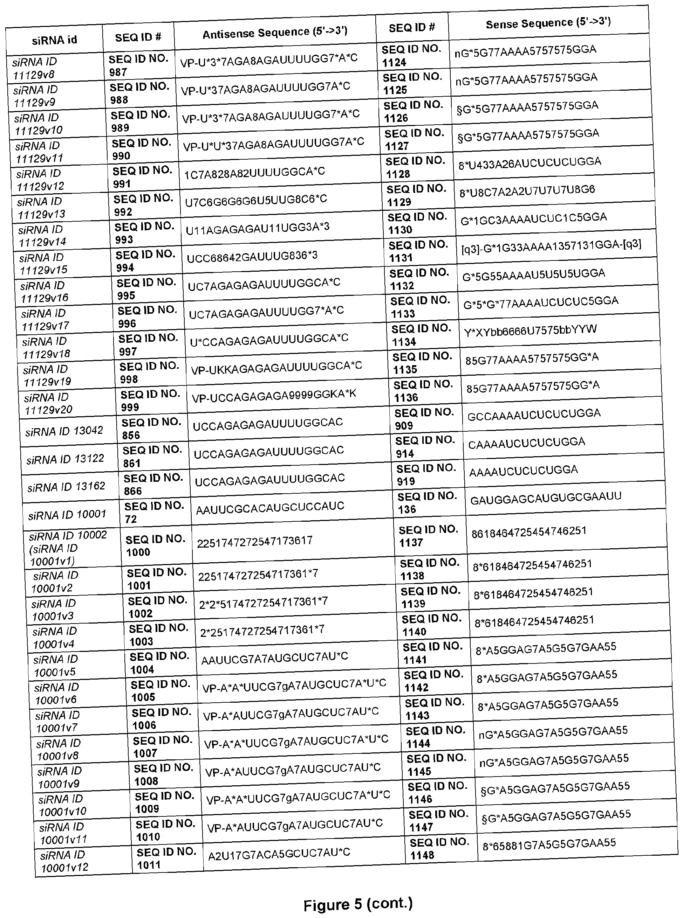

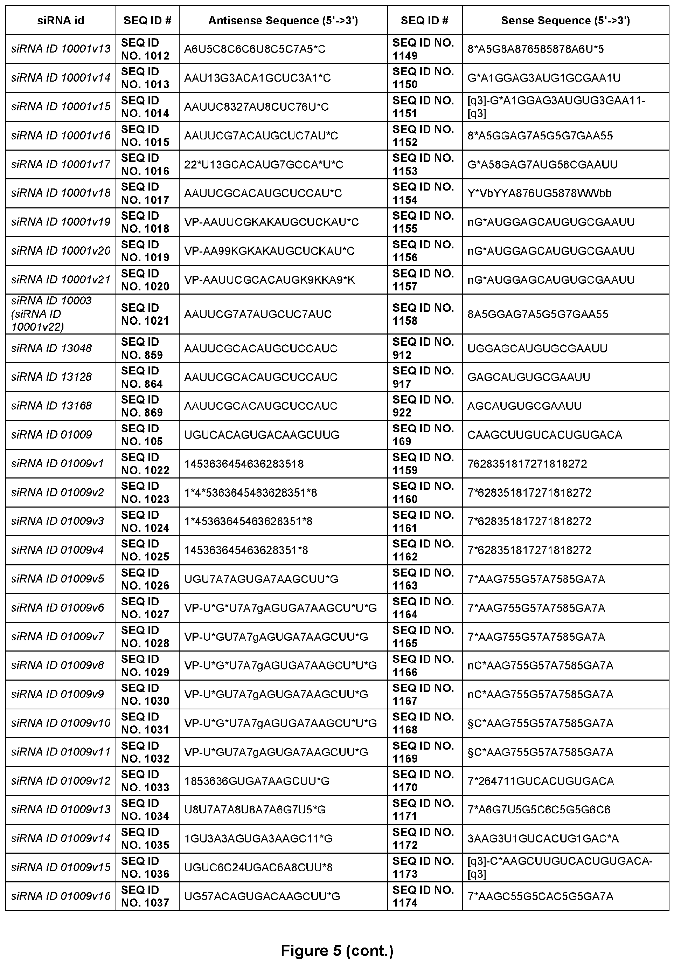

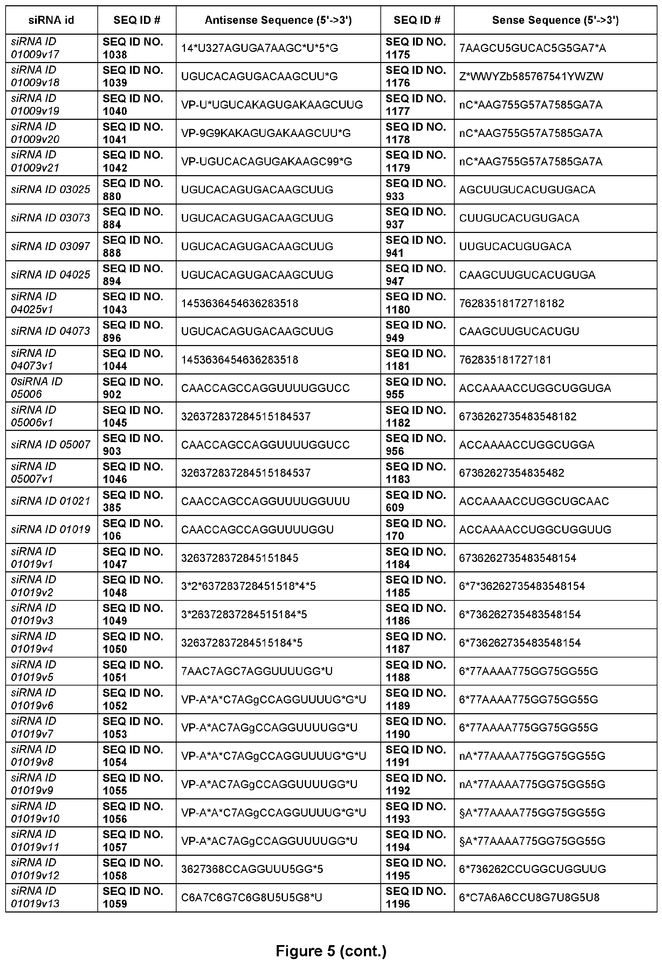

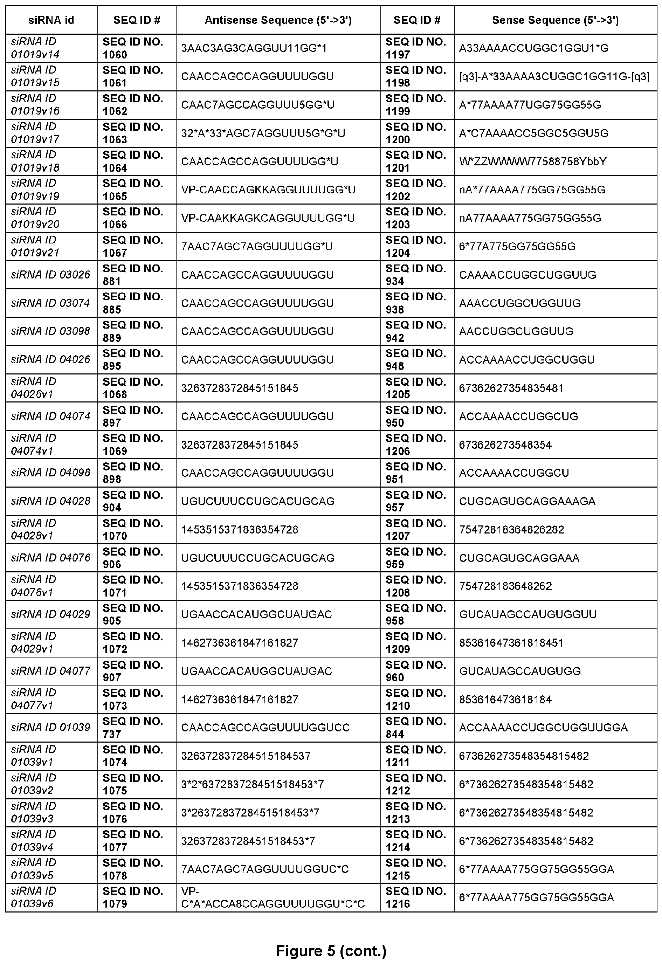

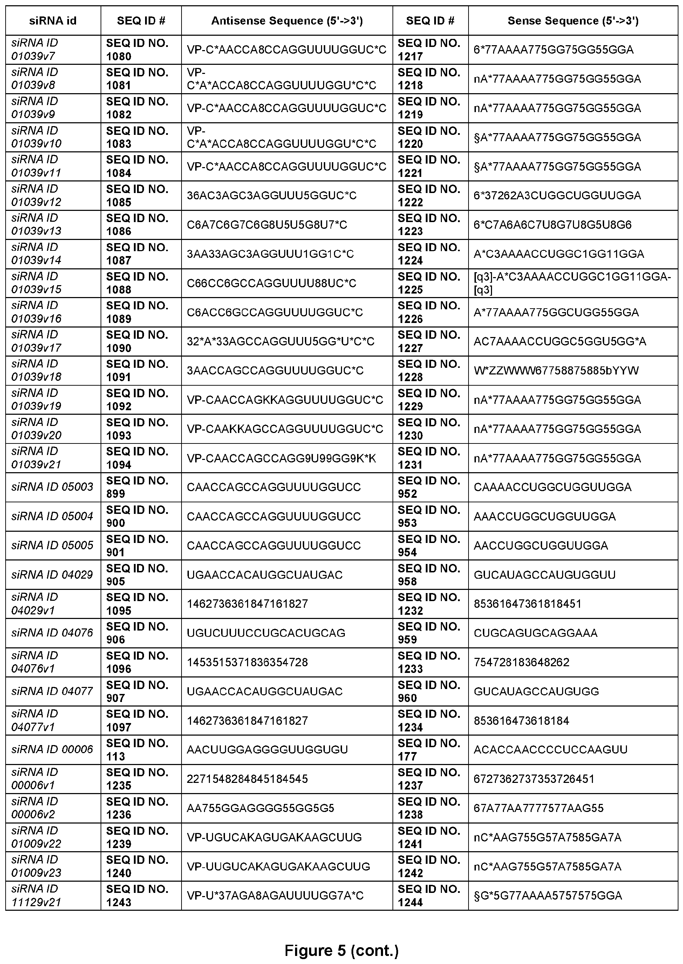

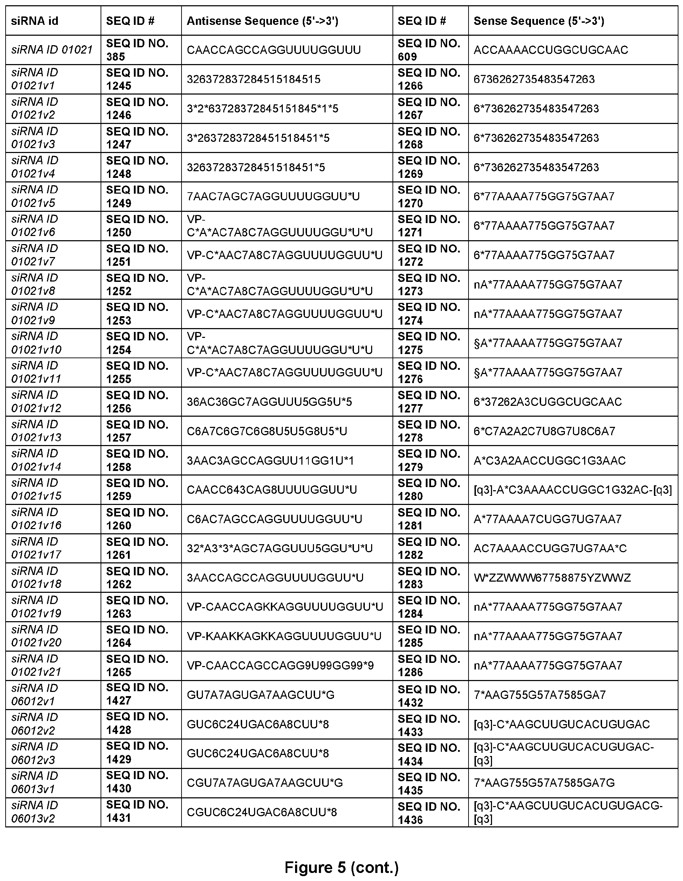

**FIG.** 5: shows oligonucleotide sequences comprising short fragments of 14-21 nucleotides corresponding to nucleotide sequences of the sense (SEQ ID NO: 129, SEQ ID NO: 130, SEQ ID NO: 136, SEQ ID NO: 169, SEQ ID NO: 170, SEQ ID NO: 609, SEQ ID NO: 844, SEQ ID NO: 908, SEQ ID NO: 909, SEQ ID NO: 912 - SEQ ID NO: 914, SEQ ID NO: 917 - SEQ ID NO: 919, SEQ ID NO: 922, SEQ ID NO: 933, SEQ ID NO: 934, SEQ ID NO: 937, SEQ ID NO: 938, SEQ ID NO: 941, SEQ ID NO: 942, SEQ ID NO: 947 - SEQ ID NO: 951, SEQ ID NO: 955 - SEQ ID NO: 907, SEQ ID NO: 1098 - SEQ ID NO: 1234, SEQ ID NO: 1244 - SEQ ID NO: 1252) and antisense (SEQ ID NO: 65, SEQ ID NO: 66, SEQ ID NO: 72, SEQ ID NO: 105, SEQ ID NO: 106, SEQ ID NO: 385, SEQ ID NO: 737, SEQ ID NO: 855, SEQ ID NO: 856, SEQ ID NO: 859 - SEQ ID NO: 861, SEQ ID NO: 864 - SEQ ID NO: 866, SEQ ID NO: 869, SEQ ID NO: 880, SEQ ID NO: 881, SEQ ID NO: 884, SEQ ID NO: 885, SEQ ID NO: 888, SEQ ID NO: 889, SEQ ID NO: 894 - SEQ ID NO: 898, SEQ ID NO: 902 - SEQ ID NO: 907, SEQ ID NO: 961 - SEQ ID NO: 1097, SEQ ID NO: 1235 - SEQ ID NO: 1243) strands for the siRNAs of the present disclosure. Legend: "1" is a 2'-fluoro U (2'F U); ; "2" is a 2'-fluoro A (2'F A); "3" is a 2'-fluoro C (2'F C); "4" is a 2'-fluoro G (2'F G); "5" is a 2'-O-Methyl U RNA base (2'-OMe U); "6" is a 2'-O-Methyl A RNA base (2'-OMe A); "7" is a 2'-O-Methyl C RNA base (2'-OMe C); "8" is a 2'-O-Methyl G RNA base (2'-OMe G); "9" is a 5-methyl-Uridine; "X" is a deoxiribonucleotide (2'H) of T (deoxythymidine, dT); "VP" represents a 2'-OMe-nucleotide with a 5'-vinylphosphonate group.; "§" represents a 5'-O-Methyl nucleobase, being "§C" a 5'-O-Methyl C DNA base, "§G" a 5'-O-Methyl G DNA base, "§A" a 5'-O-Methyl A RNA base, and "§U" a 5'-O-Methyl U RNA base; "n" is a monomer of unlocked nucleic acid (UNA); "gX" is a GNA (gA, gC or gU); "[q3]-" is an inverted abasic deoxyribose; "b" is a 2'-deoxyinosine; "V" is a deoxyuridine; "W" is a deoxy-Adenosine (dA); "Y" is a deoxy-Guanosine (dG); "Z" is a deoxy-Citidina (dC); "K" is a 5-methyl C; "*" represents a phosphorothioate internucleotide bond.
**FIG.** 6: shows TSC1 gene expression levels after transfection of siRNAs targeting TSC1 in human retinal ARPE-19 cells.
**FIG.** 7: shows TSC1 gene expression levels after transfection of siRNAs targeting TSC1 in mouse muscle C2C12 cells.
**FIG.** 8: shows TSC2 gene expression levels after transfection of siRNAs targeting TSC2 in human retinal ARPE-19 cells.
**FIG.** 9: shows TSC2 gene expression levels after transfection of siRNAs targeting TSC1 in mouse muscle C2C12 cells.
**FIG.** 10: shows TSC1 gene expression levels after transfection of combination of siRNAs targeting TSC1 and TSC2 in human retinal ARPE-19 cells.
**FIG. 11****:** shows TSC1 gene expression levels after transfection of siRNAs of combination of siRNAs targeting TSC1 and TSC2 in mouse C2C12 cells.
**FIG.** 12: shows TSC2 gene expression levels after transfection of siRNAs targeting TSC2 in human retinal ARPE-19 cells.
**FIG.** 13: shows TSC2 gene expression levels after transfection of different combinations of siRNAs targeting TSC1 and TSC2 in mouse C2C12 cells.
**FIG.** 14: shows TSC1 and TSC2 gene expression levels after transfection of different combinations of siRNAs targeting TSC1 and TSC2 in human ARPE-19 cells.
**FIG.** 15: shows TSC1 and TSC2 gene expression levels after transfection of siRNAs targeting TSC1 and TSC2 in mouse C2C12 cells.
**FIG.** 16: shows mRNA levels of TSC1 in ARPE-19 cells after transfection of siRNAs chemically modified targeting TSC1.
**FIG.** 17: shows mRNA levels of TSC1 in murine C2C12 cells after transfection of siRNAs chemically modified targeting TSC1.
**FIG.** 18: shows mRNA levels of TSC2 in ARPE-19 cells after transfection of chemically modified siRNAs targeting TSC2.
**FIG.** 19: mRNA levels of TSC2 in murine C2C12 cells after transfection of chemically modified siRNAs targeting TSC2.
**FIG.** 20: shows mRNA levels of TSC1 in human ARPE-19 cells after transfection of siRNAs chemically modified targeting TSC1 and TSC2, individually and combined. NAC: non-active compound.
**FIG. 21****:** shows mRNA levels of TSC2 in human ARPE-19 cells after transfection of siRNAs chemically modified targeting TSC1 and TSC2, individually and combined. NAC: non-active compound.
**FIG.** 22: shows mRNA levels of TSC1 in murine C2C12 cells after transfection of siRNAs chemically modified targeting TSC1 and TSC2, individually and combined.
**FIG.** 23: shows mRNA levels of TSC2 in murine C2C12 cells after transfection of siRNAs chemically modified targeting TSC1 and TSC2, individually and combined.

### DETAILED DESCRIPTION OF THE INVENTION

The present invention relates to the provision and use of RNAi oligonucleotides and siRNA molecules targeting either or both of the tuberous sclerosis 2 (TSC2) gene and the tuberous sclerosis 1 (TSC1) gene, two genes encoding the protein products TSC2 (also known as tuberin) and TSC1 (also known as hamartin) which form a complex that has emerged as a critical integrator of growth-factor, nutrient and stress signals to control protein synthesis, cell growth and other cellular processes.

In one aspect, the present invention relates to the provision of RNA interfering (RNAi) oligonucleotides which are useful for use as a medicament, or in the treatment and/or prevention of a retinal dystrophy. Said RNAi oligonucleotides reduce the expression and/or activity of the tuberous sclerosis complex 2 (TSC2) gene when introduced into a cell, preferably an ocular cell, and comprise or consist of at least an siRNA molecule that specifically targets any of the nucleotide sequences SEQ ID NO. 41 to SEQ ID NO. 64 (preferably any of SEQ ID NO. 41 to SEQ ID NO. 49, and more preferably any of sequences SEQ ID NO. 41 to SEQ ID NO. 45 and SEQ ID NO. 47 to SEQ ID NO. 49) of the TSC2 gene. Preferably the target sequence comprises or consists of SEQ ID NO. 41 or SEQ ID NO: 42.

Optionally, in addition to comprising an siRNA molecule targeting any of the sequences of the TSC2 gene, the RNAi oligonucleotide can further comprise another siRNA molecule which specifically targets a sequence selected from the group consisting or comprising of: SEQ ID NO. 1 to SEQ ID NO. 40 (preferably any of SEQ ID NO. 1 to SEQ ID NO. 10, more preferably any of sequences selected from SEQ ID NO. 1 to SEQ ID NO. 8, and even more preferably any of sequences selected from SEQ ID NO. 2, SEQ ID NO.1 and SEQ ID NO. 7) of the tuberous sclerosis complex 1 (TSC1) gene, and that reduces the expression and/or activity of the TSC1 gene when introduced into a cell, preferably an ocular cell, to reduce additionally the expression of the TSC1 gene, as well as that of the TSC2 gene, in the cell or the ocular cell. Alternatively, for the use in the treatment and/or prevention of retinal dystrophy, the RNAi oligonucleotide that reduces the expression of the TSC2 gene in the cell or the ocular cell, can be used in combination with another RNAi oligonucleotide that reduces the expression of the TSC1 gene in the same cell, which comprises or consists of an siRNA molecule which specifically targets a sequence selected from the group consisting or comprising of: SEQ ID NO. 1 to SEQ ID NO. 40 (preferably any of SEQ ID NO. 1 to SEQ ID NO. 10, and more preferably any of sequences selected from SEQ ID NO. 2, SEQ ID NO.1 and SEQ ID NO. 7) of the TSC1 gene. Such use in combination can be for example as a medicament or composition that comprises a first RNAi oligonucleotide comprising or consisting of a siRNA molecule that specifically targets any of the nucleotide sequences above of the TSC2 gene, and a second RNAi oligonucleotide comprising or consisting of a siRNA molecule that specifically targets any of the nucleotide sequences above of the TSC1 gene. Another example of use in combination can be the use of the RNAi oligonucleotide that reduces the expression of the TSC2 gene in a method of treatment or prevention of retinal dystrophy that comprises additionally reducing the expression of the TSC1 gene with another RNAi oligonucleotide comprising or consisting of a siRNA molecule that specifically targets any of the nucleotide sequences above of the TSC1 gene.

For the treatment in combination, the RNAi oligonucleotides targeting the TSC2 gene and the TSC1 gene can be administered using a single composition or medicament which comprises both RNAi oligonucleotides or using two or more compositions or medicaments each comprising the different RNAi oligonucleotides, which may be administered jointly or separately. However, joint administration using a single composition or medicament, or two or more compositions administered at once are preferred for the combined treatment and can be advantageous for patients and practitioners as it makes the administration safer, faster and easier, avoiding mistakes or undesired effects resulting from multiple administration steps.

A gene is "targeted" by a RNAi oligonucleotide or a siRNA molecule according to the present invention when, for example, the RNAi oligonucleotide or the siRNA molecule selectively decreases or inhibits the expression of the gene. The phrase "selectively decrease or inhibit" as used herein encompasses RNAi oligonucleotides and siRNAs that affect expression of one gene, in this case the TSC2 gene or the TSC1 gene. Alternatively, an RNAi oligonucleotide or a siRNA targets a gene when (one strand of) the oligonucleotide or siRNA hybridizes under stringent conditions to the gene transcript, i.e. its mRNA. Hybridizing "under stringent conditions" means annealing to the target mRNA region under standard conditions, e.g., high temperature and/or low salt content which tend to disfavour hybridization. A suitable protocol (involving 0.1×SSC, 68 °C for 2 hours) is described in Maniatis, T., et al., Molecular Cloning: A Laboratory Manual, Cold Spring Harbor Laboratory, 1982, on pages 387-389.

As used herein, the term "ocular cell" refers to any cell located in the eye or associated with the function and/or structures of the eye. The term may refer to any one or more of photoreceptor cells, including rod, cone and photosensitive ganglion cells, retinal pigment epithelium (RPE) cells, glial cells, astrocytes, Muller cells, bipolar cells, horizontal cells, and amacrine cells. In one embodiment, the ocular cell is an RPE cell. In another embodiment, the ocular cells is a photoreceptor cell. In another embodiment, the ocular cell is a ganglion cell. Such ocular cell is preferably an ocular cell from a mammal, including from a non-primate mammal (such as a mouse, a rat, a hamster, a guinea pig, a rabbit, and a dog) or a primate mammal (such as a human, a non-human primate, e.g., a monkey, a macaque and a chimpanzee). In one embodiment, the ocular cell is a human ocular cell (e.g. a human RPE cell, a human photoreceptor cell, a human ganglion cell, a human glial cell, a human Muller cell, a human bipolar cell, a human horizontal cell or a human amacrine cell). In another embodiment, the ocular cell is a mouse ocular cell. In another embodiment, the ocular cell is a rat ocular cell.

According to the present disclosure, the referred RNAi oligonucleotides can consist of at least one of the siRNA molecules above targeting the TSC2 gene, or can comprise at least one of the siRNA molecules targeting the TSC2 gene and a further siRNA molecule, preferably one of the siRNA molecules above targeting the TSC1 gene, which can be connected to one another by one or more moieties selected from a linker or a spacer, or even also from a branching point when three or more siRNA molecules are comprised in the RNAi oligonucleotide. When the RNAi oligonucleotide comprises more than one siRNA molecule connected or bound together, preferably, 2, 3, 4, 5, 6, 7 or 8 siRNA molecules, each double-stranded siRNA is independently connected to the linker, spacer or branching point at the 3 ' end or at the 5 ' end of the sense strand or the antisense strand. For example, WO2017132669A1, whose content is hereby incorporated by reference, discloses examples of linkers, spacers, branching moieties and different configurations for connecting nucleic acids, including siRNA, with such linkers and/or spacers and/or branching points. The linker or the spacer can be independently selected from an ethylene glycol chain, an alkyl chain, a peptide, RNA, DNA, a phosphate, a phosphonate, a phosphoramidate, an ester, an amide, a triazole, and combinations thereof, and taking into account that any carbon or oxygen atom of the linker or spacer can be optionally replaced with a nitrogen atom, bear a hydroxyl substituent, or bear an oxo substituent. The branching moiety can be a polyvalent organic species or a derivative of a polyvalent organic species, which comprise carbon and three or more valencies (i. e., points of attachment with moieties such as a linker, an spacer or a nucleic acid, such as the RNAi oligonucleotides or siRNA molecules of the present invention). Non-limiting examples of polyvalent organic species include triols (e.g. , glycerol, phloroglucinol, and the like), tetrols (e.g., ribose, pentaerythritol, 1 ,2,3,5-tetrahydroxybenzene, and the like), tri-carboxylic acids (e.g., citric acid, 1 ,3,5-cyclohexanetricarboxylic acid, trimesic acid, and the like), tetra-carboxylic acids (e.g. , ethyl enediaminetetraacetic acid, pyromellitic acid, and the like), tertiary amines (e.g. , tripropargylamine, triethanolamine, and the like), triamines (e.g. , diethyl enetriamine and the like), tetramines, and species comprising a combination of hydroxyl, thiol, amino, and/or carboxyl moieties (e.g., amino acids such as lysine, serine, cysteine, and the like), and a derivative of any of them. Depending on the combination of linkers, spacers and/or branching points used and its configuration, the RNAi oligonucleotide may comprise 2, 3, 4, 5, 6, 7 or 8 siRNA molecules, and preferably comprising 2 or 4 siRNA molecules, which can be or comprise:
- a same repeated siRNA molecule targeting any of the particular sequences of the TSC2 gene as defined above (that is, multiple copies of the same siRNA are present in the RNAi oligonucleotide); or
- an siRNA molecule targeting one of the particular sequences of the TSC2 gene, repeated or not, and another siRNA molecule targeting any other of the particular sequences of the TSC2 gene defined above, also repeated or not; or
- an siRNA molecule targeting one of the particular sequences of the TSC2 gene, repeated or not, and another siRNA molecule targeting any of the particular sequences of the TSC1 gene defined above, also repeated or not.

Alternatively, when the RNAi oligonucleotide comprises more than one siRNA molecule, preferably 2, 3, 4, 5, 6, 7 or 8 siRNA molecules, at least one, two or more of them can be as double-stranded siRNA not necessarily bound or connected to one another and being in an admixture of molecules constituting the RNAi oligonucleotide. For example, in certain embodiments, the RNAi oligonucleotide can constitute an admixture of at least a first siRNA molecule targeting the TSC2 gene and at least a second siRNA molecule targeting the other gene (TSC1) or a different target sequence of the same gene (for example, SEQ ID NO. 41 and SEQ ID NO. 42 of the TSC2 gene, as the targets for the first and second siRNAs). In certain other embodiments, the RNAi oligonucleotide can constitute an admixture of at least a first siRNA molecule targeting the TSC1 gene and at least a second siRNA molecule targeting the other gene (TSC2) or a different target sequence of the same gene (for example, SEQ ID NO. 41 or SEQ ID NO. 42 of the TSC2 gene). Optionally, the second siRNA in these cases may be also bound or connected to another siRNA molecule, which can be a siRNA against a target sequence of the same or a different gene.

Nucleic acid sequences cited herein are written in a 5' to 3' direction unless otherwise indicated. The term "nucleic acid" refers to either DNA or RNA or a modified form thereof comprising the purine or pyrimidine bases present in DNA (adenine "A", cytosine "C", guanine "G", thymine "T") or in RNA (adenine "A", cytosine "C", guanine "G", uracil "U"). Interfering RNAs provided herein may comprise "T" bases, for example at 3' ends, even though "T" bases do not naturally occur in RNA. In some cases, these bases may appear as "dT" or "X" to differentiate deoxyribonucleotides present in a chain of ribonucleotides.

The target sequence as defined above is described as a target DNA sequence as used for definition of transcript variants in databases used for the purposes of designing siRNAs and RNAi compounds, whereas the specific compounds to be used will be RNA sequences defined as such.

An expert in the field can access any target gene sequence through public data bases. For example, some GenBank Accession Numbers corresponding to human TSC2 mRNAs include NM_000548.5, NM_001077183.3, NM_001114382.3, NM_001318827.2, NM_001318829.2, NM_001318831.2, NM_001318832.2, NM_001363528.2, NM_001370404.1, NM_001370405.1, NM_001406663.1, NM_001406664.1, NM_001406665.1, NM_001406667.1, NM_001406668.1, NM_001406670.1, NM_001406671.1, NM_001406673.1, NM_001406675.1, NM_001406676.1, NM_001406677.1, NM_001406678.1, NM_001406679.1, NM_001406680.1, NM_001406681.1, NM_001406682.1, NM_001406683.1, NM_001406684.1, NM_001406685.1, NM_001406686.1, NM_001406687.1, NM_001406688.1, NM_001406689.1, NM_001406690.1, NM_001406691.1, NM_001406692.1, NM_001406693.1, NM_001406694.1, NM_001406695.1, NM_001406696.1, NM_001406697.1, NM_001406698.1, and NM_021055.3 (Gene ID: 7249). Furthermore, ENSEMBL (MBL-EBI/Wellcome Trust Sanger Institute) has the following TSC2 human Accession Numbers: ENST00000219476, ENST00000350773, ENST00000382538, ENST00000401874, ENST00000432909, ENST00000439117, ENST00000439673, ENST00000461648, ENST00000463601, ENST00000463808, ENST00000467949, ENST00000471143, ENST00000483020, ENST00000488675, ENST00000490108, ENST00000497886, ENST00000561695, ENST00000562474, ENST00000563346, ENST00000568238, ENST00000568366, ENST00000568454, ENST00000568566, ENST00000568692, ENST00000569110, ENST00000569930, ENST00000642206, ENST00000642365, ENST00000642561, ENST00000642728, ENST00000642791, ENST00000642797, ENST00000642812, ENST00000642936, ENST00000643088, ENST00000643120, ENST00000643149, ENST00000643177, ENST00000643298, ENST00000643426, ENST00000643533, ENST00000643745, ENST00000643946, ENST00000644043, ENST00000644135, ENST00000644222, ENST00000644278, ENST00000644329, ENST00000644335, ENST00000644399, ENST00000644417, ENST00000644665, ENST00000644722, ENST00000644847, ENST00000645024, ENST00000645186, ENST00000645192, ENST00000645552, ENST00000645591, ENST00000646388, ENST00000646464, ENST00000646557, ENST00000646634, ENST00000646674, ENST00000646823, ENST00000647042, ENST00000647180, ENST00000647234, and ENST00000647242. All this information is in the free-access Ensembl data base.

Analogously, GenBank Accession Numbers corresponding to human TSC1 mRNAs are NM_000368.5, NM_001162426.2, NM_001162427.2, NM_001362177.2, NM_001406592.1, NM_001406593.1, NM_001406594.1, NM_001406595.1, NM_001406596.1, NM_001406597.1, NM_001406598.1, NM_001406599.1, NM_001406600.1, NM_001406601.1, NM_001406602.1, NM_001406603.1, NM_001406604.1, NM_001406605.1, NM_001406606.1, NM_001406607.1, NM_001406608.1, NM_001406609.1, NM_001406610.1, NM_001406611.1, NM_001406612.1, NM_001406613.1, NM_001406614.1, NM_001406615.1, NM_001406616.1, NM_001406617.1, NM_001406618.1, NM_001406619.1, NM_001406620.1, NM_001406621.1, NM_001406622.1, NM_001406623.1, NM_001406624.1, NM_001406625.1, NM_001406626.1, NM_001406627.1, NM_001406628.1, NM_001406629.1, and NM_001406630.1 (Gene ID: 7248). Furthermore, ENSEMBL (MBL-EBI/Wellcome Trust Sanger Institute) has the following TSC1 human Accession Numbers: ENST00000298552, ENST00000403810, ENST00000440111, ENST00000461879, ENST00000475903, ENST00000490179, ENST00000493467, ENST00000545250, ENST00000642249, ENST00000642261, ENST00000642344, ENST00000642617, ENST00000642627, ENST00000642646, ENST00000642745, ENST00000642811, ENST00000642854, ENST00000643072, ENST00000643275, ENST00000643362, ENST00000643583, ENST00000643625, ENST00000643691, ENST00000643875, ENST00000644097, ENST00000644184, ENST00000644255, ENST00000644319, ENST00000644786, ENST00000644882, ENST00000644997, ENST00000645129, ENST00000645150, ENST00000645346, ENST00000645901, ENST00000645904, ENST00000646391, ENST00000646440, ENST00000646625, ENST00000646788, ENST00000646831, ENST00000647078, ENST00000647262, ENST00000647279, ENST00000647462, ENST00000647506, and ENST00000647534. All this information is in the free-access Ensembl data base.

Said above target sequences or regions identified by the present invention comprise or consist of at least one sequence selected from SEQ ID NO. 41 to SEQ ID NO. 64 for the TSC2 gene, or of at least one sequence selected from SEQ ID NO. 1 to SEQ ID NO. 40 for the TSC1 gene. These sequences present 100% homology between at least the following species: *Homo sapiens, Macaca mulatta, Rattus norvergicus, Canis Lupus familiaris* and *Mus musculus.* Depending on the nucleotide in the 3'-end of its sequence, SEQ ID NO. 41 can be 5'-CAAGCTTGTCACTGTGACG-3' (SEQ ID NO. 1290) or preferably 5'-CAAGCTTGTCACTGTGACA-3' (SEQ ID NO. 1287). Similarly, SEQ ID NO. 46 can be 5'-ACCAGCTGGCTGATGAGCC-3' (SEQ ID NO. 1291) or preferably 5'-ACCAGCTGGCTGATGAGCT-3' (SEQ ID NO. 1288); while SEQ ID NO. 48 can be 5'-CAAAACCTGGCTGGTTGGG-3' (SEQ ID NO. 1292), or preferably 5'-CAAAACCTGGCTGGTTGGA-3' (SEQ ID NO. 1289).

In preferred embodiments of certain aspects of the invention, said target regions of the TSC2 gene comprise or consist of a sequence selected from SEQ ID NO. 41, SEQ ID NO. 42, SEQ ID NO. 43, SEQ ID NO. 44, SEQ ID NO. 45, SEQ ID NO. 46, SEQ ID NO. 47, SEQ ID NO. 48 and SEQ ID NO. 49. In other preferred embodiments, said target regions comprise or consist of a sequence selected from SEQ ID NO. 41 and SEQ ID NO. 42. In more preferred embodiments, the target regions are those which are targeted by any or some of the siRNA molecules of the present invention having a high gene silencing efficiency (both on the mRNA expression levels of the TCS2 gene or on protein levels of protein TSC2 or tuberin) in cells of different species, preferably of 60% and above, or 70% and above, or 80% and above, or 90% and above or 95% and above, in cells of at least two of the species above mentioned having 100% homology. More preferably, the target regions are those that are targeted by any or some of the siRNA molecules having gene silencing efficiency higher than 80%, 81%, 82%, 83%, 84%, 85%, 86%, 87%, 88%, 89%, 90%, 91%, 92%, 93%, 94%, 95%, 96%, 97%, 98% or 99% in cells of at least two different species, or in cells of at least three different species, or in cells of four different species having 100% homology in the target region of the TSC2 gene, and even more preferably in cells of all different species above mentioned having 100% homology.

In preferred embodiments of certain aspects of the invention, said target regions of the TSC1 gene comprise or consist of a sequence selected from SEQ ID NO. 1, SEQ ID NO. 2, SEQ ID NO. 3, SEQ ID NO. 4, SEQ ID NO. 5, SEQ ID NO. 6, SEQ ID NO. 7 and SEQ ID NO. 8 (and preferably from SEQ ID NO. 1, SEQ ID NO. 2, SEQ ID NO. 3, SEQ ID NO. 7 and SEQ ID NO. 8). In other preferred embodiments, said target regions comprise or consist of a sequence selected from SEQ ID NO.2, SEQ ID NO. 1, and SEQ ID NO. 7. In other preferred embodiments, said target regions comprise or consist of a sequence selected from SEQ ID NO.3, or SEQ ID NO. 8. In more preferred embodiments, the target regions are those which are targeted by any or some of the siRNA molecules of the present invention having a high gene silencing efficiency (both on the mRNA expression levels of the TCS1 gene or on protein levels of protein TSC1 or hamartin) in cells of different species, preferably of 60% and above, or 70% and above, or 80% and above, or 90% and above or 95% and above, in cells of at least two of the species above mentioned having 100% homology. More preferably, the target regions are those that are targeted by any or some of the siRNA molecules having gene silencing efficiency higher than 80%, 81%, 82%, 83%, 84%, 85%, 86%, 87%, 88%, 89%, 90%, 91%, 92%, 93%, 94%, 95%, 96%, 97%, 98% or 99% in cells of at least two different species, or in cells of at least three different species, or in cells of four different species having 100% homology in the target region of the TSC1 gene, and even more preferably in cells of all different species above mentioned having 100% homology.

In other preferred embodiments, the target regions are those which are targeted by any or some of the siRNA molecules of the present invention having a long-lasting gene silencing efficiency in different cells of at least one species, preferably of 48 h and above, or 72 h and above, or 96 h and above, or 120 h and above, in at least two cell types of one, two or more species of the above mentioned having 100% homology. More preferably, the target regions are those that are targeted by any or some of the siRNA molecules having lasting silencing effect of at least 48 hours or 72 hours in at least two different cell types of one or two different species having 100% homology in the target region of the TSC2 gene or the TSC1 gene, and even more preferably in cells of all different species above mentioned having 100% homology.

In the RNAi field, when in vitro studies demonstrate that a human siRNA molecule or RNAi oligonucleotide is not able to induce knock-down of the animal model gene, a surrogate compound (animal-active analogue) is synthetized to analyze the efficacy of the siRNA or RNAi oligonucleotide in the relevant animal model. This surrogate is designed against the same region as the human RNAi oligonucleotide or siRNA molecule, thus the two oligonucleotides or siRNAs have the same sequence except for a few nucleotides, depending on the homology between the human and the animal target gene. This approach has been widely used for development of other oligonucleotides, specifically for toxicology and efficacy studies (Kornbrust D et al 2013).

Consequently, a siRNA molecule or RNAi oligonucleotide according to certain aspects of the present invention will preferably comprise a double stranded RNA molecule, whose antisense strand will comprise an RNA sequence substantially complementary to at least one sequence consisting of SEQ ID NO. 41 to SEQ ID NO. 64 of the TSC2 gene, and whose sense strand will comprise an RNA sequence having at least the first 14 nucleotides from the 5'-end or from the 3-end complementary to the antisense strand, wherein both strands are hybridised by standard base pairing between nucleotides. More preferably, a siRNA molecule according to certain aspects of the present invention will preferably comprise a double stranded RNA molecule, whose antisense strand will comprise an RNA sequence substantially complementary to any of sequences SEQ ID NO. 41 to SEQ ID NO. 49, and even more preferably the antisense strand comprises or consists of an RNA sequence substantially complementary to at least one of the sequences from the group consisting of SEQ ID NO. 41 to SEQ ID NO. 45 and SEQ ID NO. 47 to SEQ ID NO. 49, and even more preferably the antisense strand comprises or consists of an RNA sequence substantially complementary to SEQ ID NO.41 or SEQ ID NO. 42.

Within the meaning of the present invention "substantially complementary" to a target mRNA sequence may also be understood as having between an 85% to 100% complementarity to said target sequence over the length of the siRNA strand. However, the complementarity between sense and antisense strands of a siRNA within the present invention may be in the range of from 70% to 100% over the length of the double stranded portion of the siRNA. "Identity" as is known by one of ordinary skill in the art is the degree of sequence relatedness between nucleotide sequences as determined by matching the order and identity of nucleotides between sequences. In one embodiment the antisense strand of an siRNA having 85%, 86%, 87%, 88%, 89%, 90%, 91%, 92%, 93%, 94%, 95%, 96%, 97%, 98% or 99% complementarity to the target mRNA sequence are considered substantially complementary and may be used in the present invention. The percentage of complementarity describes the percentage of contiguous nucleotides in a first nucleic acid molecule that can base pair in the Watson-Crick sense with a set of contiguous nucleotides in a second nucleic acid molecule. In a preferred embodiment, the antisense strand of the siRNA is 100% complementary to the target mRNA sequence, and the sense strand is 100% complementary to the antisense strand over the double stranded portion of the siRNA. The double stranded portion of the siRNA molecule comprises at least 14 base pairs, preferably between 14 and 21 base pairs, and more preferably comprises 14 base pairs, 15 base pairs, 16 base pairs, 17 base pairs, 18 base pairs, 19 base pairs. In other preferred embodiments, the antisense strand of the siRNA can be 95% complementary to the target mRNA sequence (for example, one nucleotide of a 19 nucleotide-long antisense strand, preferably the first nucleotide of the 5'-end, is not complementary to the corresponding nucleotide of the targeted mRNA sequence of the same length), and the sense strand is 100% complementary to the antisense strand over the double stranded portion of the siRNA. The siRNA molecule may also include unpaired overhangs, for example, 3' mononucleotide overhangs, preferably U or dT, and 3' dinucleotide overhangs, preferably UU or dTdT.

Preferred antisense strands of the siRNA molecule that target sequences SEQ ID NO. 41 to SEQ ID NO. 64 of TSC2 gene or the corresponding mRNA comprise or consist of oligonucleotide sequences having 19, 20 or 21 nucleotides from one of the nucleotide sequences defined in the group consisting of: SEQ ID NO. 105 to SEQ ID NO. 128, SEQ ID NO. 385 to SEQ ID NO. 416, SEQ ID NO. 737 to SEQ ID NO. 747, SEQ ID NO. 880 to SEQ ID NO. 907, and SEQ ID NO. 1293 to SEQ ID NO. 1295, which correspond to the RNA sequences complementary to the targeted regions of the TSC2 mRNAs accessible to the siRNAs or RNAi oligonucleotides of the invention. Preferred sense strands of the siRNA molecule which are complementary to these antisense strands comprise or consist of oligonucleotide sequences having 14, 15, 16, 17, 18, 19, 20 or 21 nucleotides from one of the nucleotide sequences defined in the group consisting of: SEQ ID NO. 169 to SEQ ID NO. 192, SEQ ID NO. 609 to SEQ ID NO. 640, SEQ ID NO. 844 to SEQ ID NO. 854, SEQ ID NO. 933 to SEQ ID NO. 960, and SEQ ID NO. 1296 to SEQ ID NO. 1298, respectively. Non-limiting examples of siRNA molecules having such sense and antisense sequences are shown in FIG. 2, FIG. 3 and FIG. 4. Other non-limiting examples include siRNA molecules such as siRNA ID 06013 (sense sequence of SEQ ID NO. 1296: 5'-CAAGCUUGUCACUGUGACG-3'; antisense sequence of SEQ ID NO. 1293: 5'-CGUCACAGUGACAAGCUUG-3'), siRNA ID 06015 (sense sequence of SEQ ID NO. 1297: 5'-ACCAGCUGGCUGAUGAGCC-3'; antisense sequence of SEQ ID NO. 1294: 5'-GGCUCAUCAGCCAGCUGGU-3'), and siRNA ID 06017 (sense sequence of SEQ ID NO. 1298: 5'-CAAAACCUGGCUGGUUGGG-3'; antisense sequence of SEQ ID NO. 1295: 5'-CCCAACCAGCCAGGUUUUG-3').

Additionally, preferred antisense strands of the siRNA molecule that specifically target sequences SEQ ID NO. 41 to SEQ ID NO. 64 of TSC2 gene or the corresponding mRNA may also include others that comprise or consist of oligonucleotide sequences having 18 nucleotides from one of the nucleotide sequences defined in the group consisting of: SEQ ID NO. 1339 (5'-YGUCACAGUGACAAGCUU-3'), SEQ ID NO. 1340 (5'-CAACCAGCCAGGUUUUGG-3'), SEQ ID NO. 1341 (5'-CUCAUCAGCCAGCUGGUG-3'), SEQ ID NO. 1342 (5'-UGUCUUUCCUGCACUGCA-3'), SEQ ID NO. 1343 (5'-UGAACCACAUGGCUAUGA-3'), SEQ ID NO. 1344 (5'-RGCUCAUCAGCCAGCUGG-3'), SEQ ID NO. 1345 (5'-GUCACAGUGACAAGCUUG-3'), SEQ ID NO. 1346 (5'-YCCAACCAGCCAGGUUUU-3'), SEQ ID NO. 1347 (5'-AACUUGGAGGGGUUGGUG-3'), SEQ ID NO. 1348 (5'-ACGUCCAGCACCUUGAUG-3'), SEQ ID NO. 1349 (5'-AUGUCUUUCCUGCACUGC-3'), SEQ ID NO. 1350 (5'-UAGAACUGCCUGUUGAUG-3'), SEQ ID NO. 1351 (5'-GCUCAUCAGCCAGCUGGU-3'), SEQ ID NO. 1352 (5'-ACCAGCCAGGUUUUGGUC-3'), SEQ ID NO. 1353 (5'-AACCAGCCAGGUUUUGGU-3'), SEQ ID NO. 1354 (5'-CCAACCAGCCAGGUUUUG-3'), SEQ ID NO. 1355 (5'-GACACCUGCUGCCCAGCC-3'), SEQ ID NO. 1356 (5'-UGCCAGCAGUAGGUGAAC-3'), SEQ ID NO. 1357 (5'-AUGCCAGCAGUAGGUGAA-3'), SEQ ID NO. 1358 (5'-UCCUUGGUGGGCAUCAGG-3'), SEQ ID NO. 1359 (5'-AUCCUUGGUGGGCAUCAG-3'), SEQ ID NO. 1360 (5'-CGGAAGAGCAGGGAGUAG-3'), SEQ ID NO. 1361 (5'-ACGGAAGAGCAGGGAGUA-3') and SEQ ID NO. 1362 (5'-GGGGCUGAGCGGGUUCUC-3'), which correspond to the RNA sequences complementary to the 18 first nucleotides from the 5'-end of the DNA targeted regions defined by SEQ ID NO. 41 to SEQ ID NO. 64 of the TSC2 gene. Preferred sense strands of the siRNA molecule which are complementary to these antisense strands comprise or consist of oligonucleotide sequences having 14, 15, 16, 17 or 18 nucleotides, and preferably 18 nucleotides, from one of the nucleotide sequences defined in the group consisting of: SEQ ID NO. 1403 (5'-AAGCUUGUCACUGUGACR-3'), SEQ ID NO. 1404 (5'-CCAAAACCUGGCUGGUUG-3'), SEQ ID NO. 1405 (5'-CACCAGCUGGCUGAUGAG-3'), SEQ ID NO. 1406 (5'-UGCAGUGCAGGAAAGACA-3'), SEQ ID NO. 1407 (5'-UCAUAGCCAUGUGGUUCA-3'), SEQ ID NO. 1408 (5'-CCAGCUGGCUGAUGAGCY-3'), SEQ ID NO. 1409 (5'-CAAGCUUGUCACUGUGAC-3'), SEQ ID NO. 1410 (5'-AAAACCUGGCUGGUUGGR-3'), SEQ ID NO. 1411 (5'-CACCAACCCCUCCAAGUU-3'), SEQ ID NO. 1412 (5'-CAUCAAGGUGCUGGACGU-3'), SEQ ID NO. 1413 (5'-GCAGUGCAGGAAAGACAU-3'), SEQ ID NO. 1414 (5'-CAUCAACAGGCAGUUCUA-3'), SEQ ID NO. 1415 (5'-ACCAGCUGGCUGAUGAGC-3'), SEQ ID NO. 1416 (5'-GACCAAAACCUGGCUGGU-3'), SEQ ID NO. 1417 (5'-ACCAAAACCUGGCUGGUU-3'), SEQ ID NO. 1418 (5'-CAAAACCUGGCUGGUUGG-3'), SEQ ID NO. 1419 (5'-GGCUGGGCAGCAGGUGUC-3'), SEQ ID NO. 1420 (5'-GUUCACCUACUGCUGGCA-3'), SEQ ID NO. 1421 (5'-UUCACCUACUGCUGGCAU-3'), SEQ ID NO. 1422 (5'-CCUGAUGCCCACCAAGGA-3'), SEQ ID NO. 1423 (5'-CUGAUGCCCACCAAGGAU-3'), SEQ ID NO. 1424 (5'-CUACUCCCUGCUCUUCCG-3'), SEQ ID NO. 1425 (5'-UACUCCCUGCUCUUCCGU-3'), SEQ ID NO. 1426 (5'-GAGAACCCGCUCAGCCCC-3'), respectively. For example, non-limiting embodiments of siRNA molecules having such sense and antisense sequences would include molecules such as siRNA ID 06012 (sense sequence of SEQ ID NO. 1409: 5'-CAAGCUUGUCACUGUGAC-3'; antisense sequence of SEQ ID NO. 1345: 5'-GUCACAGUGACAAGCUUG-3'), siRNA ID 06014 (sense sequence of SEQ ID NO. 1415: 5'-ACCAGCUGGCUGAUGAGC-3'; antisense sequence of SEQ ID NO. 1351: 5'-GCUCAUCAGCCAGCUGGU-3') or siRNA ID 06016 (sense sequence of SEQ ID NO. 1418: 5'-CAAAACCUGGCUGGUUGG-3'; antisense sequence of SEQ ID NO. 1354: 5'-CCAACCAGCCAGGUUUUG-3').

In embodiments, an antisense strand of the siRNA molecule targeting the sequence SEQ ID NO. 41 of the TSC2 gene or its corresponding mRNA comprises or consists of an oligonucleotide sequence having 19, 20 or 21 nucleotides from one of the nucleotide sequences defined in the group consisting of SEQ ID NO. 105, SEQ ID NO. 880, SEQ ID NO. 884, SEQ ID NO. 888, SEQ ID NO. 892 - SEQ ID NO. 894, SEQ ID NO. 896. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 169, SEQ ID NO. 933, SEQ ID NO. 937, SEQ ID NO. 941, SEQ ID NO. 945 - SEQ ID NO. 947, SEQ ID NO. 949, respectively.

In embodiments, an antisense strand of the siRNA molecule targeting the sequence SEQ ID NO. 41 of the TSC2 gene or its corresponding mRNA comprises or consists of an oligonucleotide sequence having 18 or 19 nucleotides from one of the nucleotide sequences defined in the group consisting of SEQ ID NO. 1293 (5'-CGUCACAGUGACAAGCUUG-3') and SEQ ID NO. 1345 (5'-GUCACAGUGACAAGCUUG-3'). In embodiments, the sense strand of the siRNA molecule which is complementary to this TSC2 antisense strand comprises or consists of an oligonucleotide sequence having 14, 15, 16, 17, 18 or 19 nucleotides from the nucleotide sequences defined in the group consisting of SEQ ID NO. 1296 (5'-CAAGCUUGUCACUGUGACG-3') and SEQ ID NO. 1409 (5'-CAAGCUUGUCACUGUGAC-3'), respectively.

In embodiments, an antisense strand of the siRNA molecule targeting the sequence SEQ ID NO. 42 of the TSC2 gene or its corresponding mRNA comprises or consists of an oligonucleotide sequence having 19, 20 or 21 nucleotides from one of the nucleotide sequences defined in the group consisting of SEQ ID NO. 106, SEQ ID NO. 385 - SEQ ID NO. 392, SEQ ID NO. 737 - SEQ ID NO. 741, SEQ ID NO. 881, SEQ ID NO. 885, SEQ ID NO. 889, SEQ ID NO. 895, SEQ ID NO. 897 - SEQ ID NO. 903. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 170, SEQ ID NO. 609 - SEQ ID NO. 616, SEQ ID NO. 844 - SEQ ID NO. 848, SEQ ID NO. 934, SEQ ID NO. 938, SEQ ID NO. 942, SEQ ID NO. 948, SEQ ID NO. 950 - SEQ ID NO. 956, respectively.

In embodiments, an antisense strand of the siRNA molecule targeting the sequence SEQ ID NO. 43 of the TSC2 gene or its corresponding mRNA comprises or consists of an oligonucleotide sequence having 19, 20 or 21 nucleotides from one of the nucleotide sequences defined in the group consisting of SEQ ID NO. 107. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 171, respectively.

In embodiments, an antisense strand of the siRNA molecule targeting the sequence SEQ ID NO. 44 of the TSC2 gene or its corresponding mRNA comprises or consists of an oligonucleotide sequence having 19, 20 or 21 nucleotides from one of the nucleotide sequences defined in the group consisting of SEQ ID NO. 108, SEQ ID NO. 882, SEQ ID NO. 886, SEQ ID NO. 890, SEQ ID NO. 904, SEQ ID NO. 906. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 172, SEQ ID NO. 935, SEQ ID NO. 939, SEQ ID NO. 943, SEQ ID NO. 957, SEQ ID NO. 959, respectively.

In embodiments, an antisense strand of the siRNA molecule targeting the sequence SEQ ID NO. 45 of the TSC2 gene or its corresponding mRNA comprises or consists of an oligonucleotide sequence having 19, 20 or 21 nucleotides from one of the nucleotide sequences defined in the group consisting of SEQ ID NO. 109, SEQ ID NO: 883, SEQ ID NO. 887, SEQ ID NO. 891, SEQ ID NO. 905, SEQ ID NO. 907. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 173, SEQ ID NO. 936, SEQ ID NO. 940, SEQ ID NO. 944, SEQ ID NO. 958, SEQ ID NO. 960, respectively.

In embodiments, an antisense strand of the siRNA molecule targeting the sequence SEQ ID NO. 46 of the TSC2 gene or its corresponding mRNA comprises or consists of an oligonucleotide sequence having 19, 20 or 21 nucleotides from one of the nucleotide sequences defined in the group consisting of SEQ ID NO. 110, SEQ ID NO. 393 - SEQ ID NO. 400. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 174, SEQ ID NO. 617 - SEQ ID NO. 624, respectively.

In embodiments, an antisense strand of the siRNA molecule targeting the sequence SEQ ID NO. 46 of the TSC2 gene or its corresponding mRNA comprises or consists of an oligonucleotide sequence having 18 or 19 nucleotides from one of the nucleotide sequences defined in the group consisting of SEQ ID NO. 1294 (5'-GGCUCAUCAGCCAGCUGGU-3') and SEQ ID NO. 1351 (5'-GCUCAUCAGCCAGCUGGU-3'). In embodiments, the sense strand of the siRNA molecule which is complementary to this TSC2 antisense strand comprises or consists of an oligonucleotide sequence having 14, 15, 16, 17, 18 or 19 nucleotides from the nucleotide sequences defined in the group consisting of SEQ ID NO. 1297 (5'-ACCAGCUGGCUGAUGAGCC-3') and SEQ ID NO. 1415 (5'-ACCAGCUGGCUGAUGAGC-3'), respectively.

In embodiments, an antisense strand of the siRNA molecule targeting the sequence SEQ ID NO. 47 of the TSC2 gene or its corresponding mRNA comprises or consists of an oligonucleotide sequence having 19, 20 or 21 nucleotides from one of the nucleotide sequences defined in the group consisting of SEQ ID NO. 111. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 175, respectively.

In embodiments, an antisense strand of the siRNA molecule targeting the sequence SEQ ID NO. 48 of the TSC2 gene or its corresponding mRNA comprises or consists of an oligonucleotide sequence having 19, 20 or 21 nucleotides from one of the nucleotide sequences defined in the group consisting of SEQ ID NO. 112, SEQ ID NO. 401 - SEQ ID NO. 408. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 176, SEQ ID NO. 625 - SEQ ID NO. 632, respectively.

In embodiments, an antisense strand of the siRNA molecule targeting the sequence SEQ ID NO. 48 of the TSC2 gene or its corresponding mRNA comprises or consists of an oligonucleotide sequence having 18 or 19 nucleotides from one of the nucleotide sequences defined in the group consisting of SEQ ID NO. 1295 (5'-CCCAACCAGCCAGGUUUUG-3') and SEQ ID NO. 1354 (5'-CCAACCAGCCAGGUUUUG-3'). In embodiments, the sense strand of the siRNA molecule which is complementary to this TSC2 antisense strand comprises or consists of an oligonucleotide sequence having 14, 15, 16, 17, 18 or 19 nucleotides from the nucleotide sequences defined in the group consisting of SEQ ID NO. 1298 (5'-CAAAACCUGGCUGGUUGGG-3') and SEQ ID NO. 1418 (5'-CAAAACCUGGCUGGUUGG-3'), respectively.

In embodiments, an antisense strand of the siRNA molecule targeting the sequence SEQ ID NO. 49 of the TSC2 gene or its corresponding mRNA comprises or consists of an oligonucleotide sequence having 19, 20 or 21 nucleotides from one of the nucleotide sequences defined in the group consisting of SEQ ID NO. 113. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 177, respectively.

In embodiments, an antisense strand of the siRNA molecule targeting the sequence SEQ ID NO. 50 of the TSC2 gene or its corresponding mRNA comprises or consists of an oligonucleotide sequence having 19, 20 or 21 nucleotides from one of the nucleotide sequences defined in the group consisting of SEQ ID NO. 114. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 178, respectively.

In embodiments, an antisense strand of the siRNA molecule targeting the sequence SEQ ID NO. 51 of the TSC2 gene or its corresponding mRNA comprises or consists of an oligonucleotide sequence having 19, 20 or 21 nucleotides from one of the nucleotide sequences defined in the group consisting of SEQ ID NO. 115. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 179, respectively.

In embodiments, an antisense strand of the siRNA molecule targeting the sequence SEQ ID NO. 52 of the TSC2 gene or its corresponding mRNA comprises or consists of an oligonucleotide sequence having 19, 20 or 21 nucleotides from one of the nucleotide sequences defined in the group consisting of SEQ ID NO. 116. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 180, respectively.

In embodiments, an antisense strand of the siRNA molecule targeting the sequence SEQ ID NO. 53 of the TSC2 gene or its corresponding mRNA comprises or consists of an oligonucleotide sequence having 19, 20 or 21 nucleotides from one of the nucleotide sequences defined in the group consisting of SEQ ID NO. 117, SEQ ID NO. 742 - SEQ ID NO. 743. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 181, SEQ ID NO. 849 - SEQ ID NO. 850, respectively.

In embodiments, an antisense strand of the siRNA molecule targeting the sequence SEQ ID NO. 54 of the TSC2 gene or its corresponding mRNA comprises or consists of an oligonucleotide sequence having 19, 20 or 21 nucleotides from one of the nucleotide sequences defined in the group consisting of SEQ ID NO. 118. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 182, respectively.

In embodiments, an antisense strand of the siRNA molecule targeting the sequence SEQ ID NO. 55 of the TSC2 gene or its corresponding mRNA comprises or consists of an oligonucleotide sequence having 19, 20 or 21 nucleotides from one of the nucleotide sequences defined in the group consisting of SEQ ID NO. 119, SEQ ID NO. 744 - SEQ ID NO. 745. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 183, SEQ ID NO. 851 - SEQ ID NO. 852, respectively.

In embodiments, an antisense strand of the siRNA molecule targeting the sequence SEQ ID NO. 56 of the TSC2 gene or its corresponding mRNA comprises or consists of an oligonucleotide sequence having 19, 20 or 21 nucleotides from one of the nucleotide sequences defined in the group consisting of SEQ ID NO. 120, SEQ ID NO. 409 - SEQ ID NO. 416, SEQ ID NO. 746 - SEQ ID NO. 747. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 184, SEQ ID NO. 633 - SEQ ID NO. 640, SEQ ID NO. 853 - SEQ ID NO. 854, respectively.

In embodiments, an antisense strand of the siRNA molecule targeting the sequence SEQ ID NO. 57 of the TSC2 gene or its corresponding mRNA comprises or consists of an oligonucleotide sequence having 19, 20 or 21 nucleotides from one of the nucleotide sequences defined in the group consisting of SEQ ID NO. 121. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 185, respectively.

In embodiments, an antisense strand of the siRNA molecule targeting the sequence SEQ ID NO. 58 of the TSC2 gene or its corresponding mRNA comprises or consists of an oligonucleotide sequence having 19, 20 or 21 nucleotides from one of the nucleotide sequences defined in the group consisting of SEQ ID NO. 122. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 186, respectively.

In embodiments, an antisense strand of the siRNA molecule targeting the sequence SEQ ID NO. 59 of the TSC2 gene or its corresponding mRNA comprises or consists of an oligonucleotide sequence having 19, 20 or 21 nucleotides from one of the nucleotide sequences defined in the group consisting of SEQ ID NO. 123. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 187, respectively.

In embodiments, an antisense strand of the siRNA molecule targeting the sequence SEQ ID NO. 60 of the TSC2 gene or its corresponding mRNA comprises or consists of an oligonucleotide sequence having 19, 20 or 21 nucleotides from one of the nucleotide sequences defined in the group consisting of SEQ ID NO. 124. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 188, respectively.

In embodiments, an antisense strand of the siRNA molecule targeting the sequence SEQ ID NO. 61 of the TSC2 gene or its corresponding mRNA comprises or consists of an oligonucleotide sequence having 19, 20 or 21 nucleotides from one of the nucleotide sequences defined in the group consisting of SEQ ID NO. 125. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 189, respectively.

In embodiments, an antisense strand of the siRNA molecule targeting the sequence SEQ ID NO. 62 of the TSC2 gene or its corresponding mRNA comprises or consists of an oligonucleotide sequence having 19, 20 or 21 nucleotides from one of the nucleotide sequences defined in the group consisting of SEQ ID NO. 126. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 190, respectively.

In embodiments, an antisense strand of the siRNA molecule targeting the sequence SEQ ID NO. 63 of the TSC2 gene or its corresponding mRNA comprises or consists of an oligonucleotide sequence having 19, 20 or 21 nucleotides from one of the nucleotide sequences defined in the group consisting of SEQ ID NO. 127. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 191, respectively.

In embodiments, an antisense strand of the siRNA molecule targeting the sequence SEQ ID NO. 64 of the TSC2 gene or its corresponding mRNA comprises or consists of an oligonucleotide sequence having 19, 20 or 21 nucleotides from one of the nucleotide sequences defined in the group consisting of SEQ ID NO. 128. In embodiments, the sense strand of the siRNA molecule which is complementary to this TSC2 antisense strand comprises or consists of an oligonucleotide sequence having 14, 15, 16, 17, 18, 19, 20 or 21 nucleotides from the nucleotide sequences defined in the group consisting of SEQ ID NO. 192, respectively.

Preferred antisense strands of the siRNA molecule that target sequences SEQ ID NO. 1 to SEQ ID NO. 40 of TSC1 gene or the corresponding mRNA comprise or consist of oligonucleotide sequences having 19, 20 or 21 nucleotides from one of the nucleotide sequences defined in the group consisting of: SEQ ID NO. 65 to SEQ ID NO. 104, SEQ ID NO. 193 to SEQ ID NO. 384, SEQ ID NO. 641 to SEQ ID NO. 736 and SEQ ID NO. 855 to SEQ ID NO. 879, which correspond to the RNA sequences complementary to the regions of the TSC1 mRNAs accessible to the siRNAs or RNAi oligonucleotides of the invention. Preferred sense strands of the siRNA molecules which are complementary to these TSC1 antisense strands comprise or consist of oligonucleotide sequences having 14, 15, 16, 17, 18, 19, 20 or 21 nucleotides from one of the nucleotide sequences defined in the group consisting of: SEQ ID NO. 129 to SEQ ID NO. 168, SEQ ID NO. 417 to SEQ ID NO. 608, SEQ ID NO. 748 to SEQ ID NO. 843 and SEQ ID NO. 908 to SEQ ID NO. 932, respectively. Non-limiting examples of siRNA molecules having such sense and antisense sequences are shown in FIG. 2, FIG. 3 and FIG. 4.

Additionally, preferred antisense strands of the siRNA molecule that specifically target sequences SEQ ID NO. 1 to SEQ ID NO. 40 of TSC1 gene or the corresponding mRNA may also include others that comprise or consist of oligonucleotide sequences having 18 nucleotides from one of the nucleotide sequences defined in the group consisting of: SEQ ID NO. 1299 (5'-GAUCCAGUCACUAAUUCC-3'), SEQ ID NO. 1300 (5'-UCCAGAGAGAUUUUGGCA-3'), SEQ ID NO. 1301 (5'-UGAAUUCGCACAUGCUCC-3'), SEQ ID NO. 1302 (5'-AGAGAGAUUUUGGCACAC-3'), SEQ ID NO. 1303 (5'-GAGAGAUUUUGGCACACU-3'), SEQ ID NO. 1304 (5'-CAGAGAGAUUUUGGCACA-3'), SEQ ID NO. 1305 (5'-GGUCCUUGGAUCCAGUCA-3'), SEQ ID NO. 1306 (5'-AAUUCGCACAUGCUCCAU-3'), SEQ ID NO. 1307 (5'-UCUUCCACCUUCGAGGGU-3'), SEQ ID NO. 1308 (5'-AUCCAGUCACUAAUUCCG-3'), SEQ ID NO. 1309 (5'-AUGAGUCUCAUUGUAGUC-3'), SEQ ID NO. 1310 (5'-CACAUGCUCCAUCAUUGG-3'), SEQ ID NO. 1311 (5'-GGAUCCAGUCACUAAUUC-3'), SEQ ID NO. 1312 (5'-UGACCACUUCUUCAAAAG-3'), SEQ ID NO. 1313 (5'-CCAGAGAGAUUUUGGCAC-3'), SEQ ID NO. 1314 (5'-CCUUGGAUCCAGUCACUA-3'), SEQ ID NO. 1315 (5'-CUUGGAUCCAGUCACUAA-3'), SEQ ID NO. 1316 (5'-AAGGUGGUCUUUCUUGGC-3'), SEQ ID NO. 1317 (5'-UAUGGGAGUAAAGGCUUG-3'), SEQ ID NO. 1318 (5'-AGUAAAGGCUUGCUUUGG-3'), SEQ ID NO. 1319 (5'-UCUAUGGGAGUAAAGGCU-3'), SEQ ID NO. 1320 (5'-UUCAAGAACUCCAUCUGC-3'), SEQ ID NO. 1321 (5'-AGUCUCAUUGUAGUCCAU-3'), SEQ ID NO. 1322 (5'-UGGAUCCAGUCACUAAUU-3'), SEQ ID NO. 1323 (5'-UUGGAUCCAGUCACUAAU-3'), SEQ ID NO. 1324 (5'-UCCUUGGAUCCAGUCACU-3'), SEQ ID NO. 1325 (5'-GUCCUUGGAUCCAGUCAC-3'), SEQ ID NO. 1326 (5'-UGGUCCUUGGAUCCAGUC-3'), SEQ ID NO. 1327 (5'-GCACAUGCUCCAUCAUUG-3'), SEQ ID NO. 1328 (5'-CGCACAUGCUCCAUCAUU-3'), SEQ ID NO. 1329 (5'-UCGCACAUGCUCCAUCAU-3'), SEQ ID NO. 1330 (5'-UUCGCACAUGCUCCAUCA-3'), SEQ ID NO. 1331 (5'-AUUCGCACAUGCUCCAUC-3'), SEQ ID NO. 1332 (5'-GAAUUCGCACAUGCUCCA-3'), SEQ ID NO. 1333 (5'-ACCUUCGAGGGUCCAGUU-3'), SEQ ID NO. 1334 (5'-CACCUUCGAGGGUCCAGU-3'), SEQ ID NO. 1335 (5'-CCACCUUCGAGGGUCCAG-3'), SEQ ID NO. 1336 (5'-UCCACCUUCGAGGGUCCA-3'), SEQ ID NO. 1337 (5'-UUCCACCUUCGAGGGUCC-3') and SEQ ID NO. 1338 (5'-CUUCCACCUUCGAGGGUC-3'), which correspond to the RNA sequences complementary to the 18 first nucleotides from the 5'-end of the DNA targeted regions defined by SEQ ID NO. 1 to SEQ ID NO. 40 of the TSC1 gene. Preferred sense strands of the siRNA molecule which are complementary to these antisense strands comprise or consist of oligonucleotide sequences having 14, 15, 16, 17 or 18 nucleotides, and preferably 18 nucleotides, from one of the nucleotide sequences defined in the group consisting of: SEQ ID NO. 1363 (5'-GGAAUUAGUGACUGGAUC-3'), SEQ ID NO. 1364 (5'-UGCCAAAAUCUCUCUGGA-3'), SEQ ID NO. 1365 (5'-GGAGCAUGUGCGAAUUCA-3'), SEQ ID NO. 1366 (5'-GUGUGCCAAAAUCUCUCU-3'), SEQ ID NO. 1367 (5'-AGUGUGCCAAAAUCUCUC-3'), SEQ ID NO. 1368 (5'-UGUGCCAAAAUCUCUCUG-3'), SEQ ID NO. 1369 (5'-UGACUGGAUCCAAGGACC-3'), SEQ ID NO. 1370 (5'-AUGGAGCAUGUGCGAAUU-3'), SEQ ID NO. 1371 (5'-ACCCUCGAAGGUGGAAGA-3'), SEQ ID NO. 1372 (5'-CGGAAUUAGUGACUGGAU-3'), SEQ ID NO. 1373 (5'-GACUACAAUGAGACUCAU-3'), SEQ ID NO. 1374 (5'-CCAAUGAUGGAGCAUGUG-3'), SEQ ID NO. 1375 (5'-GAAUUAGUGACUGGAUCC-3'), SEQ ID NO. 1376 (5'-CUUUUGAAGAAGUGGUCA-3'), SEQ ID NO. 1377 (5'-GUGCCAAAAUCUCUCUGG-3'), SEQ ID NO. 1378 (5'-UAGUGACUGGAUCCAAGG-3'), SEQ ID NO. 1379 (5'-UUAGUGACUGGAUCCAAG-3'), SEQ ID NO. 1380 (5'-GCCAAGAAAGACCACCUU-3'), SEQ ID NO. 1381 (5'-CAAGCCUUUACUCCCAUA-3'), SEQ ID NO. 1382 (5'-CCAAAGCAAGCCUUUACU-3'), SEQ ID NO. 1383 (5'-AGCCUUUACUCCCAUAGA-3'), SEQ ID NO. 1384 (5'-GCAGAUGGAGUUCUUGAA-3'), SEQ ID NO. 1385 (5'-AUGGACUACAAUGAGACU-3'), SEQ ID NO. 1386 (5'-AAUUAGUGACUGGAUCCA-3'), SEQ ID NO. 1387 (5'-AUUAGUGACUGGAUCCAA-3'), SEQ ID NO. 1388 (5'-AGUGACUGGAUCCAAGGA-3'), SEQ ID NO. 1389 (5'-GUGACUGGAUCCAAGGAC-3'), SEQ ID NO. 1390 (5'-GACUGGAUCCAAGGACCA-3'), SEQ ID NO. 1391 (5'-CAAUGAUGGAGCAUGUGC-3'), SEQ ID NO. 1392 (5'-AAUGAUGGAGCAUGUGCG-3'), SEQ ID NO. 1393 (5'-AUGAUGGAGCAUGUGCGA-3'), SEQ ID NO. 1394 (5'-UGAUGGAGCAUGUGCGAA-3'), SEQ ID NO. 1395 (5'-GAUGGAGCAUGUGCGAAU-3'), SEQ ID NO. 1396 (5'-UGGAGCAUGUGCGAAUUC-3'), SEQ ID NO. 1397 (5'-AACUGGACCCUCGAAGGU-3'), SEQ ID NO. 1398 (5'-ACUGGACCCUCGAAGGUG-3'), SEQ ID NO. 1399 (5'-CUGGACCCUCGAAGGUGG-3'), SEQ ID NO. 1400 (5'-UGGACCCUCGAAGGUGGA-3'), SEQ ID NO. 1401 (5'-GGACCCUCGAAGGUGGAA-3') and SEQ ID NO. 1402 (5'-GACCCUCGAAGGUGGAAG-3'), respectively.

In embodiments, an antisense strand of the siRNA molecule targeting the sequence SEQ ID NO. 1 of the TSC1 gene or the corresponding mRNA comprises or consists of an oligonucleotide sequence having 19, 20 or 21 nucleotides from one of the nucleotide sequences defined in the group consisting of SEQ ID NO. 65, SEQ ID NO. 641 - SEQ ID NO. 642, SEQ ID NO. 855, SEQ ID NO. 860, SEQ ID NO. 865, SEQ ID NO. 870 - SEQ ID NO. 871. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 129, SEQ ID NO. 748 - SEQ ID NO. 749, SEQ ID NO. 908, SEQ ID NO. 913, SEQ ID NO. 918, SEQ ID NO. 923 - SEQ ID NO. 924, respectively.

In embodiments, an antisense strand of the siRNA molecule targeting the sequence SEQ ID NO. 2 of the TSC1 gene or the corresponding mRNA comprises or consists of an oligonucleotide sequence having 19, 20 or 21 nucleotides from one of the nucleotide sequences defined in the group consisting of SEQ ID NO. 66, SEQ ID NO. 193 - SEQ ID NO. 200, SEQ ID NO. 856, SEQ ID NO. 861, SEQ ID NO. 866, SEQ ID NO. 872 - SEQ ID NO. 873. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 130, SEQ ID NO. 417 - SEQ ID NO. 424, SEQ ID NO. 909, SEQ ID NO. 914, SEQ ID NO. 919, SEQ ID NO. 925 - SEQ ID NO. 926, respectively.

In embodiments, an antisense strand of the siRNA molecule targeting the sequence SEQ ID NO. 3 of the TSC1 gene or the corresponding mRNA comprises or consists of an oligonucleotide sequence having 19, 20 or 21 nucleotides from one of the nucleotide sequences defined in the group consisting of SEQ ID NO. 67, SEQ ID NO. 201 - SEQ ID NO. 208, SEQ ID NO. 857, SEQ ID NO. 862, SEQ ID NO. 867, SEQ ID NO. 874 - SEQ ID NO. 875. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 131, SEQ ID NO. 425 - SEQ ID NO. 432, SEQ ID NO. 910, SEQ ID NO. 915, SEQ ID NO. 920, SEQ ID NO. 927 - SEQ ID NO. 928, respectively.

In embodiments, an antisense strand of the siRNA molecule targeting the sequence SEQ ID NO. 4 of the TSC1 gene or the corresponding mRNA comprises or consists of an oligonucleotide sequence having 19, 20 or 21 nucleotides from one of the nucleotide sequences defined in the group consisting of SEQ ID NO. 68, SEQ ID NO. 643 - SEQ ID NO. 644. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 132, SEQ ID NO. 750 - SEQ ID NO. 751, respectively.

In embodiments, an antisense strand of the siRNA molecule targeting the sequence SEQ ID NO. 5 of the TSC1 gene or the corresponding mRNA comprises or consists of an oligonucleotide sequence having 19, 20 or 21 nucleotides from one of the nucleotide sequences defined in the group consisting of SEQ ID NO. 69. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 133, respectively.

In embodiments, an antisense strand of the siRNA molecule targeting the sequence SEQ ID NO. 6 of the TSC1 gene or the corresponding mRNA comprises or consists of an oligonucleotide sequence having 19, 20 or 21 nucleotides from one of the nucleotide sequences defined in the group consisting of SEQ ID NO. 70, SEQ ID NO. 209 - SEQ ID NO. 216, SEQ ID NO. 645 - SEQ ID NO. 649. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 134, SEQ ID NO. 433 - SEQ ID NO. 440, SEQ ID NO. 752 - SEQ ID NO. 756, respectively.

In embodiments, an antisense strand of the siRNA molecule targeting the sequence SEQ ID NO. 7 of the TSC1 gene or the corresponding mRNA comprises or consists of an oligonucleotide sequence having 19, 20 or 21 nucleotides from one of the nucleotide sequences defined in the group consisting of SEQ ID NO. 71, SEQ ID NO. 217 - SEQ ID NO. 224, SEQ ID NO. 650 - SEQ ID NO. 651, SEQ ID NO. 858, SEQ ID NO. 863, SEQ ID NO. 868, SEQ ID NO. 876 - SEQ ID NO. 877. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 135, SEQ ID NO. 441 - SEQ ID NO. 448, SEQ ID NO. 757 - SEQ ID NO. 758, SEQ ID NO. 911, SEQ ID NO. 916, SEQ ID NO. 921, SEQ ID NO. 929 - SEQ ID NO. 930, respectively.

In embodiments, an antisense strand of the siRNA molecule targeting the sequence SEQ ID NO. 8 of the TSC1 gene or the corresponding mRNA comprises or consists of an oligonucleotide sequence having 19, 20 or 21 nucleotides from one of the nucleotide sequences defined in the group consisting of SEQ ID NO. 72, SEQ ID NO. 225 - SEQ ID NO. 232, SEQ ID NO. 652 - SEQ ID NO. 656, SEQ ID NO. 859, SEQ ID NO. 864, SEQ ID NO. 869, SEQ ID NO. 878 - SEQ ID NO. 879. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 136, SEQ ID NO. 449 - SEQ ID NO. 456, SEQ ID NO. 759 - SEQ ID NO. 763, SEQ ID NO. 912, SEQ ID NO. 917, SEQ ID NO. 922, SEQ ID NO. 931 - SEQ ID NO. 932, respectively.

In embodiments, an antisense strand of the siRNA molecule targeting the sequence SEQ ID NO. 9 of the TSC1 gene or the corresponding mRNA comprises or consists of an oligonucleotide sequence having 19, 20 or 21 nucleotides from one of the nucleotide sequences defined in the group consisting of SEQ ID NO. 73, SEQ ID NO. 233 - SEQ ID NO. 240. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 137, SEQ ID NO. 457 - SEQ ID NO. 464, respectively.

In embodiments, an antisense strand of the siRNA molecule targeting the sequence SEQ ID NO. 10 of the TSC1 gene or the corresponding mRNA comprises or consists of an oligonucleotide sequence having 19, 20 or 21 nucleotides from one of the nucleotide sequence SEQ ID NO. 74. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequence defined in SEQ ID NO. 138, respectively.

In embodiments, an antisense strand of the siRNA molecule targeting the sequence SEQ ID NO. 11 of the TSC1 gene or the corresponding mRNA comprises or consists of an oligonucleotide sequence having 19, 20 or 21 nucleotides from one of the nucleotide sequence SEQ ID NO. 75. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequence defined in SEQ ID NO. 139, respectively.

In embodiments, an antisense strand of the siRNA molecule targeting the sequence SEQ ID NO. 12 of the TSC1 gene or the corresponding mRNA comprises or consists of an oligonucleotide sequence having 19, 20 or 21 nucleotides from one of the nucleotide sequence SEQ ID NO. 76. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequence defined in SEQ ID NO. 140, respectively.

In embodiments, an antisense strand of the siRNA molecule targeting the sequence SEQ ID NO. 13 of the TSC1 gene or the corresponding mRNA comprises or consists of an oligonucleotide sequence having 19, 20 or 21 nucleotides from one of the nucleotide sequences defined in the group consisting of SEQ ID NO. 77, SEQ ID NO. 241 - SEQ ID NO. 248 and SEQ ID NO. 657 - SEQ ID NO. 661. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 141, SEQ ID NO. 465 - SEQ ID NO. 472 and SEQ ID NO. 764 - SEQ ID NO. 768, respectively.

In embodiments, an antisense strand of the siRNA molecule targeting the sequence SEQ ID NO. 14 of the TSC1 gene or the corresponding mRNA comprises or consists of an oligonucleotide sequence having 19, 20 or 21 nucleotides from one of the nucleotide sequence SEQ ID NO. 78. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequence defined in SEQ ID NO. 142, respectively.

In embodiments, an antisense strand of the siRNA molecule targeting the sequence SEQ ID NO. 15 of the TSC1 gene or the corresponding mRNA comprises or consists of an oligonucleotide sequence having 19, 20 or 21 nucleotides from one of the nucleotide sequences defined in the group consisting of SEQ ID NO. 79, SEQ ID NO. 249 - SEQ ID NO. 256 and SEQ ID NO. 662 - SEQ ID NO. 663. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 143, SEQ ID NO. 473 - SEQ ID NO. 480 and SEQ ID NO. 769 - SEQ ID NO. 770, respectively.

In embodiments, an antisense strand of the siRNA molecule targeting the sequence SEQ ID NO. 16 of the TSC1 gene or the corresponding mRNA comprises or consists of an oligonucleotide sequence having 19, 20 or 21 nucleotides from one of the nucleotide sequences defined in the group consisting of SEQ ID NO. 80, SEQ ID NO. 257 - SEQ ID NO. 264 and SEQ ID NO. 664 - SEQ ID NO. 668. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 144, SEQ ID NO. 481 - SEQ ID NO. 488 and SEQ ID NO. 771 - SEQ ID NO. 775, respectively.

In embodiments, an antisense strand of the siRNA molecule targeting the sequence SEQ ID NO. 17 of the TSC1 gene or the corresponding mRNA comprises or consists of an oligonucleotide sequence having 19, 20 or 21 nucleotides from one of the nucleotide sequences defined in the group consisting of SEQ ID NO. 81, SEQ ID NO. 265 - SEQ ID NO. 272 and SEQ ID NO. 669 - SEQ ID NO. 673. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 145, SEQ ID NO. 489 - SEQ ID NO. 496 and SEQ ID NO. 776 - SEQ ID NO. 780, respectively.

In embodiments, an antisense strand of the siRNA molecule targeting the sequence SEQ ID NO. 18 of the TSC1 gene or the corresponding mRNA comprises or consists of an oligonucleotide sequence having 19, 20 or 21 nucleotides from one of the nucleotide sequence SEQ ID NO. 82. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequence defined in SEQ ID NO. 146, respectively.

In embodiments, an antisense strand of the siRNA molecule targeting the sequence SEQ ID NO. 19 of the TSC1 gene or the corresponding mRNA comprises or consists of an oligonucleotide sequence having 19, 20 or 21 nucleotides from one of the nucleotide sequence SEQ ID NO. 83. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequence defined in SEQ ID NO. 147, respectively.

In embodiments, an antisense strand of the siRNA molecule targeting the sequence SEQ ID NO. 20 of the TSC1 gene or the corresponding mRNA comprises or consists of an oligonucleotide sequence having 19, 20 or 21 nucleotides from one of the nucleotide sequence SEQ ID NO. 84. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequence defined in SEQ ID NO. 148, respectively.

In embodiments, an antisense strand of the siRNA molecule targeting the sequence SEQ ID NO. 21 of the TSC1 gene or the corresponding mRNA comprises or consists of an oligonucleotide sequence having 19, 20 or 21 nucleotides from one of the nucleotide sequence SEQ ID NO. 85. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequence defined in SEQ ID NO. 149, respectively.

In embodiments, an antisense strand of the siRNA molecule targeting the sequence SEQ ID NO. 22 of the TSC1 gene or the corresponding mRNA comprises or consists of an oligonucleotide sequence having 19, 20 or 21 nucleotides from one of the nucleotide sequence SEQ ID NO. 86. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequence defined in SEQ ID NO. 150, respectively.

In embodiments, an antisense strand of the siRNA molecule targeting the sequence SEQ ID NO. 23 of the TSC1 gene or the corresponding mRNA comprises or consists of an oligonucleotide sequence having 19, 20 or 21 nucleotides from one of the nucleotide sequence SEQ ID NO. 87. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequence defined in SEQ ID NO. 151, respectively.

In embodiments, an antisense strand of the siRNA molecule targeting the sequence SEQ ID NO. 24 of the TSC1 gene or the corresponding mRNA comprises or consists of an oligonucleotide sequence having 19, 20 or 21 nucleotides from one of the nucleotide sequences defined in the group consisting of SEQ ID NO. 88, SEQ ID NO. 273 - SEQ ID NO. 280 and SEQ ID NO. 674 - SEQ ID NO. 678. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 152, SEQ ID NO. 497 - SEQ ID NO. 504 and SEQ ID NO. 781 - SEQ ID NO. 785, respectively.

In embodiments, an antisense strand of the siRNA molecule targeting the sequence SEQ ID NO. 25 of the TSC1 gene or the corresponding mRNA comprises or consists of an oligonucleotide sequence having 19, 20 or 21 nucleotides from one of the nucleotide sequences defined in the group consisting of SEQ ID NO. 89, SEQ ID NO. 281 - SEQ ID NO. 288 and SEQ ID NO. 679 - SEQ ID NO. 683. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 153, SEQ ID NO. 505 - SEQ ID NO. 512 and SEQ ID NO. 786 - SEQ ID NO. 790, respectively.

In embodiments, an antisense strand of the siRNA molecule targeting the sequence SEQ ID NO. 26 of the TSC1 gene or the corresponding mRNA comprises or consists of an oligonucleotide sequence having 19, 20 or 21 nucleotides from one of the nucleotide sequences defined in the group consisting of SEQ ID NO. 90, SEQ ID NO. 289 - SEQ ID NO. 296 and SEQ ID NO. 684 - SEQ ID NO. 688. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 154, SEQ ID NO. 513 - SEQ ID NO. 520 and SEQ ID NO. 791 - SEQ ID NO. 795, respectively.

In embodiments, an antisense strand of the siRNA molecule targeting the sequence SEQ ID NO. 27 of the TSC1 gene or the corresponding mRNA comprises or consists of an oligonucleotide sequence having 19, 20 or 21 nucleotides from one of the nucleotide sequences defined in the group consisting of SEQ ID NO. 91, SEQ ID NO. 297 - SEQ ID NO. 304 and SEQ ID NO. 689 - SEQ ID NO. 693. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 155, SEQ ID NO. 521 - SEQ ID NO. 528 and SEQ ID NO. 796 - SEQ ID NO. 800, respectively.

In embodiments, an antisense strand of the siRNA molecule targeting the sequence SEQ ID NO. 28 of the TSC1 gene or the corresponding mRNA comprises or consists of an oligonucleotide sequence having 19, 20 or 21 nucleotides from one of the nucleotide sequences defined in the group consisting of SEQ ID NO. 92 and SEQ ID NO. 305 - SEQ ID NO. 312. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 156 and SEQ ID NO. 529 - SEQ ID NO. 536, respectively.

In embodiments, an antisense strand of the siRNA molecule targeting the sequence SEQ ID NO. 29 of the TSC1 gene or the corresponding mRNA comprises or consists of an oligonucleotide sequence having 19, 20 or 21 nucleotides from one of the nucleotide sequences defined in the group consisting of SEQ ID NO. 93 and SEQ ID NO. 694 - SEQ ID NO. 695. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 157 and SEQ ID NO. 801 - SEQ ID NO. 802, respectively.

In embodiments, an antisense strand of the siRNA molecule targeting the sequence SEQ ID NO. 30 of the TSC1 gene or the corresponding mRNA comprises or consists of an oligonucleotide sequence having 19, 20 or 21 nucleotides from one of the nucleotide sequences defined in the group consisting of SEQ ID NO. 94, SEQ ID NO. 313 - SEQ ID NO. 320 and SEQ ID NO. 696 - SEQ ID NO. 700. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 158, SEQ ID NO. 537 - SEQ ID NO. 544 and SEQ ID NO. 803 - SEQ ID NO. 807, respectively.

In embodiments, an antisense strand of the siRNA molecule targeting the sequence SEQ ID NO. 31 of the TSC1 gene or the corresponding mRNA comprises or consists of an oligonucleotide sequence having 19, 20 or 21 nucleotides from one of the nucleotide sequences defined in the group consisting of SEQ ID NO. 95, SEQ ID NO. 321 - SEQ ID NO. 328 and SEQ ID NO. 701 - SEQ ID NO. 705. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 159, SEQ ID NO. 545 - SEQ ID NO. 552 and SEQ ID NO. 808 - SEQ ID NO. 812, respectively.

In embodiments, an antisense strand of the siRNA molecule targeting the sequence SEQ ID NO. 32 of the TSC1 gene or the corresponding mRNA comprises or consists of an oligonucleotide sequence having 19, 20 or 21 nucleotides from one of the nucleotide sequences defined in the group consisting of SEQ ID NO. 96, SEQ ID NO. 329 - SEQ ID NO. 336 and SEQ ID NO. 706 - SEQ ID NO. 710. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 160, SEQ ID NO. 553 - SEQ ID NO. 560 and SEQ ID NO. 813 - SEQ ID NO. 817, respectively.

In embodiments, an antisense strand of the siRNA molecule targeting the sequence SEQ ID NO. 33 of the TSC1 gene or the corresponding mRNA comprises or consists of an oligonucleotide sequence having 19, 20 or 21 nucleotides from one of the nucleotide sequences defined in the group consisting of SEQ ID NO. 97, SEQ ID NO. 337 - SEQ ID NO. 344 and SEQ ID NO. 711 - SEQ ID NO. 715. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 161, SEQ ID NO. 561 - SEQ ID NO. 568 and SEQ ID NO. 818 - SEQ ID NO. 822, respectively.

In embodiments, an antisense strand of the siRNA molecule targeting the sequence SEQ ID NO. 34 of the TSC1 gene or the corresponding mRNA comprises or consists of an oligonucleotide sequence having 19, 20 or 21 nucleotides from one of the nucleotide sequences defined in the group consisting of SEQ ID NO. 98, SEQ ID NO. 345 - SEQ ID NO. 352 and SEQ ID NO. 716 - SEQ ID NO. 717. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 162, SEQ ID NO. 569 - SEQ ID NO. 576 and SEQ ID NO. 823 - SEQ ID NO. 824, respectively.

In embodiments, an antisense strand of the siRNA molecule targeting the sequence SEQ ID NO. 35 of the TSC1 gene or the corresponding mRNA comprises or consists of an oligonucleotide sequence having 19, 20 or 21 nucleotides from one of the nucleotide sequence SEQ ID NO. 99. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequence defined in SEQ ID NO. 163, respectively.

In embodiments, an antisense strand of the siRNA molecule targeting the sequence SEQ ID NO. 36 of the TSC1 gene or the corresponding mRNA comprises or consists of an oligonucleotide sequence having 19, 20 or 21 nucleotides from one of the nucleotide sequences defined in the group consisting of SEQ ID NO. 100, SEQ ID NO. 353 - SEQ ID NO. 360 and SEQ ID NO. 718 - SEQ ID NO. 719. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 164, SEQ ID NO. 577 - SEQ ID NO. 584 and SEQ ID NO. 825 - SEQ ID NO. 826, respectively.

In embodiments, an antisense strand of the siRNA molecule targeting the sequence SEQ ID NO. 37 of the TSC1 gene or the corresponding mRNA comprises or consists of an oligonucleotide sequence having 19, 20 or 21 nucleotides from one of the nucleotide sequences defined in the group consisting of SEQ ID NO. 101 and SEQ ID NO. 720 - SEQ ID NO. 724. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 165 and SEQ ID NO. 827 - SEQ ID NO. 831, respectively.

In embodiments, an antisense strand of the siRNA molecule targeting the sequence SEQ ID NO. 38 of the TSC1 gene or the corresponding mRNA comprises or consists of an oligonucleotide sequence having 19, 20 or 21 nucleotides from one of the nucleotide sequences defined in the group consisting of SEQ ID NO. 102, SEQ ID NO. 361 - SEQ ID NO. 368 and SEQ ID NO. 725 - SEQ ID NO. 729. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 166, SEQ ID NO. 585 - SEQ ID NO. 592 and SEQ ID NO. 832 - SEQ ID NO. 836, respectively.

In embodiments, an antisense strand of the siRNA molecule targeting the sequence SEQ ID NO. 39 of the TSC1 gene or the corresponding mRNA comprises or consists of an oligonucleotide sequence having 19, 20 or 21 nucleotides from one of the nucleotide sequences defined in the group consisting of SEQ ID NO. 103, SEQ ID NO. 369 - SEQ ID NO. 376 and SEQ ID NO. 730 - SEQ ID NO. 734. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 167, SEQ ID NO. 593 - SEQ ID NO. 600 and SEQ ID NO. 837 - SEQ ID NO. 841, respectively.

In embodiments, an antisense strand of the siRNA molecule targeting the sequence SEQ ID NO. 40 of the TSC1 gene or the corresponding mRNA comprises or consists of an oligonucleotide sequence having 19, 20 or 21 nucleotides from one of the nucleotide sequences defined in the group consisting of SEQ ID NO. 104, SEQ ID NO. 377 - SEQ ID NO. 384 and SEQ ID NO. 735 - SEQ ID NO. 736. In embodiments, a sense strand of the siRNA molecule which is complementary to this TSC1 antisense strand comprises or consists of an oligonucleotide sequence having 14, 15, 16, 17, 18, 19, 20 or 21 nucleotides from the nucleotide sequences defined in the group consisting of SEQ ID NO. 168, SEQ ID NO. 601 - SEQ ID NO. 608 and SEQ ID NO. 842 - SEQ ID NO. 843, respectively.

The RNAi oligonucleotides or siRNA molecules of the present disclosure are useful for the treatment of a retinal dystrophy and can be used in a method of treatment of a patient with a retinal dystrophy, for example by administering the siRNA or oligonucleotide to the patient in need, preferably locally to the eye or the retina, and more preferably by topical or intravitreal administration in the eye.

Within the meaning of the present invention the retinal dystrophy can refer to a retinal dystrophy characterized by dysfunction or progressive loss of photoreceptor cells (i.e. cones and/or rods), including for example cone dominated dystrophies, such as achromatopsia, incomplete achromatopsia, blue cone monochromatism, cone-rod dystrophy (CRD) and X-linked progressive cone dystrophy; rod dominated dystrophies, where either rod photoreceptors are predominantly affected, or rod photoreceptors are the first affected, and including rod dystrophies and rod-cone dystrophies, such as, retinitis pigmentosa or congenital stationary night blindness (CSNB); generalized retinal dystrophies, which involves both rods and cones, such as Leber Congenital Amaurosis (LCA); but also retinal dystrophies associated with a syndrome, such as, Usher syndrome, Bardet-Biedl syndrome, Joubert syndrome, Senior-Loken syndrome, Cohen syndrome and Alström syndrome. Likewise, the retinal dystrophy can also referto an inherited retinal dystrophy. In embodiments, said retinal dystrophy is selected from the group consisting of retinitis pigmentosa, Leber Congenital Amaurosis (LCA) and retinal dystrophy associated to Alstrom syndrome.

As it is known from the state of the art, many different structures have been proposed to achieve RNA interference. Generally double stranded siRNA molecules are from about 19 to about 25 nucleotides in length and include blunt-ended structures as well as those with overhangs. Overhangs have been described to be advantageous and may be present on the 5' ends or on the 3' ends of either strand as they reduce recognition by RNAses and imitate Dicer's natural substrate. Some authors recommend including overhangs on both 3 ' ends of the molecules, whereas others consider one overhang to be sufficient. Others have described the use of blunt-ended structures with specific modification patterns (EP1527176, WO2005062937, WO2008104978, EP2322617, EP2348133, US20130130377, and many others) .

Overhangs may be comprised of between 1 and 5 nucleotides; typically overhangs in siRNA molecules are made up of dinucleotides. Classical molecules used in the field comprise a 19 nucleotide double stranded molecule which further comprises 3' dinucleotide overhangs preferably comprising deoxynucleotides as taught in initial studies by Tuschl (WO0244321). These overhangs are said to further enhance resistance to nuclease (RNase) degradation. Later, Kim et al 2005 describe that 21-mer products (containing dinucleotide overhangs) are necessary for loading onto RISC. Further, Bramsen et al. 2009 describe the introduction of possible destabilizing modifications to the overhangs to further increase silencing efficiency.

As such, a preferred embodiment of the various aspects of the present invention refers to RNAi oligonucleotides that comprise siRNA molecules targeting any sequence selected from the group consisting of SEQ ID NO. 1 to SEQ ID NO. 64 which comprise at least one overhang, preferably a 3' overhang in the sense and/or the antisense strand. Where the invention relates to RNAi oligonucleotides comprising or consisting of a siRNA molecule targeting at least one sequence selected from any of SEQ ID NO. 1 to SEQ ID NO. 64, the siRNA molecule can include an antisense strand of equivalent length and complementary to the target, and a sense strand of equivalent length and complementary to the antisense strand. The antisense and sense strands may further include additional bases or abasic residues (e.g. inverted abasic deoxyribose) which are not complementary to the other strand or the target, and/or which are not paired in the double stranded portion of the siRNA. For example, SEQ ID NO 41 is a 19 nucleotide sequence; the siRNA may include a 19 bp double stranded region over this portion of sequence identity, and additional dinucleotide overhangs, such as can be exemplified by the siRNA ID 01040 which has unpaired additional UU dinucleotide overhangs in both 3'-ends of the 19 bp double stranded region formed by base-pairing of its sense (SEQ ID NO. 847) and antisense (SEQ ID NO. 740) strands.

A preferred embodiment of the various aspects of the present invention refers to RNAi oligonucleotides comprising siRNA molecules targeting at least one sequence selected from the group consisting of SEQ ID NO. 41 to SEQ ID NO. 64 (preferably of SEQ ID NO .41 to SEQ ID NO. 49, and more preferably of SEQ ID NO. 41, SEQ ID NO. 42 and SEQ ID NO. 49), and optionally comprising also siRNA molecules targeting SEQ ID NO. 1 to SEQ ID NO. 40 (preferably of SEQ ID NO. 1 to SEQ ID NO. 17, and more preferably of SEQ ID NO. 1 to SEQ ID NO. 8, including SEQ ID NO. 1, SEQ ID NO. 2, SEQ ID NO. 7, SEQ ID NO. 3 and/or SEQ ID NO. 8), wherein each strand of the double-stranded siRNA molecules is about 18 to about 28 or more (e.g. , about 18, 19, 20, 21, 22, 23, 24, 25, 26, 27 or 28 or more) nucleotides long .

In preferred embodiments of the various aspects of the present invention, the RNAi oligonucleotide comprises or consists of a siRNA molecule of 19-28 nucleotides long or more and comprising a nucleotide sequence selected from the group consisting of SEQ ID NO. 105 to SEQ ID NO. 128, which in embodiments may optionally comprise also a siRNA molecule of 19-28 nucleotides long or more and comprising a nucleotide sequence selected from the group consisting of SEQ ID NO. 65 to SEQ ID NO. 104. More preferably, the double-stranded siRNA molecule or molecules are at least 19 nucleotides long and selected from the group consisting of SEQ ID NO. 105 to SEQ ID NO. 128, and optionally also from any one of the group consisting of SEQ ID NO. 65 to SEQ ID NO. 104.

In other preferred embodiments of the various aspects of the present invention, the RNAi oligonucleotides comprise or consists of blunt-ended siRNA molecules.

In other preferred embodiments of the various aspects of the present invention, the RNAi oligonucleotide comprises or consists of a siRNA comprising or consisting of a 19-nucleotide double-stranded structure targeting at least one sequence selected from the group consisting of SEQ ID NO. 41 to SEQ ID NO. 64. More preferably, the siRNA comprising or consisting of a 19-nucleotide double-stranded structure targets at least one sequence selected from the group consisting of SEQ ID NO. 41 to SEQ ID NO. 49, and even more preferably targets SEQ ID NO. 41 or SEQ ID NO. 42. In further preferred embodiments, the RNAi oligonucleotide or siRNA optionally further comprises another siRNA comprising or consisting of a 19-nucleotide double-stranded structure targeting at least one sequence selected from the group consisting of SEQ ID NO. 1 to SEQ ID NO. 40. Preferably, the another siRNA comprising or consisting of a 19-nucleotide double-stranded structure targets at least one sequence selected from the group consisting of SEQ ID NO. 1 to SEQ ID NO. 17, more preferably targets at least one sequence selected from the group consisting of SEQ ID NO. 1 to SEQ ID NO. 8, and even more preferably targets any of the sequences from the group consisting of SEQ ID NO. 2, SEQ ID NO. 1 and SEQ ID NO. 7, and/or from the group consisting of SEQ ID NO. 3 and SEQ ID NO. 8.

In other preferred embodiments of the various aspects of the present invention, the RNAi oligonucleotide comprises or consists of a siRNA comprising or consisting of a 19 nucleotide, blunt-ended, double-stranded structure targeting at least one sequence selected from the group consisting of SEQ ID NO. 41 to SEQ ID NO. 64. More preferably, said blunt-ended double-stranded structure targets at least one sequence selected from the group consisting of SEQ ID NO. 41 to SEQ ID NO. 49, and even more preferably targets SEQ ID NO. 41 or SEQ ID NO. 42. In embodiments, the antisense strand of this siRNA is at least 80%, preferably at least 90%, complementary to at least one sequence selected from the group consisting of SEQ ID NO. 41 to SEQ ID NO. 49. In preferred embodiments, the RNAi oligonucleotide comprises or consists of a siRNA comprising or consisting of at least one antisense sequence selected from the group consisting of: SEQ ID NO. 105 to SEQ ID NO. 113. In a preferred embodiment, this compound comprises or consists of at least one antisense sequence selected from the group consisting of SEQ ID NO. 105 or SEQ ID NO. 106.

In other preferred embodiments of the various aspects of the present invention, the RNAi oligonucleotide comprises or consists of a siRNA comprising or consisting of an antisense strand with sequence selected from the group consisting of SEQ ID NO. 105 to SEQ ID NO. 113, and a sense strand which is complementary to the antisense strand. Preferably, the sense strand has between 14 and 21 nucleotides in length having at least 14, 15, 16, 17, 18 or 19 nucleotides complementary to the antisense strand.

In more preferred embodiments, the RNAi oligonucleotide comprises or consists of a siRNA as defined by siRNA ID 01009 (antisense strand SEQ ID NO. 105: 5'-UGUCACAGUGACAAGCUUG-3' and sense strand SEQ ID NO. 169: 5'-CAAGCUUGUCACUGUGACA-3') or siRNA ID 1019 (antisense strand SEQ ID NO. 106: 5'-CAACCAGCCAGGUUUUGGU-3' and sense strand SEQ ID NO. 170: 5'-ACCAAAACCUGGCUGGUUG-3') in Figure 2.

Furthermore, as described in the section termed background of the invention, an important issue with RNAi oligonucleotides and siRNA molecules is their instability in biological fluids due to the ubiquitous nature of RNases. Consequently, the use of many different chemical modifications to nucleotides has been described with the purpose of enhancing compound stability.

Another inherent problem of RNAi oligonucleotides and siRNA molecules is their immunogenicity, whereby siRNAs have been found to induce unspecific activation of the innate immune system, including upregulation of certain cytokines, e.g. type I and/or type II interferon as well as IL-12 , IL-6 and/or TNF-alpha production . The origin of these effects is thought to be activation of Toll-like receptors such as TLR7 , TLR8 and/or TLR3 by siRNA.

Both of these effects, recognition by RNases and immunogenicity, have also been described to be sequence dependent.

Some of the chemical modifications which enhance compound stability by decreasing susceptibility to RNases are also able to reduce induction of immune recognition and consequently reduce the subsequent immune response. However, insertion of chemically modified nucleotides in a siRNA or RNAi oligonucleotide may also result in decreased silencing efficacy as described in the previous section, and hence must be approached with caution. Consequently, in a preferred embodiment of the various aspects of the present invention, the RNAi oligonucleotide or the siRNA molecule comprised in it further comprises at least one nucleotide with a chemical modification. In embodiments, the at least one chemically modified nucleotide is on the sense strand, on the antisense strand or on both strands of the siRNA. In other embodiments, all of the nucleotides of the sense strand and all of the nucleotides of the antisense strand of the siRNA molecule comprise a modification.

Preferred chemical modifications which enhance stability and reduce immunogenic effects include 2 ' -O-methyl nucleotides, 2'-fluoro nucleotides, 2'-amino nucleotides, 2'-deoxy nucleotides, or nucleotides containing 2'-O or 4'-C methylene bridges. Other preferred chemical modifications for exonuclease protection include the ExoEndoLight pattern of modification (EEL): modification of all pyrimidines in the sense strand to 2 ' -O-methyl residues, and modification of all pyrimidines in a 5' -DAS' or 5' -CA-3' motif in the antisense strand to 2'-O-methyl residues. In addition, position 1 of the sense strand can also be changed to 2'-O-methyl to prevent 5'-phosphorylation of the sense strand and thus increasing strand-specificity of the siRNA. In addition, the sense strand can also include a 2'-O-methyl modification in position 14, because 2'-O-Me residues at this position inactivate the sense strand and therefore increase strand-specificity of the siRNAs. In addition, other preferred chemical modifications for nuclease protection include Methyl-Fluoro modification pattern (MEF): alternating 2'-fluoro and 2'-O-methyl modifications starting (5'-end) with a 2'-F on the sense strand and starting with 2'-O-Me on the antisense strand. In addition, position 1 of the sense strand can also be changed to 2'-O-Me and position 1 of the antisense strand to 2'-F (as 2' F residues are compatible with 5' -phosphorylation whereas 2'-O-Me residues are bulky and generally impair phosphorylation). This modification pattern not only stabilizes the molecule but also disables the ability of the RISC to use the sense strand thus promoting strand-specificity. Also, modification of the ribonucleotide backbone can be performed by binding the nucleotides by using phosphorothioate bonds instead of phosphodiester links. A further preferred chemical modification within the meaning of the present invention relates to: 4'-Thioribose, 5-Propynyluracile 3', 5 '-methyluridine or the substitution of uracyl ribonucleotides with deoxythymidine (deoxyribonucleotides). In another preferred embodiment of the present invention, the at least one chemically modified nucleotide and/or the at least one chemical modification in the ribonucleotide backbone is on the sense strand, on the antisense strand or on both strands of the siRNA.

In some embodiments, at least one of the modified nucleotides is selected from the group consisting of a deoxy-nucleotide, a 3'-terminal deoxythimidine (dT) nucleotide, a 2'-O-methyl modified nucleotide, a 2'-fluoro modified nucleotide, a 2'-deoxy-modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, 2'-O-alkyl-modified nucleotide, a 2'-methoxyethyl modified nucleotide, a 2'-O-alkyl-modified nucleotide, a morpholino nucleotide, a phosphoramidate, a non-natural base comprising nucleotide, a tetrahydropyran modified nucleotide, a 1 ,5-anhydrohexitol modified nucleotide, a cyclohexenyl modified nucleotide, a nucleotide comprising a phosphorothioate group, a nucleotide comprising a methylphosphonate group, a nucleotide comprising a 5'-phosphate, a nucleotide comprising a 5 '-phosphate mimic, a glycol modified nucleotide, and a 2-O-(N-methylacetamide) modified nucleotide; and combinations thereof.

In some embodiments, at least one of the modified nucleotides is selected from the group consisting of a 2'-fluoro modified nucleotide, a 2'-O-methyl modified nucleotide, a 5-methyl-uridine, a 5-methyl-citidine, a deoxy-nucleotide (or deoxiribonucleotide), a 2'-deoxyinosine, a 3'-terminal or internal deoxythimidine (dT) nucleotide, a 2'-O-methyl nucleotide with a 5'-vivylphosphonate group (5'-phosphate mimic), a 5'-O-methyl-nucleobase, a nucleotide comprising a phosphorothioate group, an UNA monomer (unlocked nucleotide), a GNA monomer (glycol modified nucleotide), an inverted abasic deoxyribose; and combinations thereof.

In embodiments, at least one of the modified nucleotides is selected from the group consisting of: 2'-O-methyl modification, 2'-fluoro modification, 2'-O-methyl-5'-vinylphosphonate modification, 5'-O-methyl modification, substitution of uracil with 5'-methyluridine, substitution of cytosine with 5-methylcytosine, substitution of uridine or cytosine with 2'-deoxyinosine, substitution of uracyl ribose nucleotide with deoxythymidine nucleotide, substitution of ribose nucleotide with deoxyribose nucleotide, substitution of ribose nucleotide with unlocked nucleic acid (UNA) monomer, substitution of ribose nucleotide with glycol nucleic acid (GNA) monomer, substitution of ribose nucleotide with inverted abasic deoxyribose, introduction of phosphorothioate modified nucleotides, and combinations thereof.

siRNA antisense strands having chemical modifications or modified nucleotides targeting the TSC2 gene according to the present invention include for example the nucleotide sequences defined by any of the group consisting of from SEQ ID NO. 1022 to SEQ ID NO. 1097, SEQ ID NO. 1235, SEQ ID NO. 1236, SEQ ID NO. 1239, SEQ ID NO. 1240, from SEQ ID NO. 1245 to SEQ ID NO. 1265 and from SEQ ID NO. 1427 to SEQ ID NO. 1431. siRNA sense strands having chemical modifications or modified nucleotides which are complementary to these chemically modified antisense strands (at least in 14 or more consecutive nucleotides) include amongst others the nucleotide sequences defined by any of the group consisting of: from SEQ ID NO. 1159 to SEQ ID NO. 1234, SEQ ID NO. 1237, SEQ ID NO. 1238, SEQ ID NO. 1241, SEQ ID NO. 1242, from SEQ ID NO. 1266 to SEQ ID NO. 1286 and from SEQ ID NO. 1432 to SEQ ID NO. 1436.

siRNA antisense strands having chemical modifications or modified nucleotides targeting the TSC1 gene according to the present invention include for example the nucleotide sequences defined by any of the group consisting of from SEQ ID NO. 961 to SEQ ID NO. 1021, and SEQ ID NO. 1243. siRNA sense strands having chemical modifications or modified nucleotides which are complementary to these chemically modified antisense strands (at least in 14 or more consecutive nucleotides) include amongst others the nucleotide sequences defined by any of the group consisting of: from SEQ ID NO. 1 098 to SEQ ID NO. 1158, and SEQ ID NO. 1244.

In some embodiments, at least one of the modified nucleotides is selected from the group consisting of: 2'-O-methyl modification, 2'-deoxy-2'-fluoro modification (also referred as 2'-fluoro modification), 2'-O-methyl-5'-vinylphosphonate modification, substitution of cytosine with 5-methylcytosine, substitution of ribose nucleotide with unlocked nucleic acid (UNA) monomer, substitution of ribose nucleotide with inverted abasic deoxyribose, introduction of phosphorothioate modified nucleotides; and combinations thereof.

Accordingly, preferred modified antisense strands of the siRNA molecule targeting the sequence SEQ ID NO. 41 of the TSC2 gene or its corresponding mRNA can comprise or consist of an oligonucleotide sequence selected from the group consisting of SEQ ID NO. 1022 - SEQ ID NO. 1044, SEQ ID NO. 1239 and SEQ ID NO. 1240. Preferred modified sense strands of the siRNA molecule which are complementary to such antisense strands can comprise or consist of an oligonucleotide sequence selected from the group consisting of SEQ ID NO. 1159 - SEQ ID NO. 1181, SEQ ID NO. 1241 and SEQ ID NO. 1242, respectively. Alternatively, preferred modified antisense strands of the siRNA molecule targeting the sequence SEQ ID NO. 41 of the TSC2 gene or its corresponding mRNA can comprise or consist of an oligonucleotide sequence selected from the group consisting of SEQ ID NO. 1427, SEQ ID NO. 1428, SEQ ID NO. 1429, SEQ ID NO. 1430 and SEQ ID NO. 1431. Preferred modified sense strands complementary to such antisense strands can comprise or consist of an oligonucleotide sequence selected from the group consisting of SEQ ID NO. 1432, SEQ ID NO. 1433, SEQ ID NO. 1434, SEQ ID NO. 1435 and SEQ ID NO. 1436, respectively.

Preferred modified antisense strands of the siRNA molecule targeting the sequence SEQ ID NO. 42 of the TSC2 gene or its corresponding mRNA can comprise or consist of an oligonucleotide sequence selected from the group consisting of SEQ ID NO. 1045 - SEQ ID NO. 1069, SEQ ID NO. 1074 - SEQ ID NO. 1094, SEQ ID NO. 1239, SEQ ID NO. 1240 and SEQ ID NO. 1245 - SEQ ID NO. 1265. Preferred modified sense strands of the siRNA molecule which are complementary to such antisense strands can comprise or consist of an oligonucleotide sequence selected from the group consisting of SEQ ID NO. 1182 - SEQ ID NO. 1206, SEQ ID NO. 1211 - SEQ ID NO. 1231, SEQ ID NO. 1241, SEQ ID NO. 1242 and SEQ ID NO. 1266 - SEQ ID NO. 1286, respectively.

Preferred modified antisense strands of the siRNA molecule targeting the sequence SEQ ID NO. 44 of the TSC2 gene or its corresponding mRNA can comprise or consist of an oligonucleotide sequence selected from the group consisting of SEQ ID NO. 1070, SEQ ID NO. 1071 and SEQ ID NO. 1096. Preferred modified sense strands of the siRNA molecule which are complementary to such antisense strands can comprise or consist of an oligonucleotide sequence selected from the group consisting of SEQ ID NO. 1207, SEQ ID NO. 1208 and SEQ ID NO. 1233, respectively.

Preferred modified antisense strands of the siRNA molecule targeting the sequence SEQ ID NO. 45 of the TSC2 gene or its corresponding mRNA can comprise or consist of an oligonucleotide sequence selected from the group consisting of SEQ ID NO. 1072, SEQ ID NO. 1073 and SEQ ID NO. 1095. Preferred modified sense strands of the siRNA molecule which are complementary to such antisense strands can comprise or consist of an oligonucleotide sequence selected from the group consisting of SEQ ID NO. 1209, SEQ ID NO. 1210 and SEQ ID NO. 1232, respectively.

Preferred modified antisense strands of the siRNA molecule targeting the sequence SEQ ID NO. 49 of the TSC2 gene or its corresponding mRNA can comprise or consist of an oligonucleotide sequence selected from the group consisting of SEQ ID NO. 1235 and SEQ ID NO. 1236. Preferred modified sense strands of the siRNA molecule which are complementary to such antisense strands can comprise or consist of an oligonucleotide sequence selected from the group consisting of SEQ ID NO. 1237* and SEQ ID NO. 1238*, respectively.

Preferred modified antisense strands of the siRNA molecule targeting the sequence SEQ ID NO. 2 of the TSC1 gene or its corresponding mRNA can comprise or consist of an oligonucleotide sequence selected from the group consisting of SEQ ID NO. 980 - SEQ ID NO. 999 and SEQ ID NO. 1243. Preferred modified sense strands of the siRNA molecule which are complementary to such antisense strands can comprise or consist of an oligonucleotide sequence selected from the group consisting of SEQ ID NO. 1117 - SEQ ID NO. 1136 and SEQ ID NO. 1244, respectively.

Preferred modified antisense strands of the siRNA molecule targeting the sequence SEQ ID NO. 1 of the TSC1 gene or its corresponding mRNA can comprise or consist of an oligonucleotide sequence selected from the group consisting of SEQ ID NO. 961 - SEQ ID NO. 979. Preferred modified sense strands of the siRNA molecule which are complementary to such antisense strands can comprise or consist of an oligonucleotide sequence selected from the group consisting of SEQ ID NO. 1098 - SEQ ID NO. 1116, respectively.

Preferred modified antisense strands of the siRNA molecule targeting the sequence SEQ ID NO. 8 of the TSC1 gene or its corresponding mRNA can comprise or consist of an oligonucleotide sequence selected from the group consisting of SEQ ID NO: 1000 - SEQ ID NO: 1021. Preferred modified sense strands of the siRNA molecule which are complementary to such antisense strands can comprise or consist of an oligonucleotide sequence selected from the group consisting of SEQ ID NO: 1137 - SEQ ID NO: 1158, respectively.

In a preferred embodiment, the siRNA targeting SEQ ID NO. 41 of TSC2 gene, or its mRNA, comprises or consists of an antisense strand which comprises or consists of at least one sequence selected from the group consisting of SEQ ID NO. 1022, SEQ ID NO. 1023, SEQ ID NO. 1024, SEQ ID NO. 1025, SEQ ID NO. 1026, SEQ ID NO. 1027, SEQ ID NO. 1028, SEQ ID NO. 1029, SEQ ID NO. 1030, SEQ ID NO. 1031, SEQ ID NO. 1032, SEQ ID NO. 1033, SEQ ID NO. 1034, SEQ ID NO. 1035, SEQ ID NO. 1036, SEQ ID NO. 1037, SEQ ID NO. 1038, SEQ ID NO. 1039, SEQ ID NO. 1040, SEQ ID NO. 1041, SEQ ID NO. 1042, SEQ ID NO. 1043, SEQ ID NO. 1044, SEQ ID NO. 1239 and SEQ ID NO. 1240, and a sense strand which is complementary to the antisense strand which is selected from the group consisting of SEQ ID NO. 1159, SEQ ID NO. 1160, SEQ ID NO. 1161, SEQ ID NO. 1162, SEQ ID NO. 1163, SEQ ID NO. 1164, SEQ ID NO. 1165, SEQ ID NO. 1166, SEQ ID NO. 1167, SEQ ID NO. 1168, SEQ ID NO. 1169, SEQ ID NO. 1170, SEQ ID NO. 1171, SEQ ID NO. 1172, SEQ ID NO. 1173, SEQ ID NO. 1174, SEQ ID NO. 1175, SEQ ID NO. 1176, SEQ ID NO. 1177, SEQ ID NO. 1178, SEQ ID NO. 1179, SEQ ID NO. 1180, SEQ ID NO. 1181, SEQ ID NO. 1241 and SEQ ID NO. 1242, respectively. In a preferred embodiment, the siRNA targeting SEQ ID NO. 41 of TSC2 gene, or its mRNA, comprises or consists of an antisense strand which comprises or consists of at least one sequence selected from SEQ ID NO. 1427, SEQ ID NO. 1428, SEQ ID NO. 1429, SEQ ID NO. 1430 and SEQ ID NO. 1431, and a sense strand which is complementary to the antisense strand which is selected from the group consisting of SEQ ID NO. 1432, SEQ ID NO. 1433, SEQ ID NO. 1434, SEQ ID NO. 1435 and SEQ ID NO. 1436, respectively.

In preferred embodiments, the siRNA targeting SEQ ID NO. 42 of TSC2 gene, or its mRNA, comprises or consists of an antisense strand which comprises or consists of at least one sequence selected from the group consisting of SEQ ID NO. 1045, SEQ ID NO. 1046, SEQ ID NO. 1047, SEQ ID NO. 1048, SEQ ID NO. 1049, SEQ ID NO. 1050, SEQ ID NO. 1051, SEQ ID NO. 1052, SEQ ID NO. 1053, SEQ ID NO. 1054, SEQ ID NO. 1055, SEQ ID NO. 1056, SEQ ID NO. 1057, SEQ ID NO. 1058, SEQ ID NO. 1059, SEQ ID NO. 1060, SEQ ID NO. 1061, SEQ ID NO. 1062, SEQ ID NO. 1063, SEQ ID NO. 1064, SEQ ID NO. 1065, SEQ ID NO. 1066, SEQ ID NO. 1067, SEQ ID NO. 1068, SEQ ID NO. 1069, SEQ ID NO. 1074, SEQ ID NO. 1075, SEQ ID NO. 1076, SEQ ID NO. 1077, SEQ ID NO. 1078, SEQ ID NO. 1079, SEQ ID NO. 1080, SEQ ID NO. 1081, SEQ ID NO. 1082, SEQ ID NO. 1083, SEQ ID NO. 1084, SEQ ID NO. 1085, SEQ ID NO. 1086, SEQ ID NO. 1087, SEQ ID NO. 1088, SEQ ID NO. 1089, SEQ ID NO. 1090, SEQ ID NO. 1091, SEQ ID NO. 1092, SEQ ID NO. 1093, SEQ ID NO. 1094, SEQ ID NO. 1239, SEQ ID NO. 1240, SEQ ID NO. 1245, SEQ ID NO. 1246, SEQ ID NO. 1247, SEQ ID NO. 1248, SEQ ID NO. 1249, SEQ ID NO. 1250, SEQ ID NO. 1251, SEQ ID NO. 1252, SEQ ID NO. 1253, SEQ ID NO. 1254, SEQ ID NO. 1255, SEQ ID NO. 1256, SEQ ID NO. 1257, SEQ ID NO. 1258, SEQ ID NO. 1259, SEQ ID NO. 1260, SEQ ID NO. 1261, SEQ ID NO. 1262, SEQ ID NO. 1263, SEQ ID NO. 1264 and SEQ ID NO. 1265, and a sense strand which is complementary to the antisense strand which is selected from the group consisting of SEQ ID NO. 1182, SEQ ID NO. 1183, SEQ ID NO. 1184, SEQ ID NO. 1185, SEQ ID NO. 1186, SEQ ID NO. 1187, SEQ ID NO. 1188, SEQ ID NO. 1189, SEQ ID NO. 1190, SEQ ID NO. 1191, SEQ ID NO. 1192, SEQ ID NO. 1193, SEQ ID NO. 1194, SEQ ID NO. 1195, SEQ ID NO. 1196, SEQ ID NO. 1197, SEQ ID NO. 1198, SEQ ID NO. 1199, SEQ ID NO. 1200, SEQ ID NO. 1201, SEQ ID NO. 1202, SEQ ID NO. 1203, SEQ ID NO. 1204, SEQ ID NO. 1205, SEQ ID NO. 1206, SEQ ID NO. 1211, SEQ ID NO. 1212, SEQ ID NO. 1213, SEQ ID NO. 1214, SEQ ID NO. 1215, SEQ ID NO. 1216, SEQ ID NO. 1217, SEQ ID NO. 1218, SEQ ID NO. 1219, SEQ ID NO. 1220, SEQ ID NO. 1221, SEQ ID NO. 1222, SEQ ID NO. 1223, SEQ ID NO. 1224, SEQ ID NO. 1225, SEQ ID NO. 1226, SEQ ID NO. 1227, SEQ ID NO. 1228, SEQ ID NO. 1229, SEQ ID NO. 1230, SEQ ID NO. 1231, SEQ ID NO. 1241, SEQ ID NO. 1242, SEQ ID NO. 1266, SEQ ID NO. 1267, SEQ ID NO. 1268, SEQ ID NO. 1269, SEQ ID NO. 1270, SEQ ID NO. 1271, SEQ ID NO. 1272, SEQ ID NO. 1273, SEQ ID NO. 1274, SEQ ID NO. 1275, SEQ ID NO. 1276, SEQ ID NO. 1277, SEQ ID NO. 1278, SEQ ID NO. 1279, SEQ ID NO. 1280, SEQ ID NO. 1281, SEQ ID NO. 1282, SEQ ID NO. 1283, SEQ ID NO. 1284, SEQ ID NO. 1285 and SEQ ID NO. 1286, respectively.

In a preferred embodiment, the siRNA targeting SEQ ID NO. 44 of TSC2 gene, or its mRNA, comprises or consists of an antisense strand which comprises or consists of at least one sequence selected from the group consisting of SEQ ID NO. 1070, SEQ ID NO. 1071 and SEQ ID NO. 1096, and a sense strand which is complementary to the antisense strand which is selected from the group consisting of SEQ ID NO. 1207, SEQ ID NO. 1208 and SEQ ID NO. 1233, respectively.

In a preferred embodiment, the siRNA targeting SEQ ID NO. 45 of TSC2 gene, or its mRNA, comprises or consists of an antisense strand which comprises or consists of at least one sequence selected from the group consisting of SEQ ID NO. 1072, SEQ ID NO. 1073 and SEQ ID NO. 1095, and a sense strand which is complementary to the antisense strand which is selected from the group consisting of SEQ ID NO. 1209, SEQ ID NO. 1210 and SEQ ID NO. 1232, respectively.

In a preferred embodiment, the siRNA targeting SEQ ID NO. 49 of TSC2 gene, or its mRNA, comprises or consists of an antisense strand which comprises or consists of at least one sequence selected from the group consisting of SEQ ID NO. 1235 and SEQ ID NO. 1236, and a sense strand which is complementary to the antisense strand which is selected from the group consisting of SEQ ID NO. 1237 and SEQ ID NO. 1238, respectively.

In a preferred embodiment, the siRNA molecule targeting the sequence SEQ ID NO. 2 of the TSC1 gene or its corresponding mRNA comprises or consists of an oligonucleotide sequence selected from the group consisting of SEQ ID NO. 980, SEQ ID NO. 981, SEQ ID NO. 982, SEQ ID NO. 983, SEQ ID NO. 984, SEQ ID NO. 985, SEQ ID NO. 986, SEQ ID NO. 987, SEQ ID NO. 988, SEQ ID NO. 989, SEQ ID NO. 990, SEQ ID NO. 991, SEQ ID NO. 992, SEQ ID NO. 993, SEQ ID NO. 994, SEQ ID NO. 995, SEQ ID NO. 996, SEQ ID NO. 997, SEQ ID NO. 998, SEQ ID NO. 999 and SEQ ID NO. 1243, and a sense strand which is complementary to the antisense strand which is selected from the group consisting of SEQ ID NO. 1117, SEQ ID NO. 1118, SEQ ID NO. 1119, SEQ ID NO. 1120, SEQ ID NO. 1121, SEQ ID NO. 1122, SEQ ID NO. 1123, SEQ ID NO. 1124, SEQ ID NO. 1125, SEQ ID NO. 1126, SEQ ID NO. 1127, SEQ ID NO. 1128, SEQ ID NO. 1129, SEQ ID NO. 1130, SEQ ID NO. 1131, SEQ ID NO. 1132, SEQ ID NO. 1133, SEQ ID NO. 1134, SEQ ID NO. 1135, SEQ ID NO. 1136 and SEQ ID NO. 1244, respectively.

In a preferred embodiment, the siRNA molecule targeting the sequence SEQ ID NO. 1 of the TSC1 gene, or its corresponding mRNA, comprises or consists of an oligonucleotide sequence selected from the group consisting of SEQ ID NO. 961, SEQ ID NO. 962, SEQ ID NO. 963, SEQ ID NO. 964, SEQ ID NO. 965, SEQ ID NO. 966, SEQ ID NO. 967, SEQ ID NO. 968, SEQ ID NO. 969, SEQ ID NO. 970, SEQ ID NO. 971, SEQ ID NO. 972, SEQ ID NO. 973, SEQ ID NO. 974, SEQ ID NO. 975, SEQ ID NO. 976, SEQ ID NO. 977, SEQ ID NO. 978 and SEQ ID NO. 979, and a sense strand which is complementary to the antisense strand which is selected from the group consisting of SEQ ID NO. 1098, SEQ ID NO. 1099, SEQ ID NO. 1100, SEQ ID NO. 1101, SEQ ID NO. 1102, SEQ ID NO. 1103, SEQ ID NO. 1104, SEQ ID NO. 1105, SEQ ID NO. 1106, SEQ ID NO. 1107, SEQ ID NO. 1108, SEQ ID NO. 1109, SEQ ID NO. 1110, SEQ ID NO. 1111, SEQ ID NO. 1112, SEQ ID NO. 1113, SEQ ID NO. 1114, SEQ ID NO. 1115 and SEQ ID NO. 1116, respectively.

In a preferred embodiment, the siRNA molecule targeting the sequence SEQ ID NO. 8 of the TSC1 gene or its corresponding mRNA comprises or consists of an oligonucleotide sequence selected from the group consisting of SEQ ID NO: 1000, SEQ ID NO. 1001, SEQ ID NO. 1002, SEQ ID NO. 1003, SEQ ID NO. 1004, SEQ ID NO. 1005, SEQ ID NO. 1006, SEQ ID NO. 1007, SEQ ID NO. 1008, SEQ ID NO. 1009, SEQ ID NO. 1010, SEQ ID NO. 1011, SEQ ID NO. 1012, SEQ ID NO. 1013, SEQ ID NO. 1014, SEQ ID NO. 1015, SEQ ID NO. 1016, SEQ ID NO. 1017, SEQ ID NO. 1018, SEQ ID NO. 1019, SEQ ID NO. 1020 and SEQ ID NO: 1021, and a sense strand which is complementary to the antisense strand which is selected from the group consisting of SEQ ID NO: 1137, SEQ ID NO. 1138, SEQ ID NO. 1139, SEQ ID NO. 1140, SEQ ID NO. 1141, SEQ ID NO. 1142, SEQ ID NO. 1143, SEQ ID NO. 1144, SEQ ID NO. 1145, SEQ ID NO. 1146, SEQ ID NO. 1147, SEQ ID NO. 1148, SEQ ID NO. 1149, SEQ ID NO. 1150, SEQ ID NO. 1151, SEQ ID NO. 1152, SEQ ID NO. 1153, SEQ ID NO. 1154, SEQ ID NO. 1155, SEQ ID NO. 1156, SEQ ID NO. 1157 and SEQ ID NO: 1158, respectively.

The RNAi oligonucleotides or siRNA molecules as described above may be delivered to the cell interior in their native structure using methods known in the art. For example, when studying in vitro gene silencing, these compounds are administered using standard transfection reagents. To achieve effects in vivo these compounds may also be administered naked or using delivery enhancing agents such as for example liposomes, conjugation with a specific moiety, etc. although many different alternatives are known in the art, and are used differently depending on the desired target site within the body.

Alternatively, the RNAi oligonucleotides or siRNA molecules of the various aspects of the invention can be expressed within cells from eukaryotic promoters. Recombinant vectors capable of expressing the RNAi oligonucleotides or siRNA molecules can be delivered and persist in target cells. Alternatively, vectors can be used that provide for transient expression of nucleic acid molecules. Such vectors can be repeatedly administered as necessary. Once expressed, the RNAi oligonucleotide or siRNA molecule interacts with the target mRNA and generates an RNA interfering response. The siRNA molecules or RNAi oligonucleotides produced in this manner are often termed shRNA (short hairpin RNA), as their sense and antisense strands are joined by a small loop of nucleotides. Delivery of siRNA molecules expressing vectors can be systemic, such as by intravenous or intra-muscular administration, by administration to target cells ex-planted from a subject followed by reintroduction into the subject, or by any other means that would allow for introduction into the desired target cell.

A further aspect of the invention relates to the use of RNAi oligonucleotides comprising or consisting of an siRNA targeting at least one sequence of the TSC2 gene selected from the group consisting of SEQ ID NO. 41 to SEQ ID NO. 64, as defined above, and preferably any of the nucleotide sequences SEQ ID NO. 41 to SEQ ID NO. 49, in the preparation of a medicament for use in a method of treatment of a retinal dystrophy, which includes inherited retinal dystrophies such as retinitis pigmentosa, Leber's congenital amaurosis or Alström syndrome. More preferably, said siRNA targets at least one sequence which is SEQ ID NO. 41 or SEQ ID NO. 42, which can comprise or consists of any of antisense sequences from the group consisting of SEQ ID NO. 105, SEQ ID NO. 106, SEQ ID NO. 385 - SEQ ID NO. 392, SEQ ID NO. 737 - SEQ ID NO. 741, SEQ ID NO. 880, SEQ ID NO. 881, SEQ ID NO. 884, SEQ ID NO. 885, SEQ ID NO. 888, SEQ ID NO. 889, SEQ ID NO. 892 - SEQ ID NO. 903, SEQ ID NO. 1022 - SEQ ID NO. 1069, SEQ ID NO. 1074 - SEQ ID NO. 1094, SEQ ID NO. 1239, SEQ ID NO. 1240, SEQ ID NO. 1245 - SEQ ID NO. 1265, SEQ ID NO. 1293, SEQ ID NO. 1345, SEQ ID NO. 1427 - SEQ ID NO. 1431. The method comprises inhibiting expression of TSC2 in a patient. The term inhibition is used to indicate a decrease or downregulation of expression or activity. However, in addition to the inhibition of TSC2, the method may comprise inhibiting also the expression of TSC1, for which the RNAi oligonucleotide can comprise further an siRNA molecule targeting any of the nucleotide sequences SEQ ID NO. 1 to SEQ ID NO. 40, as defined above, and preferably any of SEQ ID NO. 1 to SEQ ID NO. 10, or of SEQ ID NO. 1 to SEQ ID NO. 8. In preferred embodiments, this further siRNA targets any of the sequences SEQ ID NO. 2, SEQ ID NO. 1 or SEQ ID NO. 7 of the TSC1 gene, which can comprise or consists of any of antisense sequences from the group consisting of SEQ ID NO. 65, SEQ ID NO. 66, SEQ ID NO. 71, SEQ ID NO. 193 - SEQ ID NO. 200, SEQ ID NO. 217 - SEQ ID NO. 224, SEQ ID NO. 641, SEQ ID NO. 642, SEQ ID NO. 650, SEQ ID NO. 651, SEQ ID NO. 855, SEQ ID NO. 856, SEQ ID NO. 858, SEQ ID NO. 860, SEQ ID NO. 861, SEQ ID NO. 863, SEQ ID NO. 865, SEQ ID NO. 866, SEQ ID NO. 868, SEQ ID NO. 870 - SEQ ID SEQ ID NO. 873, SEQ ID NO. 876, SEQ ID NO. 877, SEQ ID NO. 961 - SEQ ID SEQ ID NO. 999, and SEQ ID SEQ ID NO. 1243.

For inhibiting the expression and/or activity of the TSC2 and TSC1 genes, the RNAi oligonucleotide of this and the other aspects of the invention can comprise or consist of at least an siRNA molecule targeting any of the nucleotide sequences selected from any of the group consisting of SEQ ID NO. 41 (from the TSC2 gene), SEQ ID NO. 42 (from the TSC2 gene), SEQ ID NO. 3 (from the TSC1 gene) and SEQ ID NO. 8 (from the TSC1 gene), or any combination of said siRNA molecules. Advantageously, siRNA molecules targeting any of these particular sequences seem to have a high silencing effect in reducing the cellular expression levels of its target gene (TSC2 or TSC1), some of them showing decreases of its expression in cells ≥ 65-70%, but also to reduce in a certain extent the expression levels of the other gene (TSC1 or TSC2, respectively), which can be reduced in cells by up to 20-30% in case of human cells derived from the eye or the retina. This makes these siRNA molecules, as well as their related RNAi oligonucleotides, medicaments or compositions comprising them, particularly useful, and especially for the intended uses related to the present invention.

Within the context of the present invention, to "specifically target" a sequence the siRNA of the invention preferably comprises at least the same seed sequence. Thus, any sequence according to the invention that specifically targets at least one sequence selected from the group consisting of SEQ ID NO. 1 to SEQ ID NO. 64 is preferably identical in positions 2-8 of the antisense strand. More preferably, said selected sequence specifically targeted is SEQ ID NO. 41 or SEQ ID NO. 42.

Retinal dystrophies are a heterogeneous group of ocular diseases or conditions that cause progressive and severe loss of vision by altering the anatomy and/or function of the retina. These conditions can cause damage to the photoreceptor cells, either, predominantly, the cones (responsible for detailed vision and colour), rods (responsible for night and peripheral vision) or both at the same time. Preferably, the retinal dystrophy is characterised by dysfunction or progressive loss of photoreceptors. The majority of retinal dystrophies are diseases located exclusively in the eye, but can sometimes be associated with extraocular manifestations (Usher syndrome, Bardet-Biedl syndrome), in which case they are referred to as syndromic retinal dystrophies. In one embodiment, the retinal dystrophy is selected from the group comprising retinitis pigmentosa, Leber's congenital amaurosis or Alström syndrome and combinations thereof.

Also provided is a method of treatment of a retinal dystrophy characterised by dysfunction or progressive loss of photoreceptors. The method comprises inhibiting expression of TSC2 in a patient. The method may comprise administering an RNAi oligonucleotide or siRNA targeting at least one sequence selected from the group consisting of SEQ ID NO. 41 to SEQ ID NO. 49. More preferably, said targeted sequence is SEQ ID NO. 41 or SEQ ID NO. 42. In embodiments of this aspect, preferred RNAi oligonucleotides or siRNA molecules targeting such sequences can be any of the described herein for any one of the different aspects of the invention.

Therapeutic treatment with RNAi oligonucleotides or siRNAs directed against TSC2 and/or TSC1 mRNA is expected to be beneficial over traditional small molecule therapeutic agents due to its specificity, stability, potency, natural mechanism of action, and uniform chemical nature with other siRNA agents targeting the same or different gene targets since they differ only in nucleotide sequence. Treatments based on RNAi oligonucleotides or siRNAs block the synthesis of the target protein which will provoke a sustained reduction of the TSC2 and/or TSC1 gene expression and a longer-lasting effect that can diminish the frequency of repeating intravitreal injections. This is especially important in cases such as retinal dystrophies, comprising but not limited to retinitis pigmentosa, Leber's congenital amaurosis or Alström syndrome, which are incurable conditions which may require continuous intravitreal injections during their treatment. Repetitive intraocular injections increase the risk of deleterious side effects which include, among others, increased pressure in the eye, inflammation, bleeding, infection, damage to the retina or surrounding nerves or structures, vision loss, but also side effects from the medicines that are used during the procedure, such as those derived from the use of antibiotics or drugs to dilate the pupils. Besides, siRNAs can be engineered to silence the expression of mutant gene alleles differing from wild type alleles by as little as a single nucleotide. Thus, treatments based on RNAi oligonucleotides or siRNA can advantageously modulate the expression of genes having point mutations to slow or even prevent disease, by inactivating disease mutant alleles selectively while allowing continued expression of the wild type protein.

Bearing in mind the preparation of such a medicament, the siRNA of the various aspects of the present invention may be formulated as a pharmaceutical composition. Preferably, the compositions and formulations of said siRNAs may be administered locally to the organ of interest. In a preferred embodiment they may be formulated for topical administration to the eye, preferably to the corneal surface of the eye. Application to the corneal surface may, for example be in the form of eye drops, a gel, ocular ointment, lotion, cream or ocular inserts. Other administration forms to the eye may include injection into the eye. In another preferred embodiment the compositions and formulations of said siRNAs may be administered directly to the eye by ocular tissue injection such as periocular, conjunctival, subtenon, intracameral, intravitreal, intraocular, anterior or posterior juxtascleral, subretinal, subconjunctival, retrobulbar, or intracanalicular injections; by direct application to the eye using a catheter or other placement device such as a retinal pellet, intraocular insert, suppository or an implant comprising a porous, non-porous, or gelatinous material; or by a slow release device in the cul-de-sac or implanted adjacent to the sclera (transscleral) or in the sclera (intrascleral) or within the eye. Intracameral injection may be through the cornea into the anterior chamber to allow the agent to reach the trabecular meshwork. Intracanalicular injection may be into the venous collector channels draining Schlemm's canal or into Schlemm's canal.

In one embodiment, the pharmaceutical compositions may be administered into the eye, for example into the vitreous chamber of the eye, by intravitreal injection, such as with pre-filled syringes in ready-to-inject form for use by medical personnel. Despite the known multiple risks associated to intravitreal injections, intravitreal administration has become the gold standard for treatment of many retinal diseases, including neovascular age-related macular degeneration (AMD), diabetic retinopathy, and retinal vein occlusion, in which the improvement in vision or the prevention of worsening vision are among the pursued benefits of the treatment. By intravitreal administration of the RNAi oligonucleotide or the related composition or medicament, the method can reduce the expression of the target gene in an ocular tissue. Intravitreal administration route allows direct delivery of the injected compounds into the eye closer to the cells and tissues of the retina, where it is expected that the RNAi oligonucleotide or siRNA molecule will act.

A further preferred embodiment of the various aspects of the present invention relates to a siRNA specifically targeting at least one sequence selected from the group consisting of SEQ ID NO 41 to SEQ ID NO. 49 as any one described in the preceding paragraphs, for use as a medicament in the treatment of a retinal dystrophy, which can be one of the described above, including those mainly characterised by dysfunction or progressive loss of photoreceptors. More preferably, the siRNA specifically targets at least one sequence selected from SEQ ID NO. 41 or SEQ ID NO. 42. As described above, it may be a siRNA comprising or consisting of a 19-nucleotide double-stranded structure targeting at least one sequence selected from the group consisting of SEQ ID NO. 41 to SEQ ID NO. 49. This siRNA may be blunt ended. Preferably, the siRNA comprises or consists of at least one sequence selected from the group consisting of SEQ ID NO. 105 to SEQ ID NO. 113, and more preferably comprises or consists of SEQ ID NO. 105 or SEQ ID NO. 106.

In preferred embodiments of the various aspects of the present invention the siRNA molecule or molecules comprised in the RNAi oligonucleotide comprise a double-stranded region of 15, 16, 17, 18 or 19 nucleotides, and preferably being siRNA comprising a double-stranded region of 19 nucleotides which optionally can be blunt-ended.

Notwithstanding the above, the RNAi oligonucleotides or siRNAs of the various aspects of the present invention may be used to silence TSC2 and/or TSC1 expression in tissues other than the eye. Consequently, said siRNAs should be formulated accordingly. For example, an RNAi oligonucleotide or siRNA molecule can comprise a delivery vehicle, including liposomes, for administration to a subject. Carriers and diluents and their salts can be present in pharmaceutically acceptable formulations and compositions. Nucleic acid molecules can be administered to cells by a variety of methods known to those of skill in the art, including, but not restricted to, encapsulation in liposomes, by iontophoresis, or by incorporation into other vehicles, such as biodegradable polymers, hydrogels, cyclodextrins, poly (lactic-co-glycolic) acid (PLGA) and PLCA microspheres, biodegradable nanocapsules, and bioadhesive microspheres, or by proteinaceous vectors. Intracellular delivery components can be also viral components which include, but are not limited to, a fusogenic viral peptide to disrupt endosomes, allowing the nucleic acid to avoid lysosomal degradation, viral proteins to maintain expression (e.g. integrase, LTR elements, rep proteins, oriP and EBNA-1 proteins) or viral components that interact with the cell surface proteins . Suitable viral intracellular delivery components include, but are not limited to, retroviruses, herpes simplex viruses, adenoviruses and preferably adeno-associated viruses (AAV). In one embodiment, the RNAi oligonucleotide or siRNA molecule is delivered through a cell-specific siRNA carrier that combines components of a virus and liposomes. In another embodiment, the nucleic acid molecules of the invention can also be formulated or complexed with polyethyleneimine and derivatives thereof, such as polyethyleneimine- polyethyleneglycol-N- acetylgalactosamine (PEI-PEG-GAL) or polyethyleneimine-polyethyleneglycol-tri-N-acetylgalactosamine (PEI-PEG-triGAL) derivatives. In preferred embodiments, compositions of the invention are aqueous solutions, specifically water solutions or saline solutions such as phosphate-buffered saline (PBS) with a pH range of about 7.0 to about 7.4, preferably with a pH of 7.2 + 0.5.

Another aspect of the invention refers to pharmaceutical compositions, preferably suitable for ocular delivery.

The present invention also includes pharmaceutical compositions and formulations which include the siRNA molecules or RNAi oligonucleotides of the invention. In one embodiment, provided herein are pharmaceutical compositions suitable for ocular delivery containing a TSC2 siRNA and/or a TSC1 siRNA, or an RNAi oligonucleotide of the invention comprising them, as described herein, and a pharmaceutically acceptable carrier. The pharmaceutical compositions containing the siRNA compound(s) are useful for treating a retinal dystrophy. The pharmaceutical compositions of the invention may be administered in dosages sufficient to inhibit expression of the TSC2 gene and/or the TSC1 gene in an eye cell.

For ocular administration, ointments or droppable liquids may be delivered by ocular delivery systems known to the art such as applicators or eye droppers. Such compositions can include mucomimetics such as hyaluronic acid, chondroitin sulfate, hydroxypropyl methylcellulose or poly(vinyl alcohol), preservatives such as sorbic acid, EDTA or benzylchronium chloride, and the usual quantities of diluents and/or carriers.

For ocular administration, the RNAi oligonucleotides or siRNA molecules of the invention may be applied to the surface of the eye or nearby tissue, e.g., the inside of the eyelid. They can be applied topically, e.g., by spraying, in drops, as an eyewash, or an ointment. Administration can be provided by the subject or by another person, e.g., a health care provider. The medication can be provided in measured doses or in a dispenser which delivers a metered dose. The medication can also be administered to the interior of the eye and can be introduced by a needle or other delivery device which can introduce it to a selected area or structure.

In one embodiment, the RNAi oligonucleotide or siRNA molecules of the invention are administered to an ocular cell in a pharmaceutical composition by a topical route of administration.

In one embodiment, the pharmaceutical composition suitable for ocular delivery may include an RNAi oligonucleotide or an siRNA compound mixed with a topical delivery agent. The topical delivery agent can be a plurality of microscopic vesicles. The microscopic vesicles can be liposomes. In some embodiments the liposomes are cationic liposomes.

In another embodiment, the RNAi oligonucleotide or siRNA molecule is admixed with a topical penetration enhancer, such as, a fatty acid, a bile salt, a chelating agent, or a penetration enhancer. The fatty acid can be arachidonic acid, oleic acid, lauric acid, caprylic acid, capric acid, myristic acid, palmitic acid, stearic acid, linoleic acid, linolenic acid, dicaprate, tricaprate, monolein, dilaurin, glyceryl 1-monocaprate, 1-dodecylazacycloheptan-2-one, an acylcarnitine, an acylcholine, or a C1-10 alkyl ester, monoglyceride, diglyceride or pharmaceutically acceptable salt thereof. The bile salt can be cholic acid, dehydrocholic acid, deoxycholic acid, glucholic acid, glycholic acid, glycodeoxycholic acid, taurocholic acid, taurodeoxycholic acid, chenodeoxycholic acid, ursodeoxycholic acid, sodium tauro-24,25-dihydro-fusidate, sodium glycodihydrofusidate, polyoxyethylene-9-lauryl ether or a pharmaceutically acceptable salt thereof. The chelating agent can be, for example, EDTA, citric acid, a salicyclate, a N-acyl derivative of collagen, laureth-9, an N-amino acyl derivative of a beta-diketone or a mixture thereof. The penetration enhancer can be a surfactant, e.g., an ionic or nonionic surfactant, including sodium lauryl sulfate, polyoxyethylene-9-lauryl ether, polyoxyethylene-20-cetyl ether, a perfluorchemical emulsion or mixture thereof. Alternatively, the penetration enhancer can be a glycol, a pyrrol, an azone, or a terpene, or can be also selected from a group consisting of unsaturated cyclic ureas, 1-alkyl-alkones, 1-alkenylazacyclo-alakanones, steroidal antiinflammatory agents and mixtures thereof.

In another aspect, the invention provides a pharmaceutical composition suitable for ocular administration including an RNAi oligonucleotide or an siRNA molecule and a delivery vehicle.

In one embodiment, the delivery vehicle can deliver an RNAi oligonucleotide or siRNA molecule to an ocular cell by a topical route of administration. The delivery vehicle can be microscopic vesicles, such as micelles or liposomes, e.g. cationic liposomes.

In another aspect, the invention provides a pharmaceutical composition including an RNAi oligonucleotide or siRNA molecule in an injectable dosage form. In one embodiment, the injectable dosage form of the pharmaceutical composition includes sterile aqueous solutions or dispersions and sterile powders. In some embodiments the sterile solution can include a diluent such as water; saline solution; fixed oils, polyethylene glycols, glycerin, or propylene glycol.

The RNAi oligonucleotides or siRNA molecules of the invention can be incorporated into pharmaceutical compositions suitable for ocular administration. Such compositions typically include one or more species of RNAi oligonucleotides or siRNA targeting TSC2 and/or TSC1 genes and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration to an ocular cell. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

In certain embodiments, the RNAi oligonucleotides or siRNA molecules may be delivered directly to the eye by ocular tissue injection such as periocular, conjunctival, subtenon, intracameral, intravitreal, intraocular, anterior or posterior juxtascleral, subretinal, subconjunctival, retrobulbar, or intracanalicular injections; by direct application to the eye using a catheter or other placement device such as a retinal pellet, intraocular insert, suppository or an implant comprising a porous, non-porous, or gelatinous material; by topical ocular drops or ointments; or by a slow release device in the cul-de-sac or implanted adjacent to the sclera (transscleral) or in the sclera (intrascleral) or within the eye. Intracameral injection may be through the cornea into the anterior chamber to allow the agent to reach the trabecular meshwork. Intracanalicular injection may be into the venous collector channels draining Schlemm's canal or into Schlemm's canal.

In embodiments, the RNAi oligonucleotides or siRNA molecules may be administered into the eye, for example the vitreous chamber of the eye, by intravitreal injection, such as with pre-filled syringes in ready-to-inject form for use by medical practitioners.

For ophthalmic delivery, the RNAi oligonucleotides or siRNA molecules can be combined with ophthalmologically acceptable preservatives, co-solvents, surfactants, viscosity enhancers, penetration enhancers, buffers, sodium chloride, or water to form an aqueous, sterile ophthalmic suspension or solution. Solution formulations may be prepared by dissolving the RNAi oligonucleotide or siRNA molecule of the invention in a physiologically acceptable isotonic aqueous buffer, which may further include an acceptable surfactant to assist in dissolving the siRNA or RNAi oligonucleotide. Additionally, viscosity building agents may be added to improve the retention of the siRNA compounds, such as hydroxymethyl cellulose, hydroxyethyl cellulose, methylcellulose, polyvinylpyrrolidone, or the like.

The RNAi oligonucleotides or siRNA molecules of the invention can be alternatively combined with a preservative in a vehicle, such as, mineral oil, liquid lanolin, or white petrolatum to form an sterile ophthalmic ointment formulation, or in a hydrophilic base to form a sterile ophthalmic gel formulation.

The pharmaceutical composition can be administered once daily, and dosage units can also be formulated for delivery over several days, e.g., using a conventional sustained release formulation which provides sustained release of the RNAi oligonucleotide or siRNA molecule over a several days period.

In other embodiments, a single dose of the pharmaceutical compositions can be long lasting. In some embodiments of the invention, a single dose of the pharmaceutical compositions of the invention is administered bi-monthly. In other embodiments, a single dose of the pharmaceutical compositions of the invention is administered monthly. In still other embodiments, a single dose of the pharmaceutical compositions of the invention is administered quarterly. In still other embodiments, a single dose of the pharmaceutical compositions of the invention is administered bi-annually or annually.

The skilled artisan will appreciate that certain factors can influence the dosage and timing required to effectively treat a subject, including but not limited to the severity of the disease or disorder, previous treatments, the general health and/or age of the subject, and other diseases present. Moreover, treatment of a subject with a therapeutically effective amount of a composition can include a single treatment or a series of treatments. Estimates of effective dosages and in vivo half-lives for the individual RNAi oligonucleotides or siRNA molecules encompassed by the invention can be made using conventional methodologies or on the basis of in vivo testing using an appropriate animal model.

The present invention also provides methods for treating or preventing a retinal dystrophy or an ocular disease related to photoreceptor cell death or retinal disorganization in a subject. The methods include topically and/or intraocularly administering to the subject a therapeutically effective amount or prophylactically effective amount of an siRNA molecule or RNAi oligonucleotide of the invention.

As used herein, a "subject" is an animal, such as a mammal, including a primate (such as a human, a non-human primate, e.g., a monkey, a macaque, and a chimpanzee), or a non-primate (such as a mouse, a rat, a hamster, a guinea pig, a pig, a rabbit, and a dog). A subject may include a transgenic organism.

In embodiments, the subject is a human, such as a human being treated or assessed for an ocular disease, disorder or condition that would benefit from reduction in TSC2 and/or TSC1 gene expression in an ocular cell; a human at risk for a disease, disorder or condition that would benefit from reduction in TSC2 and/or TSC1 gene expression in an ocular cell; a human having an ocular disease, disorder or condition that would benefit from reduction in TSC2 and/or TSC1 gene expression in an ocular cell; and/or human being treated for a disease, disorder or condition that would benefit from reduction in TSC2 and/or TSC1 gene expression in an ocular cell, as described herein.

In some embodiments, the subject is suffering from a retinal dystrophy which appears only in the eye or is associated to a syndrome, such as, retinitis pigmentosa (RP), achromatopsia, choroideremia, retinoschisis, cone dystrophy, cone-rod dystrophy, rod dystrophy, blue cone monochromatism, familial exudative vitreoretinopathy, Leber congenital amaurosis (LCA), Best disease, Stargardt disease, congenital stational night blindness (CSNB), Oguchi disease, Alström syndrome, Stickler syndrome, and Usher syndrome.

In one embodiment, the siRNA molecules or RNAi oligonucleotides of the invention are administered to subjects suffering from retinitis pigmentosa. In another embodiment, the siRNA compounds of the invention are administered to subjects suffering from Leber congenital amaurosis. In another embodiment, the siRNA compounds of the invention are administered to subjects suffering from Alström syndrome.

In one aspect, the siRNA molecules or RNAi oligonucleotides of the invention are intraocularly administered to subjects suffering from a retinal dystrophy, such as retinitis pigmentosa, Leber congenital amaurosis or cone-rod dystrophy associated to Alström syndrome.

Intraocular administration may be via periocular, conjunctival, subtenon, intracameral, intravitreal, intraocular, anterior or posterior juxtascleral, subretinal, subconjunctival, retrobulbar, or intracanalicular injection. In embodiments, intraocular administration is via intravitreal injection.

In some embodiments, the siRNA molecules or RNAi oligonucleotides of the invention are administered to a subject in an amount effective to inhibit TSC2 and/or TSC1 expression in an ocular cell, such as an RPE (retinal pigmented epithelial) and/or photoreceptor (cone and/or rod) cell within the subject. The amount effective to inhibit TSC2 and/or TSC1 expression in an ocular cell within a subject may be assessed using methods that involve assessment of the inhibition of TSC2 and/or TSC1 mRNA, TSC2 and/or TSC1 protein, or related variables, such as photoreceptor cell death.

In some embodiments, the siRNA molecule or RNAi oligonucleotide of the invention is administered to a subject in a therapeutically or prophylactically effective amount.

"Therapeutically effective amount," as used herein, is intended to include the amount of a siRNA molecule or RNAi oligonucleotide that, when administered to a patient for treating a retinal dystrophy, is sufficient to effect treatment of the disease (e.g., by diminishing, ameliorating, or maintaining the existing disease or one or more symptoms of disease). The "therapeutically effective amount" may vary depending on the siRNA molecule or RNAi oligonucleotide, how the compound is administered, the disease and its severity and the history, age, weight, family history, genetic makeup, stage of pathological processes mediated by photoreceptor cell death and morphological retinal disorganization, the types of preceding or concomitant treatments, if any, and other individual characteristics of the patient to be treated.

"Prophylactically effective amount," as used herein, is intended to include the amount of a siRNA molecule or RNAi oligonucleotide that, when administered to a subject who does not yet experience or display symptoms of a retinal dystrophy, but who may be predisposed to the disease, is sufficient to prevent or ameliorate the disease or one or more symptoms of the disease.

Clinical diagnosis can help to identify individuals that could benefit from treatment with the RNAi oligonucleotides or siRNA of the present invention, although genomic testing or genotyping (e.g. by next-generation sequencing or NGS) also can play a key role in the management and identification of individuals or patients with retinal dystrophies. Genomic testing can be particularly important for patients with inherited retinal dystrophies or for subjects carrying known disease-causing gene variants, where early and accurate diagnosis are crucial, and especially in the case of subjects who could develop signs and symptoms at late stages of the disease, some of them may experience a delayed onset, or symptoms could even be prevented, with the treatment or the administration of the RNAi oligonucleotide or siRNA molecule of the invention.

Without limiting purposes, a list of several known mapped and identified genes by retinal disease is shown for example in the following table. The table below (Table 1) is a list of all diseases associated with each gene, sometimes assigning several diseases per gene.

**Table 1.**

| Retinal disease | Mapped and identified genes |
|---|---|
| Retinitis pigmentosa, autosomal dominant | ADIPOR1, ARL3, BEST1, CA4, CRX, FSCN2, GUCA1B, HK1, IMPDH1, IMPG1, KIF3B, KLHL7, NR2E3, NRL, PRPF3, PRPF4, PRPF6, PRPF8, PRPF31, PRPH2, RDH12, RHO, ROM1, RP1, RP9, RPE65, SAG, SEMA4A, SNRNP200, SPP2, TOPORS |
| Retinitis pigmentosa, autosomal recessive | ABCA4, AGBL5, AHR, ARHGEF18, ARL6, ARL2BP, BBS1, BBS2, BEST1, C8orf37, CERKL, CLCC1, CLRN1, CNGA1, CNGB1, CRB1, CWC27, CYP4V2, DHDDS, DHX38, EMC1, ENSA, EYS, FAM161A, GPR125, HGSNAT, IDH3B, IFT140, IFT172, IMPG2, KIAA1549, KIZ, LRAT, MAK, MERTK, MVK, NEK2, NEUROD1, NR2E3, NRL, PCARE, PDE6A, PDE6B, PDE6G, POMGNT1, PRCD, PROM1, PROS1, RBP3, REEP6, RGR, RHO, RLBP1, RP1, RP1L1, RPE65, SAG, SAMD11, SLC7A14, SPATA7, TRNT1, TTC8, TULP1 , USH2A, ZNF408, ZNF513 |
| Retinitis pigmentosa, X-linked | OFD1, RP2, RPGR |
| Leber congenital amaurosis, autosomal dominant | CRX, IMPDH1, OTX2 |
| Leber congenital amaurosis, autosomal recessive | AIPL1, CABP4, CCT2, CEP290, CLUAP1, CRB1, CRX, DTHD1, GDF6, GUCY2D, IFT140, IQCB1, KCNJ13, LCA5, LRAT, NMNAT1, PRPH2, RD3, RDH12, RPE65, RPGRIP1, SPATA7, TULP1 |
| Bardet-Biedl syndrome, autosomal recessive | ADIPOR1, ARL6, BBIP1, BBS1, BBS2, BBS4, BBS5, BBS7, BBS9, BBS10, BBS12, C8orf37, CEP19, CEP290, IFT172, IFT27, INPP5E, LZTFL1, MKKS, MKS1, NPHP1, SDCCAG8, TRIM32, TTC8 |
| Chorioretinal atrophy or degeneration, autosomal dominant | PRDM13, RGR, TEAD1 |
| Cone or cone-rod dystrophy, autosomal dominant | AIPL1, CRX, GUCA1A, GUCY2D, PITPNM3, PROM1, PRPH2, RIMS1, SEMA4A, UNC119 |
| Cone or cone-rod dystrophy, autosomal recessive (incl. achromatopsia | ABCA4, ADAM9, ATF6, C21 orf2, C8orf37, CACNA2D4, CDHR1, CEP78, CERKL, CNGA3, CNGB3, CNNM4, DYNC2I2, GNAT2, IFT81, KCNV2, PDE6C, PDE6H, POC1B, RAB28, RAX2, RDH5, RPGRIP1, SLC4A7, TTLL5 |
| Cone or cone-rod dystrophy, X-linked | CACNA1F, RPGR |
| Congenital stationary night blindness, autosomal dominant | GNAT1, PDE6B, RHO |
| Congenital stationary night blindness, autosomal recessive | CABP4, GNAT1, GNB3, GPR179, GRK1, GRM6, LRIT3, RDH5, SAG, SLC24A1, TRPM1 |
| Congenital stationary night blindness, X-linked | CACNA1F, NYX |
| Macular degeneration, autosomal dominant | BEST1, C1 QTNF5, CTNNA1, EFEMP1, ELOVL4, FSCN2, GUCA1B, HMCN1, IMPG1, OTX2, PRDM13, PROM1, PRPH2, RP1L1, TIMP3 |
| Macular degeneration, autosomal recessive | ABCA4, CFH, DRAM2, IMPG1, MFSD8 |
| Macular degeneration, X-linked | RPGR |
| Ocular-retinal developmental disease, autosomal dominant | VCAN |
| Optic atrophy, autosomal dominant | AFG3L2, MFN2, MIEF1, NR2F1, OPA1 |
| Optic atrophy, autosomal recessive | ACO2, NBAS, RTN4IP1, TMEM126A |
| Optic atrophy, X-linked | TIMM8A |
| Syndromic/systemic diseases with retinopathy, autosomal dominant | ABCC6, AFG3L2, ATXN7, COL1 1A1, COL2A1, JAG1, KCNJ13, KIF11, MFN2, OPA3, PAX2, TREX1, VCAN |
| Syndromic/systemic diseases with retinopathy, autosomal recessive (including retinitis pigmentosa in Alström syndrome caused by disease-causing mutations in the ALMS1 gene) | ABCC6, ABHD12, ACBD5, ACO2, ADAMTS18, ADIPOR1, AFG3L2, AHI1, ALMS1, CC2D2A, CEP164, CEP290, CLN3, COL9A1, CSPP1, CWC27, ELOVL4, EXOSC2, FLVCR1, GNPTG, HARS, HGSNAT, HMX1, IFT140, IFT81, INPP5E, INVS, IQCB1, LAMA1, LRP5, MKS1, MTTP, NPHP1, NPHP3, NPHP4, OPA3, PANK2, PCYT1A, PEX1, PEX2, PEX7, PHYH, PLK4, PNPLA6, POC5, POC1B, PPT1, PRPS1, RDH11, RIMS2, RPGRIP1L, SDCCAG8, SLC25A46, TMEM216, TMEM237, TRNT1, TTPA, TUB, TUBGCP4, TUBGCP6, WDPCP, WDR19, WFS1, ZNF423 |
| Syndromic/systemic diseases with retinopathy, X-linked | OFD1, TIMM8A |
| Usher syndrome, autosomal recessive | ABHD12, ADGRV1, ARSG, CDH23, CEP250, CEP78, CIB2, CLRN1, ESPN, HARS1, MYO7A, PCDH15, USH1C, USH1G, USH2A, WHRN |
| Other retinopathy, autosomal dominant | BEST1, CAPN5, CRB1, ELOVL1, FZD4, ITM2B, KIF3B, LRP5, MAPKAPK3, MIR204, OPN1SW, RB1, RCBTB1, RGR, TSPAN12, ZNF408 |
| Other retinopathy, autosomal recessive | ASRGL1, BEST1, C12orf65, CDH3, CNGA3, CNGB3, CNNM4, CYP4V2, DYNC2H1, LRP5, MFRP, MVK, NBAS, NR2E3, OAT, PLA2G5, PROM1, RBP4, RCBTB1, RGS9, RGS9BP, RLBP1 |
| Other retinopathy, mitochondrial | KSS, LHON, MT-ATP6, MT-TH, MT-TL1, MT-TP, MT-TS2 |
| Other retinopathy, X-linked (incl. blue cone monochromatism) | CACNA1F, CHM, DMD, NDP, OPN1LW, OPN1MW, PGK1, RS1 |

Symptoms that may be ameliorated include decreased visual acuity, decreased night vision, decreased peripheral vision, photosensitivity, poor color discrimination, progressive attenuation of the retinal vessels, degeneration of photoreceptor cells, retinal pigment epithelial defects (RPE) in macula or fovea, and other ophthalmoscopic symptoms or conditions associated with retinal dystrophies. Ameliorating the disease includes slowing the course of the disease or reducing the severity of later-developing disease. The "prophylactically effective amount" may vary depending on the siRNA compound or RNAi oligonucleotide, how the compound is administered, the degree of risk of disease, and the history, age, weight, family history, genetic makeup, the types of preceding or concomitant treatments, if any, and other individual characteristics of the patient to be treated.

A "therapeutically-effective amount" or "prophylacticaly effective amount" also includes an amount of an siRNA molecule or RNAi oligonucleotide that produces some desired local or systemic effect at a reasonable benefit/risk ratio applicable to any treatment. siRNA molecules or RNAi oligonucleotides employed in the methods of the present invention may be administered in a sufficient amount to produce a reasonable benefit/risk ratio applicable to such treatment.

The dose of an siRNA molecule or RNAi oligonucleotide that is administered to a subject may be tailored to balance the risks and benefits of a particular dose, for example, to achieve a desired level of TSC2 and/or TSC1 gene suppression (as assessed, e.g., based on TSC2 and/or TSC1 mRNA suppression, or TSC2 and/or TSC1 protein expression reduced) or a desired therapeutic or prophylactic effect (as assessed, e.g., based on improvements or maintenance of the visual function, or on reductions in the loss of visual function, or on improvements or maintenance of the retinal morphological structure, or on reductions in the retinal structure disorganization, or on maintenance of the number of photoreceptor cells, or on reductions in the photoreceptor cell death), while at the same time avoiding undesirable side effects. Assessment of the visual function can be monitored by visual acuity testing (e.g. minimal angle of resolution (MAR), logMAR, or ETDRS letters), low-luminance visual acuity (LLVA) testing, colour vision testing (e.g. colour discrimination can be tested using the Farnsworth-Munsell 100-Hue arrangement test under optimum lighting conditions), contrast sensitivity testing, visual field testing (e.g. static or automated perimetry, semi-automated kinetic perimetry (SKP), kinetic perimetry or Goldmann visual field (GVF)), electroretinogram (e.g. full-field electroretinogram (ERG) or multifocal ERG), multi-luminance mobility test (MLMT), or full-field stimulus threshold (FST). Assessment of the retinal structure can be evaluated by optical coherence tomography (OCT), by fundus autofluorescence, by adaptive optics scanning laser ophthalmoscopy (e.g. conventional confocal adaptive optics scanning laser ophthalmoscopy (AOSLO), split-detector AOSLO or other non-confocal AOSLO methods).

In some embodiments, the siRNA molecules or RNAi oligonucleotides are administered to the subject intravitreally. In other embodiments, the siRNA compounds are administered to the subject topically onto the corneal surface of the eye. In particular embodiments, a dose of the siRNA molecule or RNAi oligonucleotide is contained in a volume of a pharmaceutically acceptable carrier.

In some embodiments, the siRNA molecule or RNAi oligonucleotide is administered to a subject in an amount effective to inhibit TSC2 and/or TSC1 expression in an ocular cell within the subject. The amount effective to inhibit TSC2 and/or TSC1 expression in an ocular cell within a subject may be assessed using methods discussed above, including methods that involve assessment of the inhibition of TSC2 and/or TSC1 mRNA, TSC2 and/or TSC1 protein levels, or related variables, such as photoreceptor cell death.

The methods of the present invention may also improve the prognosis of the subject being treated. For example, the methods of the invention may provide to the subject a reduction in probability of a clinical worsening event during the treatment period.

In embodiments, the siRNA molecule or RNAi oligonucleotide of the invention is administered to a subject as a weight-based dose or as a fixed dose. A "weight-based dose" is a dose of the siRNA molecule or RNAi oligonucleotide that will change depending on the subject's weight (e.g., a dose in mg/kg). A "fixed dose" consists of one dose of a siRNA molecule or RNAi oligonucleotide (e.g., a dose in mg) which is used for all subjects regardless of any specific subject-related factors, such as weight. In one particular embodiment, a fixed dose of a siRNA molecule or RNAi oligonucleotide of the invention is based on a predetermined weight or age.

Subjects can be administered a therapeutic amount of siRNA molecule or RNAi oligonucleotide, such as about 0.01 mg/kg to about 50 mg/kg dsRNA. Values and ranges intermediate to the recited values are also intended to be part of this invention.

In some embodiments, the siRNA molecule or RNAi oligonucleotide is administered as a fixed dose of between about 0.01 mg to about 1 mg. In certain embodiments, the subject is administered a fixed dose of about 0.001 mg to about 1 mg of the siRNA molecule or RNAi oligonucleotide. In certain embodiments, the subject is administered a fixed dose of about 0.001 mg to about 0.5 mg of the siRNA molecule or RNAi oligonucleotide. In certain embodiments, the compound is delivered about once per month. In certain embodiments, the compound is administered once per quarter (i.e., about once every three months). In certain embodiments, the compound is administered semi-annually (i.e., about once every six months). In certain embodiments, the compound is administered daily (i.e., once per day).

In certain embodiments, the siRNA molecule or RNAi oligonucleotide is administered to a subject as a fixed dose of about 0.001, 0.003, 0.01, 0.02, 0.03, 0.04, 0.05, 0.06, 0.07, 0.08, 0.09, 0.1, 0.1, 0.2, 0.3, 0.4, 0.5, 0.6, 0.7, 0.8, 0.9, or about 1 mg once every day, once every month, once every two months, once every three months (i.e., once a quarter), once every four months, once every five months, once every six month (i.e., bi-annually), or once a year.

In some embodiments, the siRNA molecule or RNAi oligonucleotide is administered in two or more doses. In one embodiment, a subject is administered an initial dose and one or more maintenance doses of an siRNA molecule or RNAi oligonucleotide. The maintenance dose or doses can be the same or lower than the initial dose, e.g., one-half of the initial dose. Further, the treatment regimen may last for a period of time which will vary depending upon the nature of the particular disease, its severity and the overall condition of the patient. Following treatment, the patient can be monitored for changes in his/her condition. The dosage of the siRNA molecule or RNAi oligonucleotide may either be increased in the event the patient does not respond significantly to current dosage levels, or the dose may be decreased if an alleviation of the symptoms of the disease state is observed, if the disease state has been ablated, or if undesired side-effects are observed.

In certain other aspects, the invention provides kits for performing any of the methods of the invention. Such kits include a suitable container containing one or more siRNA molecules or RNAi oligonucleotides or a pharmaceutical formulation of these siRNA molecules or RNAi oligonucleotides. The kits may also include instructions for use, e.g., instructions for inhibiting expression of a TSC gene (TSC2 and/or TSC1) in an ocular cell by contacting the ocular cell with the siRNA compound(s) in an amount effective to inhibit expression of the TSC2 and/or TSC1 gene in the ocular cell. The kits may optionally further comprise means for contacting the ocular cell with the RNAi agent (e.g., an injection device, a dispenser or an eyedropper). In certain embodiments the individual components of the pharmaceutical formulation may be provided in one container. Alternatively, it may be desirable to provide the components of the pharmaceutical formulation separately in two or more containers, e.g., one container for an RNAi oligonucleotide or an siRNA molecule preparation, and at least another for a carrier compound. The kit may be packaged in a number of different configurations such as one or more containers in a single box. The different components can be combined, e.g., according to instructions provided with the kit. The components can be combined according to a method described herein, e.g., to prepare and administer a pharmaceutical composition. The kits of the invention may optionally further comprise means for administering the siRNA compounds to a subject or means for determining the therapeutically effective or prophylactically effective amount. The kits can also include a delivery device.

The siRNA molecule or RNAi oligonucleotide may be provided in any convenient form, such as a solution in sterile water for injection, or a solution in PBS for topical delivery to the eye.

The invention is further illustrated by the following examples, which should not be construed as further limiting. The contents of all references, pending patent applications and published patents cited throughout this application are hereby expressly incorporated by reference.

### Examples

### Obtaining siRNA molecules/duplexes

RNAs are preferably chemically synthesized using appropriately protected ribonucleoside phosphoramidites and a conventional DNA/RNA synthesizer. Substitution of one or both strands of a siRNA duplex by 2'-deoxy or 2'-O-methyl oligoribonucleotides abolished silencing in fly extract (Elbashir *et al.* 2001). In mammalian cells, however, it seems possible to substitute the sense siRNA by a 2'-O-methyl oligoribonucleotide (Ge *et al.* 2003).

Most conveniently, siRNAs were obtained from commercial RNA oligo synthesis suppliers, which sell RNA-synthesis products of different quality; for example, intended for target validation. In general, 19, 20 and 21-nt RNAs are not too difficult to synthesize and are readily provided in a quality suitable for RNAi. A number of commercial custom RNA synthesis companies exist; in the present examples, siRNA may be obtained from Biospring (Germany), unless otherwise specified.

### In vitro and animal studies.

In order to evaluate the therapeutic potential of the siRNAs identified against the TSC2 gene or TSC1 gene, or any of their combinations with any other siRNA against a different target gene, both *in vitro* and *in vivo* experiments in functional models that express the targets of interest and reproduce the mechanism of the retinal disease are carried out. For *in vitro* and *in vivo* experiments, siRNAs targeting TSC2 and/or TSC1 are preferably delivered formulated alone or in combination in a mixture of siRNAs, or as a combined treatment by delivering first one siRNA and separately a second siRNA, or as an RNAi product comprising two different siRNA moieties joined through a linker or spacer, or as a part of a bioconjugate and/or delivered formulated in nanoparticles (NPs) directed to the retinal tissues and cells of the eye.

### 1. In vitro experiments

### 1.1. In vitro validation of the efficacy for the different siRNA candidates of the invention

Different cell line models (human, mouse and rat) were previously selected based on their basal TSC1 and TSC2 gene expression levels and based on their cellular and tissue origin (epithelial and retinal), as well as on their suitability as in-vitro models for transfection studies. The selected cell lines expressed sufficient amount of the target genes to allow detection of silencing effects mediated by the candidates. Finally, ARPE-19 cells (human Retinal pigmented epithelium) [ATCC, # CRL-2302^{™}] and C2C12 (muscle mouse cells) [C2C12, # CRL-1772^{™}] were selected as in vitro cell models. Cell cultures were transfected with 100 nM siRNA solutions of each compound, 24 hours after transfection cell medium was changed and samples collected and analyzed for gene expression. Sample collection was performed immediately after medium change and 48 and 72 hours after transfection of siRNAs. ARPE-19 cells were successfully transfected with Oligofectamine, and C2C12 cells with Mirus Transit-X2 according to the standard procedure of the supplier. Total RNA was collected and extracted for all experimental conditions at different time points (24, 48, and 72 hours). After transfection gene expression levels of TSC1 and TSC2 were determined by QPCR. In parallel, cell viability MTS studies were carried out following the standard protocol of the supplier (Promega) to monitor the toxicity profile of each compound or combination based on a colorimetic method using solutions of the tetrazolium compound MTS [3-(4,5-dimethylthiazol-2-yl)-5-(3-carboxymethoxyphenyl)-2-(4-sulfophenyl)-2H-tetrazolium, inner salt].

In total, we have tested 40 candidate siRNAs targeting 16 different regions of the TSC1 messenger (24 candidates) and 9 regions of the TSC2 messenger (16 candidates), which are identified by their corresponding SEQ ID target sequence and siRNA ID (see Table 2 below). We also transfected a non-active siRNA.

**Table 2. Examples of TSC1 and TSC2 siRNA candidates used for in vitro validation studies, which are targeted against any of the nucleotide target sequences SEQ ID NO. 1 to SEQ ID NO. 17 of the TSC1 gene or the nucleotide target sequences SEQ ID NO. 41 to SEQ ID NO. 49 of the TSC2 gene.**

| **TSC1 Target sequence** | **Candidate siRNA** | **TSC2 Target sequence** | **Candidate siRNA** |
|---|---|---|---|
| SEQ ID NO. 6 | siRNA ID 11060 | SEQ ID NO. 43 | siRNA ID 01002 |
| SEQ ID NO. 4 | siRNA ID 11007 | SEQ ID NO. 47 | siRNA ID 01001 |
| SEQ ID NO. 6 | siRNA ID 11021 | SEQ ID NO. 42 | siRNA ID 01019 |
| SEQ ID NO. 10 | siRNA ID 10004 | SEQ ID NO. 48 | siRNA ID 01054 |
| SEQ ID NO. 1 | siRNA ID 11009 | SEQ ID NO. 48 | siRNA ID 01055 |
| SEQ ID NO. 15 | siRNA ID 11077 | SEQ ID NO. 48 | siRNA ID 01056 |
| SEQ ID NO. 14 | siRNA ID 11001 | SEQ ID NO. 48 | siRNA ID 01058 |
| SEQ ID NO. 17 | siRNA ID 11178 | SEQ ID NO. 46 | siRNA ID 01020 |
| SEQ ID NO. 9 | siRNA ID 11217 | SEQ ID NO. 41 | siRNA ID 01009 |
| SEQ ID NO. 2 | siRNA ID 11129 | SEQ ID NO. 48 | siRNA ID 01053 |
| SEQ ID NO. 12 | siRNA ID 11005 | SEQ ID NO. 42 | siRNA ID 01021 |
| SEQ ID NO. 3 | siRNA ID 11280 | SEQ ID NO. 42 | siRNA ID 01025 |
| SEQ ID NO. 13 | siRNA ID 11038 | SEQ ID NO. 42 | siRNA ID 01039 |
| SEQ ID NO. 16 | siRNA ID 11219 | SEQ ID NO. 49 | siRNA ID 00006 |
| SEQ ID NO. 4 | siRNA ID 11012 | SEQ ID NO. 44 | siRNA ID 00001 |
| SEQ ID NO. 4 | siRNA ID 11013 | SEQ ID NO. 45 | siRNA ID 00003 |
| SEQ ID NO. 5 | siRNA ID 11004 | | |
| SEQ ID NO. 6 | siRNA ID 11046 | | |
| SEQ ID NO. 6 | siRNA ID 11047 | | |
| SEQ ID NO. 6 | siRNA ID 11048 | | |
| SEQ ID NO. 6 | siRNA ID 11049 | | |
| SEQ ID NO. 6 | siRNA ID 11050 | | |
| SEQ ID NO. 7 | siRNA ID 11302 | | |
| SEQ ID NO. 8 | siRNA ID 10001 | | |

### Results from siRNAs targeting TSC1 in human cells

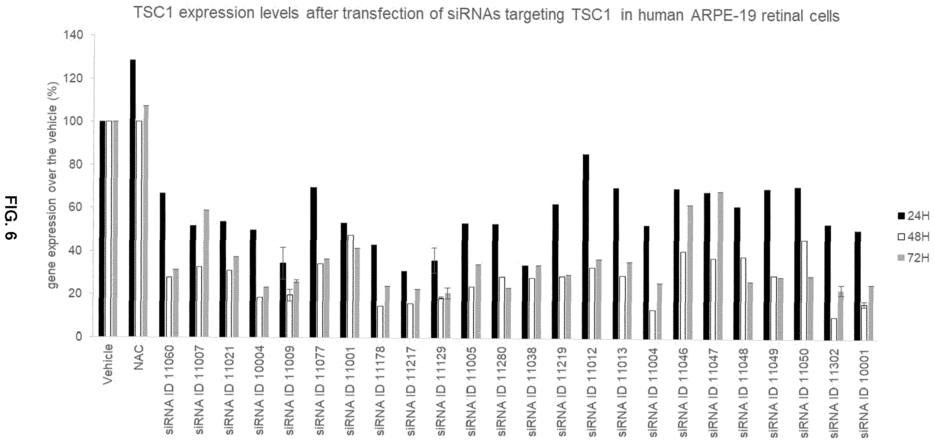

As shown in FIG. 6, all siRNAs directed against TSC1 produced a very potent reduction of mRNA levels in ARPE-19 retinal cells (≥70%), which was sustained overtime, with mRNA levels not recovering 72 hours after transfection. This reduction occurred very markedly from 48 hours after transfection for all candidates, but was particularly strong for siRNA ID 10004, siRNA ID 11009, siRNA ID 11129, siRNA ID 11280 (by 80%) and siRNA ID 11178, siRNA ID 11217, and siRNA ID 11302, siRNA ID 11004 and siRNA ID 10001 (by 90%). The non-active compound (NAC), however, did not produce any reduction in TSC1 gene expression levels. This marked reduction did not translate into changes in MTS studies at any of the times studied (data not shown).

### Results from siRNAs targeting TSC1 in mouse cells

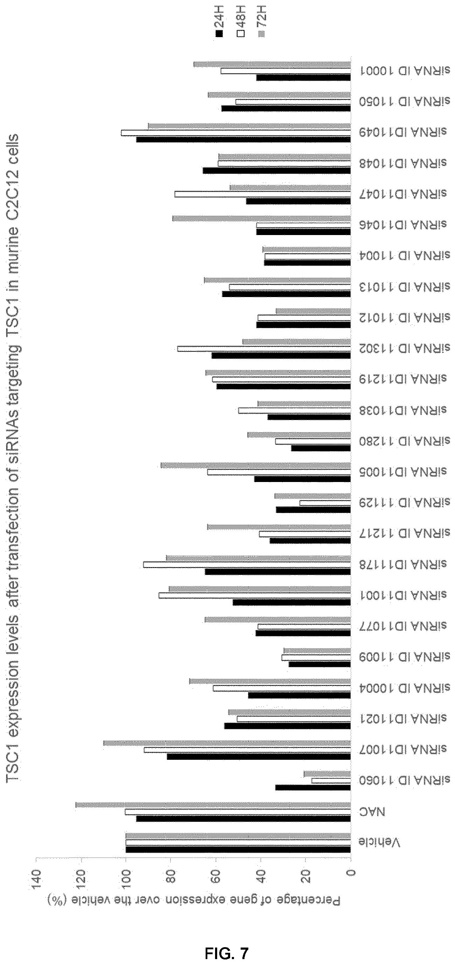

When the interference efficiency was analyzed in C2C12 mouse cells transfected with the candidates targeting TSC1, we observed that most of the candidates were effective in reducing TSC1 messenger levels, however, the reductions are somewhat lesser compared with human cells (60-80%) (Figure 7).

The candidates that reduced TSC1 mRNA 48 hours after transfection more efficiently were siRNA ID 11077 siRNA ID 11217, siRNA ID 11012, siRNA ID 11004 and siRNA ID 11046 (by 60%) and siRNA ID 11009 and siRNA ID 11280 (by 70%). Besides, siRNA ID 11129 and siRNA ID 11060 candidates on their own side were able to reduce TSC1 mRNA levels by 80% in any of the studied times considered in the study. None of these candidates fully recovered mRNA basal levels at any timepoint considered in the study.

On the other hand, siRNA ID 10001, siRNA ID 10004, siRNA ID 11077, siRNA ID 11012, siRNA ID 11005, siRNA ID 11004, siRNA ID 11047, and siRNA ID 10001 obtained a maximum reduction of 60% in some of the times studied. For all of them there is a gradual recovery of basal levels, although not completely 72 hours after transfection, except for siRNA ID 11004 and siRNA ID 11012 in which the decrease is maintained.

It is important to note how siRNAs that are effective in human cells lose their efficacy in mouse cells. This is the case for candidates such as siRNA ID11007, siRNA ID 11178, siRNA ID 11001 and siRNA 11049. This marked reduction did not translate into changes in MTS studies at any of the times studied (data not shown).

### Results from siRNAs targeting TSC2 in human cells

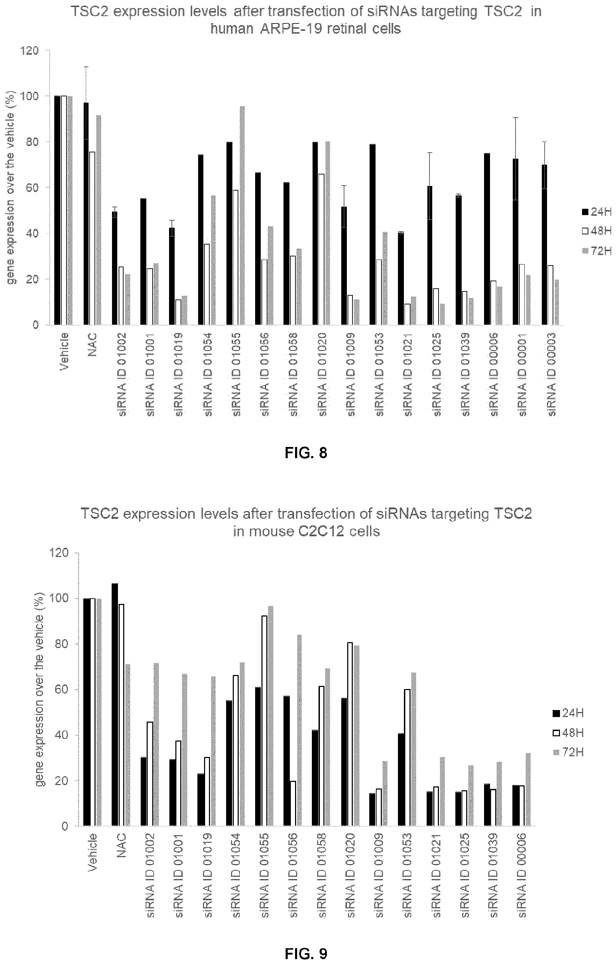

As shown in Figure 8, most of the siRNAs targeted against TSC2 were efficient in reducing TSC2 mRNA levels in human cells achieving maximum reductions between 80-90%, except for the candidates siRNA ID 01055, siRNA ID 01054 and siRNA ID 01020 in which the maximum decreases were lower (30-50%). Those candidates for which higher efficacies were described were siRNA ID 01019, siRNA ID 01009, siRNA ID 01021, siRNA ID 01025, siRNA ID 01039 with drops ≥ 90%, 72 hours after transfection, in second position we found siRNA ID 01002, siRNA ID 01001, siRNA ID 01056, siRNA ID 01058, siRNA ID 00006, siRNA ID 01001 and siRNA ID 1003 with maximum drops of 80%, and finally siRNA ID 01053 with a drop of 70%. For all candidates with efficacies >70% there was no recovery to baseline levels at any of the time points considered in the study. NAC showed no drop in TSC2 levels.

### Results from siRNAs targeting TSC2 in mouse cells

Similar behaviour was found for the different candidates when we studied their efficacy in mouse cells (Figure 9). Most of the candidates achieved efficiencies >70% at some of the experimental times considered, however, the temporal expression profile is slightly different from that shown in human cells, with the maximum drops appearing 24-48 hours after transfection and undergoing a temporal recovery of expression levels 72 hours after transfection that is not complete in most of the candidates. The most efficient candidates in mouse cells were siRNA ID 01009, siRNA ID 01021, siRNA ID01025, siRNA ID01039 and siRNA ID 00006 with a maximum drop of 80% obtained 24 hours after transfection, which was maintained 48 hours after transfection and recovered slightly 72 hours after transfection (70%). On the other hand, siRNAs siRNA ID 01002, siRNA ID 01001 and siRNA ID 01019, reduced TSC2 levels by 70% 24 hours after transfection and gradually recovered basal levels, although not completely. siRNA ID 01056, achieved its maximum decrease 48 hours after transfection (by 80%) but quickly recovered basal levels 72 hours after transfection. Again, siRNA ID 01055, siRNA ID 01020 and siRNA ID 01054 were the least effective. While siRNA ID 01055 and siRNA ID 01020 showed lower maximum drops (by 40%) and a gradual recovery, siRNA ID 01054 steadily reduced TSC2 expression levels by 40% over time. These reductions did not translate into changes in the MTS studies at any of the times considered (data not shown).

### 1.2. In vitro validation of the efficacy with the different siRNA candidates of the invention used in combination

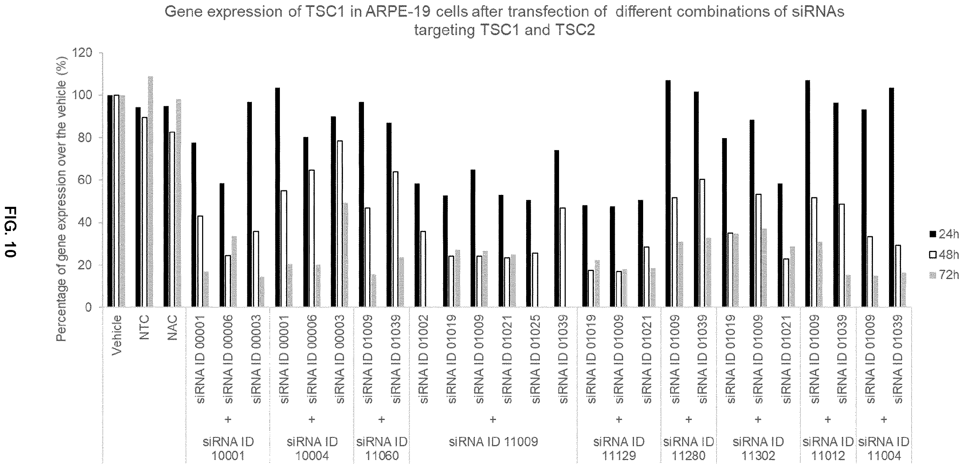

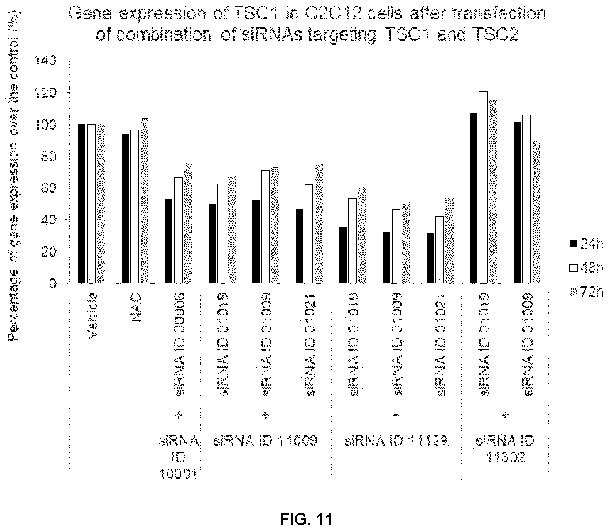

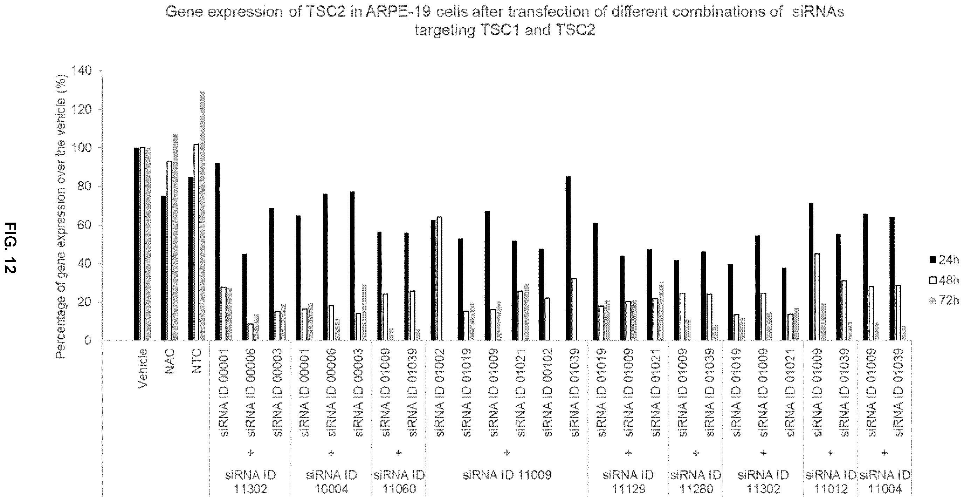

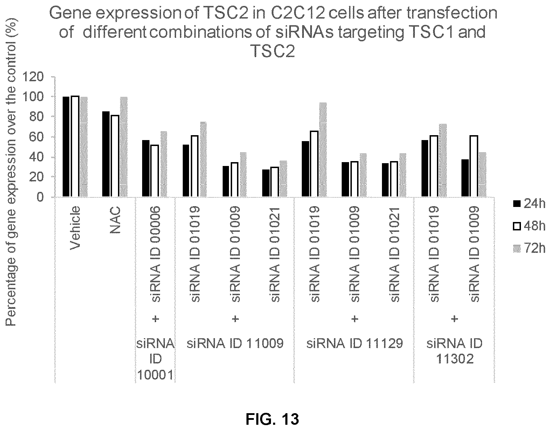

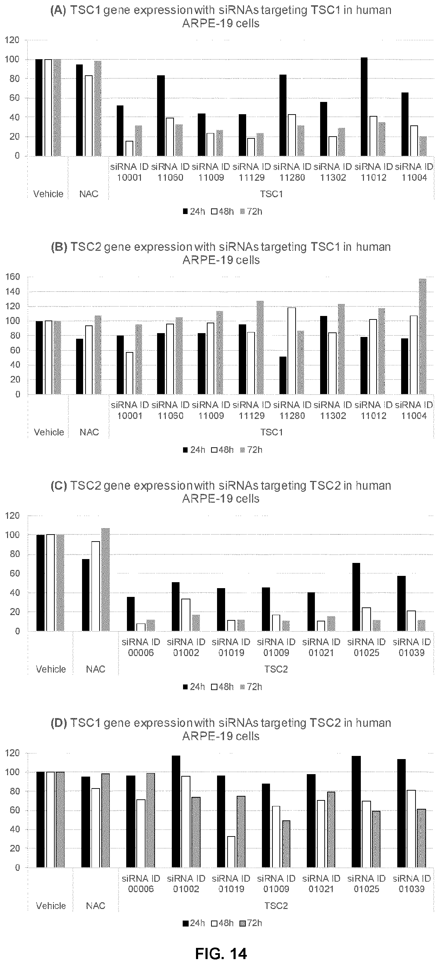

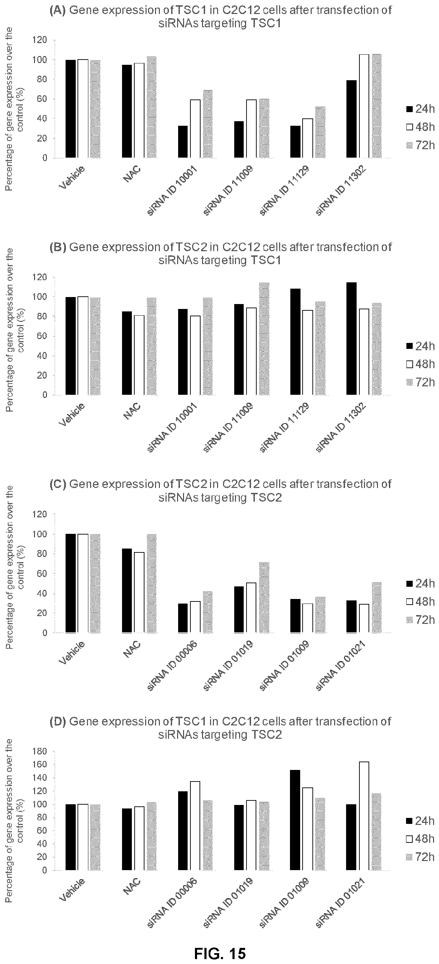

For some of the individually validated candidates, co-transfection was performed with the aim of evaluating a possible combination therapy targeting both targets. These co-transfection studies were performed in human retinal ARPE-19 cells (Figures 10 and 12) and in C2C12 mouse cells (Figures 11 and 13) following a similar procedure as described above. In the co-transfection studies, each candidate siRNA of the tested combinations were transfected, in an equimolar ratio and at a final concentration of 100 nM for each siRNA and the same transfection agents were used as in previous studies (Oligofectamine in human ARPE-19 cells and Transit-X-2 in C2C12 mouse cells). For all candidates specifically targeting each gene of interest, the expression profile of TSC1 and TSC2 was studied 24, 48 and 72 hours after transfection with the different siRNA combinations (Figures 10- 13), or the siRNA individually transfected (Figures 14 and 15) following the same protocol and at the same final concentration of 100 nM of the tested siRNAs-.

### Results on the TSC1 expression levels of different siRNA combinations tested in human cells

When we studied TSC1 levels in ARPE-19 cells with combinations of siRNA ID 10001 (TSC1) with the different siRNAs targeted for TSC2 (siRNA ID 00001, siRNA ID 00006 and siRNA ID 00003), we observed that due to competition with the siRNAs targeted against TSC2 the interference profile of siRNA ID 10001 changes markedly relative to when it was transfected alone in the cell (Figure 6). While when transfected alone it has a maximal efficacy of 80% 48 hours after transfection, when co-transfected its maximal efficacy occurs 72 hours after transfection and is 70-80%. This interference profile is consistently reproduced for the three combinations with TSC2 siRNAs (siRNA ID 00001, siRNA ID 00006 and siRNA ID 00003). The combination siRNA ID 10001 with siRNA ID 00001 or siRNA ID 00003 were perhaps the most efficient (Figure 10).

On the other hand, the combination of siRNA ID 11060 (TSC1) with the candidates directed against TSC2 (siRNA ID 01009 and siRNA ID 01039) in ARPE-19 cells shows that efficacy of siRNA ID 11060 is especially diminished 24 and 48 hours after transfection, by 30-40% when combined with any of these TSC2 siRNA candidates. 72 hours after transfection, however, the efficacy is still maintained.

In the same direction, siRNA ID 10004 (TSC1) when transfected into ARPE-19 cells combined with siRNA ID 00001, siRNA ID 00006, maintained its maximal efficiency (80%) 72 hours after transfection, but completely lost its efficiency at 24 hours post-transfection, and was markedly reduced 48 hours after transfection (50%). The combination of siRNA ID 10004 (TSC1) with siRNA ID 00003 caused siRNA ID 10004 (TSC1) to lose efficacy completely.

When we also studied the combination of siRNA ID 11009 (TSC1) with the TSC2 candidates (siRNA ID 01002, siRNA ID 01019, siRNA ID 01009, siRNA ID 01021, siRNA ID 01025 and siRNA ID 01039), we saw that it slightly lost efficacy 24 hours after transfection from 60% (when transfected alone) to 40% (when combined) in efficacy, but efficacy is maintained (by 80%) 48 and 72 hours after transfection, except for the combination with siRNA ID 01002 in which it also slightly loses efficacy (from 80% to 70% reducing TSC1 levels) and with siRNA ID 01039 (from 80% to 50%).

When siRNA ID 11280 (TSC1) was combined with siRNA ID 01009 and siRNA ID 01039 (TSC2), siRNA ID 11280 lost its efficacy significantly between 20-40% mainly 24 hours after transfection. When we studied the candidate siRNA ID 11129 (TSC1) in combination with the candidates for TSC2 (siRNA ID 01019, siRNA ID 01009 or siRNA ID 01021) in ARPE-19 cells we observed that they behave in the same way as when they are transfected individually, with 80% efficiency 48 and 72 hours after transfection, except at 24 hours after transfection when it goes from 60% efficiency to 50% (losing 10% efficiency, when competing with another siRNA).

Additionally, when we transfected siRNA ID 11302 (TSC1) in combination with the TSC2 candidates (siRNA ID 01019, siRNA ID 01009, siRNA ID 01021), we observed that siRNA ID 11302 loses much of its efficacy 24 hours post-transfection, when it is in combination with the different TSC2 candidates. It is interesting to note however that when combined with siRNA ID 01019, it also loses efficacy 72 hours post-transfection (achieving a maximum of 60% efficacy), as well as when combined with siRNA ID 01009, where it loses efficacy from 48 hours (going from 60% to 40% efficacy) and at 72 hours (going from 70% to 60% of silencing effect). However, it is also interesting to note that when siRNA ID 11302 is combined with siRNA ID 01021 it maintains its behaviour 24 hours after transfection and increases its efficiency 48 hours after transfection.

When siRNA ID 11012 (TSC1) was combined with siRNA ID 01009 or siRNA ID 01039 (TSC2), siRNA ID 11012 lost its efficacy significantly at all times considered in the study between 50-60% efficacy, 24 and 48 hours after transfection, while 72 hours after transfection it was slightly reduced (by 10%) when combined with siRNA ID 01009 while with siRNA ID 01039 it increased slightly (by 10%).

Finally, when siRNA ID 11004 was combined with siRNA ID 01009 or siRNA ID 01039, the efficiency was reduced 24 hours after transfection completely, is maintained 48 hours after transfection, and surprisingly 72 hours after transfection was slightly increased (up to 10%).

These data suggest the competition with the combined siRNA and the thermodynamic profile of each candidate included in the combination determines the entry into RISC for each of the candidates, and their spatiotemporal behaviour in terms of efficacy. The competition of the different siRNAs for entry into the RISC machinery may be determinant for the performance of both compounds when combined. This could be altered or fine-tuned by the pattern of modifications chosen for each of the individual candidates.

Besides, from studies as described above, different siRNA combinations having a better performance in reducing the expression of TSC1 in human cells can be identified. Thus, the above results taken jointly showed that different siRNAs targeting SEQ ID NO. 1 or SEQ ID NO. 2 of the TSC1 gene can be combined with siRNA targeting SEQ ID NO. 41 or SEQ ID NO. 42 of the TSC2 gene without affecting considerably the silencing effect observed when the TSC1 siRNA is used alone.

In particular, siRNA combinations consisting of siRNA ID 11009 (TSC1) + siRNA ID 01009 (TSC2), siRNA ID 11009 (TSC1) + siRNA ID 01021 (TSC2), or siRNA ID 11009 (TSC1) + siRNA ID 01025 (TSC2) showed a better efficiency.

### Results on the TSC1 expression levels of different siRNA combinations tested in mouse cells

Once the combined efficacy was studied in human cells, we proceeded similarly to study some of the combinations in C2C12 mouse cells. As in ARPE-19 cells, we co-transfected different combinations of each candidate siRNA at a final concentration of 100 nM in admixture and with the same transfection agents used in previous studies.

When siRNA ID 10001 (TSC1) and siRNA ID 00006 (TSC2) were combined, siRNA ID 10001 maintained the efficacy shown individually. On the other hand, siRNA ID 11009 (TSC1) when combined with any of siRNA ID 01019, siRNA ID 01009 and siRNA ID 01021 loses efficacy from 60% to 40%. SiRNA ID 11129 (TSC1) however, when combined with siRNA ID 01019, siRNA ID 01009 or siRNA ID 01021 (TSC2), not only does not lose its efficacy, but increases slightly at all three times studied. This improvement in efficacy is more evident for the combination of siRNA ID 11129 (TSC1) with siRNA ID 01009, or siRNA ID 01021 (TSC2), going from 60% efficacy (transfected individually) to 70% (in combination) 24 hours after transfection. Finally, siRNA ID 11302 (TSC1) completely lost efficacy when transfected in combination with siRNA ID 01019 (TSC2) or siRNA ID 01009 (TSC2).

Jointly, these data in mouse cells showed that siRNAs targeting SEQ ID NO. 2 of the TSC1 gene can be combined with different siRNAs targeting SEQ ID NO. 41 or SEQ ID NO. 42 of the TSC2 gene with hardly any loss of silencing efficacy in comparison to that of the TSC1 siRNA alone. In particular, siRNA combinations consisting of siRNA ID 11129 (TSC1) + siRNA ID 01009 (TSC2) showed better results in murine cells.

### Results on the TSC2 expression levels of different siRNA combinations tested in human and mouse cells

In addition to studying the impact of co-transfection on TSC1 expression levels, we studied the impact of different combinations of siRNAs targeting TSC1 and siRNAs targeting TSC2 on the TSC2 expression levels in both ARPE-19 human retinal cells (Figure 12) and C2C12 mouse cells (Figure 13). Such studies allow to identify different appropriate siRNA combinations having a better performance in reducing the expression of TSC2 in cells of one or more species of interest, which can be useful for therapeutic purposes.

*Human cells.-* As can be seen in Figure 12, when we combined siRNA ID 11302 (TSC1) with any of the TSC2 siRNA compounds siRNA ID 00001, siRNA ID 00006 or siRNA ID 00003 (TSC2) in human ARPE-19 cells, siRNA ID 00001 behaved similarly to when this was singly transfected, whereas siRNA ID 00006 improved its efficiency 24 hours after transfection (from 20% to 60% efficiency) and also 48 and 72 hours after transfection, achieving a maximum efficiency of 90% compared to the 80% it was able to obtain individually. Something similar occurred for siRNA ID 00003, which slightly improved its efficiency (5-10%) 24 hours post-transfection.

When we combined siRNA ID 10004 (TSC1) with any of siRNA ID 00001, siRNA ID 00006 or siRNA ID 00003 (TSC2), we observed that all compounds maintained their efficacy profile (maximum efficacy values of 80%, 48- and 72-hours post-transfection), except for siRNA ID 00003 which lost 10% efficacy 72 hours post-transfection by reducing TSC2 levels, when combined with siRNA ID 10004.

When siRNA ID 11060 (TSC1) is combined in ARPE-19 cells with siRNA ID 01009 or siRNA ID 01039 (TSC2), these two TSC2 siRNAs improve their efficacy achieving a maximum reduction of 95% 72 hours post-transfection.

When we combine, however, siRNA ID 11009 (TSC1) with siRNA ID 01002, siRNA ID 01019, siRNA ID 01009, siRNA ID 01021, siRNA ID 01025 or siRNA ID 01039 (TSC2), we can identify changes in efficacy due to competition for the RISC machinery. While siRNA ID 01002, siRNA ID 01039, siRNA ID 01021 lost efficacy between 20% and 30% 48 hours post-transfection, siRNA ID 01009 and siRNA ID 01019 still maintained the same profile with maximum efficacies ≥ 80%. In the same direction, when we combined siRNA ID 11129 (TSC1) with siRNA ID 01009, siRNA ID 01019, or SIRNA ID 01021 (TSC2), similar efficacy profiles were maintained with maximal efficacy values 48- and 72-hours post-transfection ≥ 80%.

Also for combinations of siRNA ID 11280 (TSC1) with siRNA ID 01009 or siRNA ID 01039 (TSC2), similar efficacy profiles were obtained to those obtained for each candidate individually with maximum efficacy values of 80%-90% 48- and 72-hours post-transfection.

The combination of siRNA ID 11302 (TSC1) with siRNA ID 01009, siRNA ID 01019 or siRNA ID 01021 of TSC2 did not produce any changes 24 hours post-transfection but slight reductions of around 5% were found 48- and 72-hours post-transfection.

Finally, combinations of siRNA ID 11012 (TSC1) with siRNA ID 01009 or siRNA ID 01039 of TSC2, did impact the efficacy of both compounds reducing it by decreasing 40% of mRNA expression levels, especially 48 hours after transfection, while 72 hours after transfection the efficacy for siRNA ID 01009 and siRNA ID 01039 was not affected (by 80%). The combination of siRNA ID 11004 (TSC1) with siRNA ID 01009 and siRNA ID 01039, slightly reduced the efficacy 48 hours after transfection (by 10%), while at 72 hours it was slightly higher (>90%) for both candidates.

In general, these data obtained in human cells suggest that the combination of siRNAs targeting SEQ ID. NO. 41 or SEQ ID NO. 42 of TSC2 with a siRNA targeting any of SEQ ID NO. 6, SEQ ID NO. 1, SEQ ID NO. 2, SEQ ID NO. 3 or SEQ ID NO. 5 of TSC1 gene would improve or maintain the silencing profile on the TSC2 siRNA. Besides, the combination of a TSC2 siRNA targeting SEQ ID NO. 44 or SEQ ID NO. 45 with a TSC1 siRNA targeting SEQ ID NO. 7 would have a similar behaviour, or even slightly better, in relation to the TSC2 siRNA alone for reducing the TSC2 mRNA expression levels.

In particular, some siRNA combinations showed some improvements for reducing TSC2 expression at any of the three times studied (24h, 48h or 72h). This was the case of siRNA combinations consisting of siRNA ID 00006 (TSC2) + siRNA ID 11302 (TSC1), siRNA ID 00003 (TSC2) + siRNA ID 11302 (TSC1), siRNA ID 01009 (TSC2) + siRNA ID 11060 (TSC1) and siRNA ID 01039 (TSC2) + siRNA ID 11060 (TSC1). In other cases, the combination resulted in a TSC2 silencing profile similar to the TSC2 siRNA alone, with hardly reductions in the efficacy levels (of about 5% or less), such as in case of the combination of siRNA ID 00001 (TSC2) with siRNA ID 11302 (TSC1) or siRNA ID 10004 (TSC1), the combination of siRNA ID 00006 (TSC2) with siRNA ID 10004 (TSC1), the combination of siRNA ID 01009 (TSC2) with any of siRNA ID 11009 (TSC1), siRNA ID 11129 (TSC1), siRNA ID 11280 (TSC1) or siRNA ID 11302 (TSC1), the combination of siRNA ID 01019 (TSC2) with any of siRNA ID 11009 (TSC1), siRNA ID 11129 (TSC1) or siRNA ID 11302 (TSC1), the combination of siRNA ID 01021 (TSC2) with siRNA ID 11129 (TSC1) or siRNA ID 11302 (TSC1), or the combination of siRNA ID 01039 (TSC2) and siRNA ID 11280 (TSC1).

Considering jointly the silencing effects over the TSC2 expression levels at the different time points studied, as well as the compatibility of the two combined siRNAs that induced less changes in its expression, the combination of siRNA ID 11129 targeting SEQ ID NO. 2 of TSC1 with siRNA ID 01009 targeting SEQ ID NO. 41 of TSC2 seems to have a better performance in human ARPE-19 cells.

*Mouse cells.-* As for TSC1, we evaluated the impact of some of the combinations on the RNA knockdown efficiency of TSC2 in mouse cells. Forthis purpose, we performed co-transfections similarly at the reference concentration 100 nM in C2C12 cells and with the same transfection agent (Transit-X-2).

As shown in Figure 13, the combination of siRNA ID 10001 (TSC1) and siRNA ID 00006 reduced by 40% the efficacy of siRNA ID 00006. The combination of siRNA ID 11009 (TSC1) with any of the siRNAs targeting TSC2 as siRNA ID 01009, siRNA ID 01019 and siRNA ID 01021, reduced the efficacy of the candidate TSC2 siRNAs by 10-20% at all times considered in the study. The combination of siRNA ID 11129 (TSC1) with siRNA ID 01009 or siRNA ID 01021 (TSC2) barely reduced the efficacy by 10-20%, obtaining maximum sustained values of 60% at the three time points considered. The combination of siRNA ID siRNA IDs 01019 (TSC2) with 11129 (TSC1) reduced the efficacy by 20-30% with respect to siRNA ID 01019, reaching reductions in TSC2 of only 20-40% compared to baseline levels. siRNA ID 11302 (TSC1) in combination with siRNA ID 01009 and siRNA ID 01019, reduced the interference efficacy on TSC2 by 20 and 40%, respectively.

In summary, data obtained in mouse cells suggest that the combination of siRNAs targeting SEQ ID. NO. 41 or SEQ ID NO. 42 of TSC2 with a siRNA targeting SEQ ID NO. 2 of TSC1 gene have a slightly reduced, but sustained during 72 hours, silencing profile of the TSC2 siRNA. In particular, the combination of siRNA ID 11129 targeting SEQ ID NO. 2 of TSC1 with siRNA ID 01009 targeting SEQ ID NO. 41 of TSC2 seems to have also a better performance in mouse C2C12 cells.

### Results on the TSC1 and TSC2 expression levels of different siRNA combinations tested in human and mice cells

For some of the candidates targeting TSC1 and TSC2 and selected for evaluation in the combinations, both TSC1 (Figure 14) and TSC2 (Figure 15) expression levels were studied in parallel. As shown in Figure 14A and 14C both siRNAs targeting TSC1 (≥ 70%) and TSC2 (> 90%) showed high levels of efficacy in ARPE-19 cells for any of the three time points considered. When we evaluated TSC2 expression levels for candidates targeting TSC1 gene (Figure 14B), we observed that some candidates also reduced the expression levels of TSC2 24 or 48 hours after transfection. This is the case for siRNA ID 10001 (40%) and siRNA ID11280 (60%). The rest of the candidates directed against TSC1 produced a very slight reduction (10-20%). This reduction was quickly recovered 48 to 72 hours after transfection. Similarly, we studied TSC1 expression levels for siRNAs targeting TSC2 and observed that all candidates were able to reduce TSC1 expression at some of the experimental times considered (Figure 14D). While siRNA ID 00006, siRNA ID 01002 and siRNA ID 01021, produced a maximum downregulation of 20% over TSC1 basal levels, siRNA ID 01019 reduced TSC1 levels (70%) 48 hours after transfection, siRNA ID 01009 reduced them by 50%, siRNA ID 01025 and siRNA ID 01039 by 40%. This type of change was slightly observed (20%) for siRNAs targeting TSC1 when TSC2 levels were studied in mouse cells (Figure 15B) and was not observed for candidates targeting TSC2 when TSC1 levels were studied (Figure 15C). The dual action of a given candidate on both targets would provide value in therapeutic terms as it would allow additional control over the genes of interest.

Similarly as seen in the in vitro validation experiments with just one siRNA transfected, no significant changes in the MTS studies were observed at any of the times studied for the tested combinations (data not shown).

### Conclusions

Conjointly, the above in vitro experiments and results allow to identify optimal therapeutic RNA interfering candidates targeting any of the TSC2 and TSC1 genes for further development, which can be used alone or alternatively in a combination with another siRNA to target both genes, based on the improved silencing profile of both genes TSC1 and TSC2 when used alone or in combination.

Some RNAi candidates targeting TSC1 showed a high silencing effect in reducing TSC1 expression levels (≥ 65-70%) in cells of at least two species, but also reduce to a certain extent the TSC2 expression levels (preferably ≥ 30%), the latter at least in human cells, preferably in cells of the eye or the retina. Such TSC1 siRNAs can be particularly useful for therapeutic purposes. The above experimental data showed that RNAi candidates which have a good performance in human and mouse cells target the sequence SEQ ID NO. 3 or SEQ ID NO. 8 of the TSC1 gene, which include for example the compounds siRNA ID 10001 and siRNA ID 11129.

Similarly, some RNAi candidates targeting TSC2 showed high silencing effect in reducing TSC2 expression levels (≥ 65-70%) in cells of at least two species, but also reduce to a certain extent the TSC1 expression levels (preferably ≥ 20%), the latter at least in human cells, preferably in cells of the retina or the eye. These TSC2 siRNAs can be particularly useful for therapeutic purposes when used alone, but also when used in combination with a TSC1 siRNA providing that such combination does not substantially reduce the silencing effect on the TSC2 expression of the TSC2 siRNA. The above experimental data showed that RNAi candidates which have a good performance in human and mouse cells target the sequence SEQ ID NO. 41 or SEQ ID NO. 42 of the TSC2 gene, which include for example the compounds siRNA ID 01019 and siRNA ID 01009 with a better performance in treating human cells.

RNAi candidates targeting sequences of TSC1 gene and candidates targeting sequences of TSC2 gene particularly useful for being used in combination can be those which have a high silencing effect (for example, ≥ 70%) on TSC1 and TSC2, respectively, and whose silencing effects remain hardly unaltered, or even improve, when used in combination. The experimental data showed that the combination of RNAi candidates targeting SEQ ID NO. 2 of TSC1 and any of sequences SEQ ID NO. 41 or SEQ ID NO. 42 of TSC2 had a better performance in reducing the expression levels of both genes in human and mouse cells, which include the combination of siRNA ID 11129 with siRNA ID 01009. However, other siRNA combinations were identified as having a useful silencing profile for both genes in human retinal cells, which could be considered as alternatives for further development or human treatments. For example, a good performance in ARPE-19 cells was obtained for example with some combinations of a TSC2 siRNA targeting SEQ ID NO. 41 or SEQ ID NO. 42 with a TSC1 siRNA targeting any of the sequences SEQ ID NO. 1, SEQ ID NO. 3, SEQ ID NO. 5 or SEQ ID NO. 6, and also with some combinations of a TSC2 siRNA targeting SEQ ID NO. 44 or SEQ ID NO. 45 with a TSC1 siRNA targeting SEQ ID NO. 7.

### 1.3. In vitro validation of the efficacy with the different chemically modified and asymmetric siRNA candidates of the invention used alone or in combination.

In order to improve the stability of siRNAs of the present invention and the silencing profile on the TSC1 and TSC2 expression when used alone or in combination, different siRNA-optimized chemical modifications were introduced to the canonical sequences of the designed siRNA candidates. In addition, other siRNA candidates were designed to shorten the length of their sense strands by removing between 1 to 5 nucleotides from the 3'- or 5'- region of the dsRNA motif. Some examples of asymmetric siRNA compounds and new chemically modified entities of TSC1 and TSC2 siRNAs which were obtained to be in vitro tested are shown in Table 2.

Following similar experimental procedures as described in the above sections 1.1 and 1.2, the in vitro efficacy of different asymmetric compounds and chemically modified variants of candidates, as well as their related combinations were evaluated.

### Efficacy of chemically modified siRNAs targeting TSC1 in human ARPE-19 cells

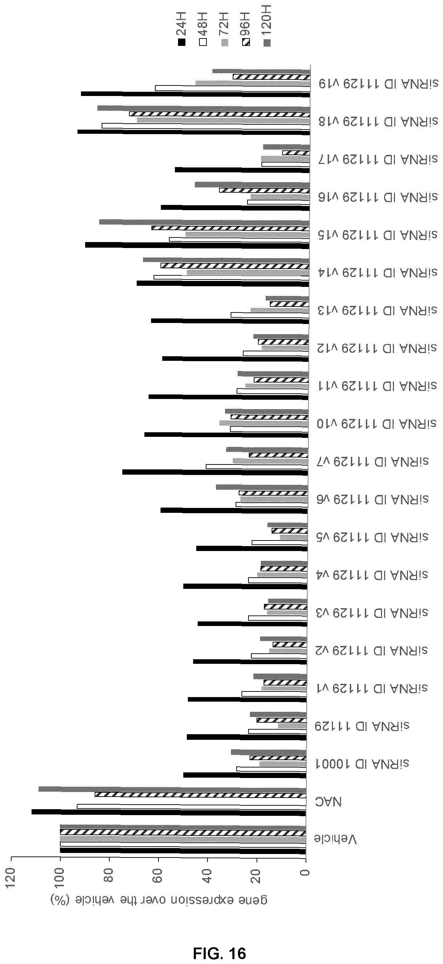

Human ARPE-19 retinal cells were transfected with a 100 nM solution of the different chemically modified variants targeting TSC1 and corresponding to the siRNA ID 11129 using Oligofectamine (Invitrogen) as transfection agent. All transfections were performed following standard manufacturer's instructions. In the same experiment a fluorescent non-active siRNA (BLOCK-iT^{™}, hereafter non-active compound or NAC) was considered to study the efficiency of the transfection and as non-active compound. Cells were collected and RNA extracted to evaluate TSC1 mRNA levels after the action of the different TSC1 siRNA candidates. RNA levels were quantified by real-time QPCR through the relative quantification 2^{-ΔΔCt} method. As Figure 16 shows most of the chemically modified siRNAs targeting TSC1 showed a significant reduction in TSC1 mRNA levels 48-72 hours after transfection and maintained overtime. Especially relevant are the siRNA candidates siRNA ID 11129v2, siRNA ID 11129v3, siRNA ID 11129v4, siRNA ID 11129v5, siRNA ID 11129v13, siRNA ID 11129v17 in which no recovery of basal levels was achieved 5 days after transfection. Some chemical modification patterns altered the efficacy of the naked compound as in the case of siRNA ID 11129v14, siRNA ID 11129v15 and siRNA ID 11129v18.

### Efficacy of chemically modified siRNAs targeting TSC1 in C2C12 cells

C2C12 mouse cells were transfected with a 100 nM solution of the different chemically modified variants targeting TSC2 and corresponding to the siRNA ID11129 using TranslT-X2 (Mirus) as transfection agent. All transfections were performed following standard manufacturer's instructions. In the same experiment a fluorescent non-active siRNA (BLOCK-iT^{™}, hereafter non-active compound or NAC) was considered to study the efficiency of the transfection and as non-active compound. Cells were collected and RNA extracted to evaluate TSC1 mRNA levels after the action of the different TSC2 siRNA candidates. RNA levels were quantified by real-time QPCR through the relative quantification 2^{-ΔΔCt} method.

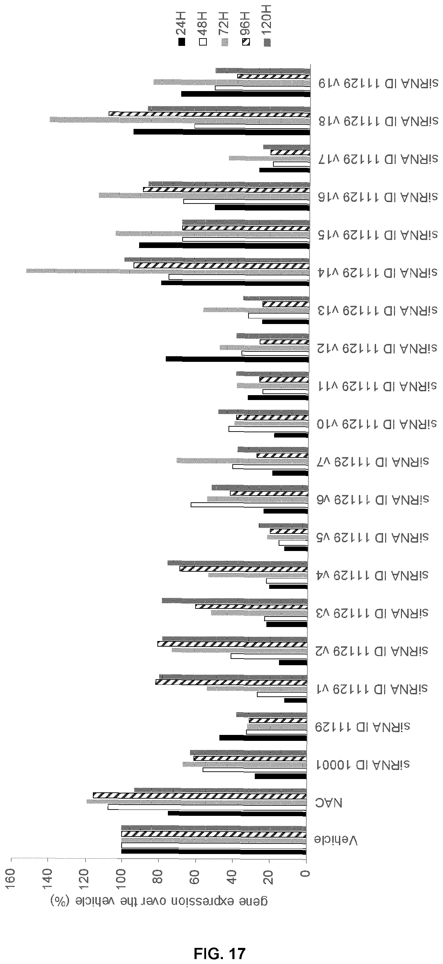

The majority of siRNAs showed the highest downregulation 24 h after transfection when compared to the vehicle, however some differences were found in term of silencing at longer time points (FIG. 17). Chemically modified siRNAs with ID 11129v1, 11129v2 and 11129v5 reduced TSC1 mRNA levels more than 85% 24 h after transfection. While siRNA compounds with ID 11129v1 and 11129v2 recovered basal levels 96 h after transfection, siRNAs with ID 11129v5 and 11129v17 reduced TSC1 mRNA levels by 75% and maintained their effect for longer times (96 and 120 h) when compared to the vehicle. Finally, siRNAs with ID 11129v14, 11129v15, 11129v16 and 11129v18 were clearly less efficient in targeting TSC1 mRNA (Fig. 17). The applied modification patterns affected the efficiency and power of the original naked sequence.

### Efficacy of chemically modified siRNAs targeting TSC2 in human ARPE-19 cells

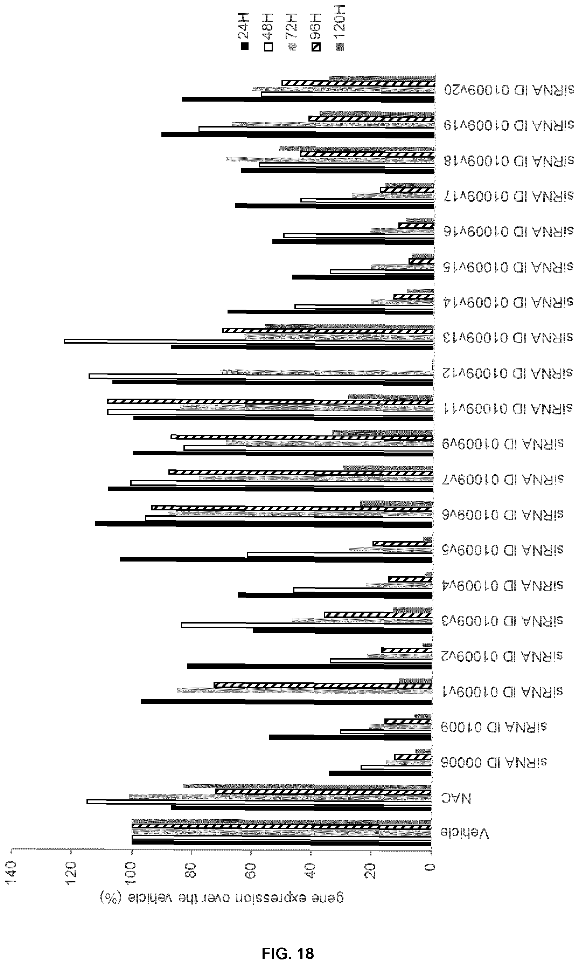

ARPE-19 human cells were transfected with a 100 nM solution of the different chemically modified variants targeting TSC2 and corresponding to the siRNA ID 01009 using Oligofectamine as transfection agent. All transfections were performed following standard manufacturer's instructions. In the same experiment a fluorescent non-active siRNA (BLOCK-iT^{™}, hereafter non-active compound or NAC) was considered to study the efficiency of the transfection and as non-active compound. Cells were collected and RNA extracted to evaluate TSC2 mRNA levels after the action of the different TSC2 siRNA candidates. RNA levels were quantified by real-time QPCR through the relative quantification 2^{-ΔΔCt} method. Most of siRNAs targeting TSC2 efficiently reduced the levels of TSC2 by 80-90% in some of the times considered in the study (Figure 18) It is important to note that different patterns of mRNA downregulation were found. Some siRNAs significantly reduced mRNA levels 24 h after transfection but showed the highest mRNA interference at longer time points (96-120 h). Other candidates showed a significant reduction of mRNA levels only 120 h after transfection (Fig. 18). siRNA compounds with siRNA ID 00006, 01009, 01009v2, 01009v4, 01009v5 and 01009v12 were the most efficient siRNAs producing more than 95% of TSC1 mRNA decay 120 h after-transfection (Fig. 18). siRNAs targeting TSC2 efficiently reduced TSC2 levels at some of the time point considered. In general terms, most of the modification patterns considered had their greatest effect in the longest times (120 h), obtaining reductions between 60-90%. Some candidates such as siRNAs with ID 01009v5, 01009v6, 01009v7, 01009v9, 01009v11 took 4 days to effectively reduce TSC2 expression levels. This could lead to the appearance of interference sustained over time and to be able to lengthen the administration and dosing pattern. None of the candidates recovered basal levels of expression at 120 hours after transfection.

### Efficacy of chemically modified siRNAs targeting TSC2 in C2C12 cells

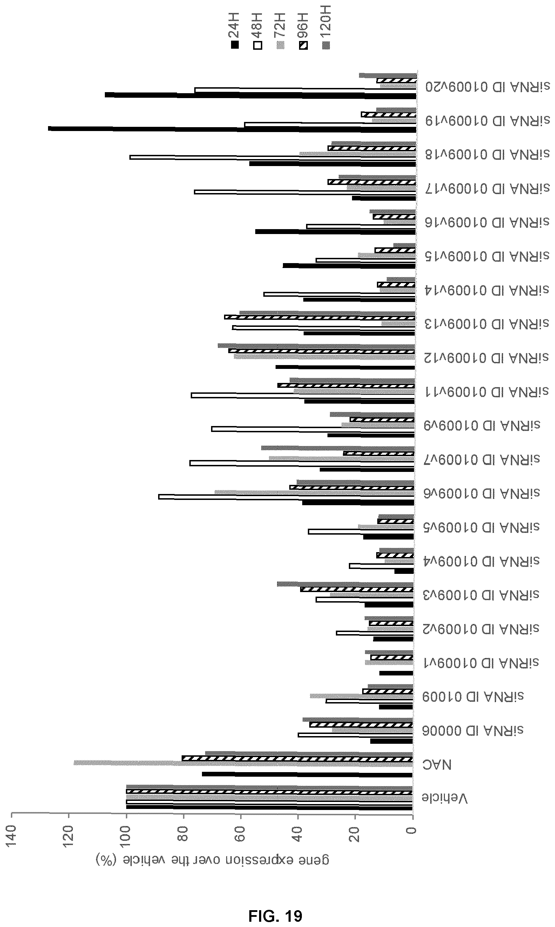

C2C12 mouse cells were transfected with a 100 nM solution of the different chemically modified variants targeting TSC2 and corresponding to the siRNA ID 01009 using TranslT-X2 (Mirus) as transfection agent. All transfections were performed following standard manufacturer's instructions. In the same experiment a fluorescent non-active siRNA (BLOCK-iT^{™}, hereafter non-active compound or NAC) was considered to study the efficiency of the transfection and as non-active compound. Cells were collected and RNA extracted to evaluate TSC2 mRNA levels after the action of the different TSC2 siRNA candidates. RNA levels were quantified by real-time QPCR through the relative quantification 2^{-ΔΔCt} method. The profile of these modified siRNAs targeting TSC2 showed different behaviors downregulating mRNA (Fig. 19). While siRNA compounds with siRNA ID 00006, 01009, 01009v1, 01009v2 and 01009v4 produced a very potent reduction 24 h post-transfection (by 85%) that are maintained during all the times considered in the study. siRNA compounds with ID 01009v5, 01009v14, 01009v15, 01009v16 and 01009v19 showed lower efficiencies in the shortest times but over time they achieved the same levels of efficacy. We could be facing patterns of modification that, in case of maintaining the sustained effect for longer times, would allow a longer therapeutic effect to be achieved.

### Efficacy of different siRNA combinations with selected modified siRNA targeting TSC1 and TSC2 tested in human and mice cells

With the aim of evaluating a possible combination therapy targeting both target genes, co-transfection studies with some of the individually validated modified candidates were performed in human ARPE-19 and murine C2C12 cells following a similar procedure as described above in section 1.2 of the Examples for siRNA combinations with unmodified siRNAs.

Each modified siRNA of the tested combinations were transfected, in an equimolar ratio and at a final concentration of 100 nM for each siRNA and the same transfection agents were used as in previous studies (Oligofectamine in human ARPE-19 cells and Transit-X-2 in C2C12 mouse cells). For all candidates specifically targeting each gene of interest, the expression profile of TSC1 and TSC2 was studied 48 and 72 hours after transfection with the different combinations of chemically modified siRNAs or the siRNA individually transfected following the same protocol and at the same final concentration of 100 nM of the tested siRNAs.

### a. Results on the TSC1 and TSC2 expression levels of different combinations of chemically modified siRNAs targeting each gene tested in human ARPE-19 cells

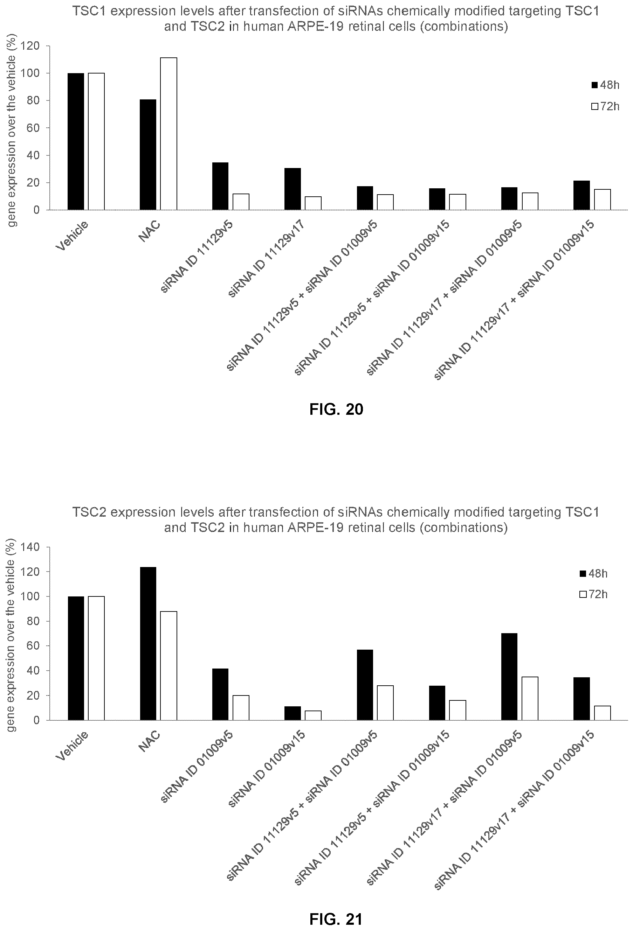

To test if the combined treatment has an impact on the efficacy of chemically modified compounds, human retinal cells ARPE-19 were transfected with different combinations of chemically modified siRNAs targeting both TSC1 (incl. siRNA ID 11129v5 and siRNA ID 11129v17) and TSC2 (incl. siRNA ID 01009v5 and siRNA ID 01009v15). For these experiments, cells were seeded as explained before, and Oligofectamine (Invitrogen) transfection reagent was used following the manufacturer's instructions. Final concentration of 100 nM for each siRNA in every combination was employed. As FIG. 20 shows, the combination of the tested modified siRNAs has no impact on the efficacy of each individual siRNA targeting TSC1. Indeed, an improvement in terms of mRNA downregulation was observed 48h after combined treatment with siRNA ID 11129v5 (see mRNA levels after treatment with combinations siRNA ID 11129v5 + siRNA ID 01009v5 and ID 11129v5 + siRNA ID 01009v15, FIG. 20) or siRNA ID 11129v17.

We also evaluated the impact of the combined treatment on the mRNA levels of TCS2 in ARPE-19 cells. As FIG. 21 shows, combination of siRNA ID 01009v5 targeting TSC2 with either siRNA ID 11129v5 or siRNA ID 11129v17 targeting TSC1 affected the efficacy of this siRNA alone since an increase in mRNA levels of TSC2 at 48h and 72h was observed. This observation was also extended to siRNA ID 01009v15, which produced higher silencing effect alone when compared to the combinations siRNA ID 11129v5 + siRNA ID 01009v15 and siRNA ID 11129v17 + siRNA ID 01009v15 (FIG. 21). However, all combinations produced a significant downregulation of TSC2 72h after transfection.

### b. Results on the TSC1 and TSC2 expression levels of different combinations of chemically modified siRNAs targeting each gene tested in mouse C2C12 cells

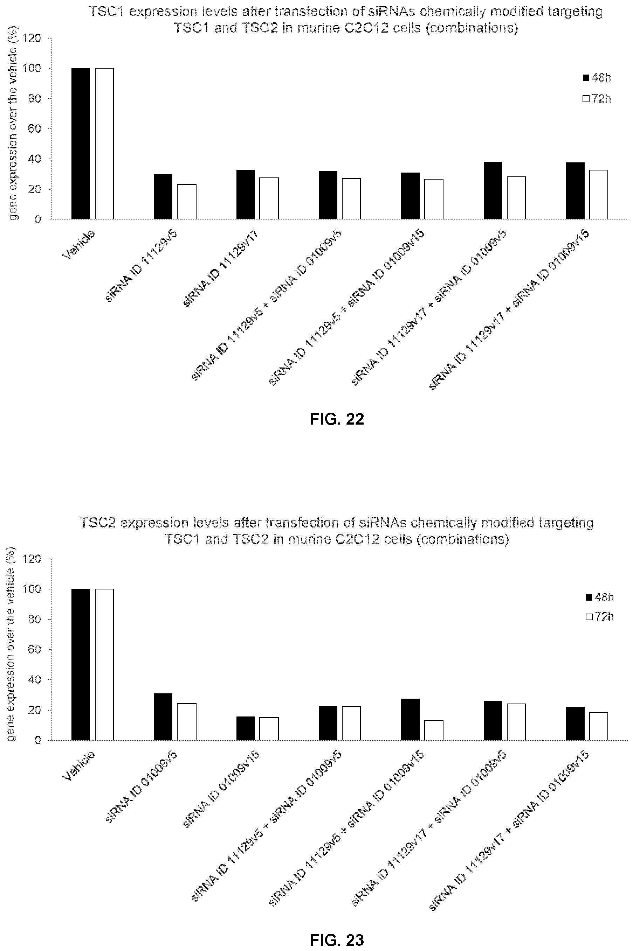

We next evaluated these combined treatments in murine C2C12 cells. siRNA treatment was performed similarly as previously described for human ARPE-19 cells, using TranslT-X2 (Mirus) as transfection reagent and 100 nM final concentration of each siRNA. No impact on the efficacy of siRNA ID 11129v5 or siRNA ID 11129v17 was observed when combined with either siRNA ID 01009v5 or siRNA ID 01009v15, meaning that these siRNAs can be used alone or in combination with other siRNAs targeting TSC2 (FIG. 22).

Combined treatment with siRNA ID 11129v5 or siRNA ID 11129v17 (TSC1) did not interfere with the silencing effect of siRNA ID 01009v5 or siRNA ID 01009v15 as shown in FIG. 23. This was also observed for siRNA ID 01009v17 in similar experiments carried out comparing the TSC2 silencing effect of such siRNA alone or in combination with either siRNA ID 11129v5 or siRNA ID 11129v17 (data not shown). Both individual and combined treatment produced significant downregulation of TSC2 (more than 75%) in C2C12 cells (FIG. 23). Indeed, combination of siRNA ID 01009v17 with siRNA ID 11129v5 showed higher silencing effects than siRNA ID 01009v17 alone at 72h after transfection.

### REFERENCES

Angaji S.A, Hedayati S.S, Poor R.H, et al. "Application of RNA interference in treating human diseases" J Genet. 2010. Vol. 89. 4. 527-37.
Bramsen J.B., Laursen M.B., Nielsen A.F., et al. (2009). "A large-scale chemical modification screen identifies design rules to generate siRNAs with high activity, high stability and low toxicity" Nucleic Acids Res 2009 Vol. 37, Issue 9, 2867-81.
Campochiaro PA. (2006). "Potential applications for RNAi to probe pathogenesis and develop new treatments for ocular disorders" . Gene Ther. 2006; 13 (6): 559-62.
Cerutti L., Mian, N. et al. (2000). "Domains in gene silencing and cell differentiation proteins: the novel PAZ domain and redefinition of the Piwi domain." Trends Biochem Sci 25(10): 481-2.
Chang C.I, Kim H.A, Dua P, et al. (2011) "Structural Diversity Repertoire of Gene Silencing Small Interfering RNAs" Nucleic Acid Ther. 2011. Vol. 21. 3. 125-31.
Chawla H, Vohra V. Retinal Dystrophies. [Updated 2023 Mar 16]. In: StatPearls [Internet]. Treasure Island (FL): StatPearls Publishing; 2023 Jan-. Available from: https://www.ncbi.nlm.nih.gov/books/NBK564379/
Chong RH, Gonzalez-Gonzalez E, et al (2013). "Gene silencing following siRNA delivery to skin via coated steel microneedles: In vitro and in vivo proof-of-concept" . J Control Release 2013, 166:211-9.
Collins R.E. and Cheng X. (2005). "Structural domains in RNAi." FEBS Lett 579(26): 5841-9.
Del Amo EM and Urtti A (2008). "Current and future ophthalmic drug delivery systems. A shift to the posterior segment". Drug Discov Today 2008, 13: 135-143.
Deleavey G.F and Damha M.J. (2012) "Designing chemically modified oligonucleotides for targeted gene silencing". Chem Biol. 2012 Vol.19.8. 937-54.
Di Nardo A, Wertz MH, Kwiatkowski E, Tsai PT, Leech JD, Greene-Colozzi E, Goto J, Dilsiz P, Talos DM, Clish CB, Kwiatkowski DJ, Sahin M (2014). Neuronal Tsc1/2 complex controls autophagy through AMPK-dependent regulation of ULK1. Hum Mol Genet. 2014 Jul 15;23(14):3865-74. doi: 10.1093/hmg/ddu101.
Doench J.G., Sharp P.A. (2004) "Specificity of microRNA target selection in translational repression" Genes Dev. 18, 504-511.
Dosta P., Ramos V., Borrós S. (2018). "Stable and efficient generation of poly(β-aminoester)s for RNAi delivery." Mol Systems Design Eng 2018, 3, 677-689.
Edelhauser HF, Ro e-Rendleman CL, Robinson MR, Dawson DG, et al (2010). "Ophthalmic drug delivery systems for the treatment of retinal diseases: basic research to clinical applications". Invest Ophthalmol Vis Sci 2010, 51:5403-5420.
Elbashir, S. M., W. Lendeckel, et al. (2001). "RNA interference is mediated by 21- and 22-nucleotide RNAs." Genes Dev 15(2): 188-200.
Fire, A., S. Xu, et al. "Potent and specific genetic interference by double-stranded RNA in Caenorhabditis elegans." Nature. 1998 391(6669): 806-11.
Godinho B.M.D.C., Khvorova A. (2019). The era of RNA interference medicines: the clinical landscape of synthetic gene silencing drugs. Saúde & Tecnologia, 21, p. 05-17.
Guzman-Aranguez A, Loma P and Pintor J (2013). "Small-interfering RNAs ( siRNAs ) as a promising tool for ocular therapy". British Journal of Pharmacology 2013, 170, 730-747.
Hutvagner, G. and Zamore P.D. (2002). "A microRNA in a multiple-turnover RNAi enzyme complex." Science 297(5589): 2056-60.
Kigasawa K, Kajimoto K, et al (2010). "Noninvasive delivery of siRNA into the epidermis by iontophoresis using an atopic dermatitis-like model rat". Int J Pharm 2010, 383:157-60.
Kim DH, Behlke MA, Rose SD, et al (2005). "Synthetic dsRNA Dicer substrates enhance RNAi potency and efficacy". Nat Biotechnol 2005, Vol. 23, Issue: 2, 222-6.
Kornbrust D, Cavagnaro J, Levin A, et al. "Oligo safety working group exaggerated pharmacology subcommittee consensus document" Nucleic Acid Ther 2013 Vol. 23, 1, Pag: 21-8.
Leachman SA, Hickerson RP, et al (2010). "First-in-human mutation- targeted siRNA phase Ib trial of an inherited skin disorder". Mol Ther 2010, 18:442-6.
Lewis, B.P., Shih I. et al. (2003) "Prediction of mammalian micro RNA targets" Cell 115:787-798.
Liu J., Carmell M.A., et al. (2004). "Argonaute2 is the catalytic engine of mammalian RNAi." Science 305(5689): 1437-41.
Ma, J. B., Yuan Y.R., et al. (2005). "Structural basis for 5'-end-specific recognition of guide RNA by the A. fulgidus Piwi protein." Nature 434(7033): 666-70.
Maniatis, T., et al., "Molecular Cloning: A Laboratory Manual". Cold Spring Harbor Laboratory, 1982, at pages 387-389.
Nakai N, Kishida T, et al (2007). "Therapeutic RNA interference of malignant melanoma by electrotransfer of small interfering RNA targeting Mitf". Gene Ther 2007, 14:357-65.
Nash BM, Wright DC, Grigg JR, Bennetts B, Jamieson RV (2015). Retinal dystrophies, genomic applications in diagnosis and prospects for therapy. Transl Pediatr. 2015;4(2):139-163. doi:10.3978/j.issn.2224-4336.2015.04.03.
Nykanen A., Haley B., et al. (2001). "ATP requirements and small interfering RNA structure in the RNA interference pathway." Cell 107(3): 309-21.
Orban, T. I. and Izaurralde E. (2005). "Decay of mRNAs targeted by RISC requires XRN1, the Ski complex, and the exosome." Rna 11(4): 459-69.
Parrish, S., J. Fleenor, et al. (2000) "Functional anatomy of a dsRNA trigger: differential requirement for the two trigger strands in RNA interference." Mol Cell. 2000 6(5): 1077-87.
Rand, T. A., Petersen S., et al. (2005). "Argonaute2 cleaves the anti-guide strand of siRNA during RISC activation." Cell 123(4): 621-9.
Sanghvi Y.S. (2011) "A status update of modified oligonucleotides for chemotherapeutics applications" Curr Protoc Nucleic Acid Chem. 2011 Vol. 4. 4 1 1-22.
Song, J. J., Smith S.K., et al. (2004). "Crystal structure of Argonaute and its implications for RISC slicer activity." Science 305(5689): 1434-7.
Talib M, Boon CJF (2020). Retinal Dystrophies and the Road to Treatment: Clinical Requirements and Considerations. Asia Pac J Ophthalmol (Phila). 2020 May-Jun;9(3):159-179. doi: 10. 1097/APO.0000000000000290.
Villarejo-Zori B., Jiménez-Loygorri J.I., Zapata-Muñoz J., Bell K. and Boya P. (2021). "New insights into the role of autophagy in retinal and eye diseases", Molecular Aspects of Medicine 82, 101038. doi:10.1016/j.mam.2021.101038.
Walton S.P, Wu M, Gredell J.A and Chan C. (2010) "Designing highly active siRNAs for therapeutic applications" FEBS J. 2010. Vol. 277. 23. 4806-13.

### Clauses

**1.** An RNA interfering (RNAi) oligonucleotide for use in the treatment of a retinal dystrophy, which reduces the expression and/or activity of the tuberous sclerosis complex 2 (TSC2) gene in a cell, preferably in ocular cells, and comprises or consists of a siRNA molecule that specifically targets any of the nucleotide sequences SEQ ID NO. 41 to SEQ ID NO. 64 (preferably any of SEQ ID NO. 41 to SEQ ID NO. 49, and more preferably any of sequences SEQ ID NO. 41 to SEQ ID NO. 45 and SEQ ID NO. 47 to SEQ ID NO. 49) of the TSC2 gene.
**2.** The RNAi oligonucleotide for use according to clause 1, wherein the retinal dystrophy is an inherited retinal dystrophy or a retinal dystrophy characterized by dysfunction or progressive loss of photoreceptors, and preferably is an inherited retinal dystrophy.
**3.** The RNAi oligonucleotide for use according to clause 1 or clause 2, wherein the retinal dystrophy is an inherited retinal dystrophy, preferably selected from retinitis pigmentosa, Leber's congenital amaurosis or Alström syndrome.
**4.** The RNAi oligonucleotide for use according to any of clauses 1 to 3, wherein the siRNA molecule comprises an antisense strand having a nucleotide sequence of at least 18 nucleotides or at least 19 nucleotides which is substantially complementary to the targeted nucleotide sequence of the gene, and a sense strand (of at least 14, 15, 16, 17, 18 or 19 nucleotides) complementary to the antisense strand.
**5.** The RNAi oligonucleotide for use according to any of clauses 1 to 4, which comprises or consists of an siRNA molecule specifically targeting any of the nucleotide sequences SEQ ID NO. 41 or SEQ ID NO. 42 of the TSC2 gene.
**6.** The RNAi oligonucleotide for use according to any of clauses 1 to 5, wherein the siRNA molecule targeting the TSC2 gene comprises an antisense strand having at least the first 19 nucleotides from the 5'-end of the sequence SEQ ID NO. 105 or SEQ ID NO. 106, and a sense strand (of at least 14, 15, 16, 17, 18 or 19 nucleotides) complementary to the antisense strand.
**7.** The RNAi oligonucleotide for use according to clause 1 to 6, which also reduce the expression and/or activity of the tuberous sclerosis complex 1 (TSC1) gene.
**8.** The RNAi oligonucleotide for use according to any of clauses 1 to 7, which further comprises an siRNA molecule targeting any of the nucleotide sequences SEQ ID NO. 1 to SEQ ID NO. 40 (preferably any of SEQ ID NO. 1 to SEQ ID NO. 10, and more preferably any of sequences SEQ ID NO. 1 to SEQ ID NO. 8) of the TSC1 gene.
**9.** The RNAi oligonucleotide for use according to any of clauses 1 to 7, which further comprises an siRNA molecule targeting the nucleotide sequence SEQ ID NO. 2, SEQ ID NO. 1 or SEQ ID NO. 7 of the TSC1 gene.
**10.** The RNAi oligonucleotide for use according to any of clauses 1 to 9, which comprises an siRNA molecule targeting any of the nucleotide sequences SEQ ID NO. 41 or SEQ ID NO. 42 of the TSC2 gene, and an siRNA molecule targeting the nucleotide sequence SEQ ID NO. 2 or SEQ ID NO. 1 of the TSC1 gene, and preferably comprising an siRNA molecule targeting the nucleotide sequences SEQ ID NO. 41 of the TSC2 gene and an siRNA molecule targeting the nucleotide sequence SEQ ID NO. 2 of the TSC1 gene.
**11.** The RNAi oligonucleotide for use according to any of clauses 8 to 10, wherein the siRNA molecule targeting the TSC1 gene comprises an antisense strand having at least the first 19 nucleotides from the 5'-end of the sequence SEQ ID NO. 66 or SEQ ID NO. 65.
**12.** The RNAi oligonucleotide for use according to any of clauses 8 to 11, wherein the siRNA molecules targeting the TSC2 gene and the TSC1 gene are administered jointly or separately.
**13.** The RNAi oligonucleotide for use according to any of clauses 8 to 12, wherein the siRNA molecules targeting the TSC2 gene and the TSC1 gene are connected by one or more moieties selected from a linker, a spacer or a branching point.
**14.** The RNAi oligonucleotide for use according to any of clauses 1 to 13, wherein the siRNA molecule or molecules are formulated in a pharmaceutical composition for local delivery to the eye.
**15.** The RNAi oligonucleotide for use according to any of clauses 1 to 14, wherein the siRNA molecule or molecules are formulated in a pharmaceutical composition for topical or intravitreal delivery to the eye.
**16.** The RNAi oligonucleotide for use according to any of clauses 1 to 15, wherein the comprised siRNA molecule or molecules are siRNA comprising a double-stranded region of 15, 16, 17, 18 or 19 nucleotides, and preferably being siRNA comprising a double-stranded region of 19 nucleotides which optionally can be blunt-ended.
**17.** The RNAi oligonucleotide for use according to any of clauses 1 to 16, wherein at least one nucleotide of the siRNA molecule or molecules comprises a chemical modification.
**18.** The RNAi oligonucleotide for use according to clause 17, wherein the chemical modification is selected from: 2'-O-methyl modification, 2'-fluoro modification, 2'-O-methyl-5'-vinylphosphonate modification, 5'-O-methyl modification, substitution of uracil with 5'-methyluridine, substitution of cytosine with 5-methylcytosine, substitution of uridine or cytosine with 2'-deoxyinosine, substitution of uracyl ribose nucleotide with deoxythymidine nucleotide, substitution of ribose nucleotide with deoxyribose nucleotide, substitution of ribose nucleotide with unlocked nucleic acid (UNA) monomer, substitution of ribose nucleotide with glycol nucleic acid (GNA) monomer, substitution of ribose nucleotide with inverted abasic deoxyribose, introduction of phosphorothioate modified nucleotides, and combinations thereof.
**19.** The RNAi oligonucleotide for use according to clause 17 or clause 18, wherein the chemical modification is on the sense strand, the antisense strand or on both strands of the siRNA molecule.
**20.** The RNAi oligonucleotide for use according to any of clauses 17 to 19, wherein the siRNA molecule targeting the TSC2 gene comprises an antisense strand having at least the first 19 nucleotides from the 5'-end of any of the sequences from the group consisting of SEQ ID NO. 1022 - SEQ ID NO. 1 097, SEQ ID NO. 1235, SEQ ID NO. 1236, SEQ ID NO. 1239, SEQ ID NO. 1240 and SEQ ID NO. 1245 - SEQ ID NO. 1265 (and preferably from the group consisting of SEQ ID NO. 1022 to SEQ ID NO. 1026, SEQ ID NO. 1035 to SEQ ID NO. 1038 and SEQ ID NO. 1040), and a sense strand (of at least 14, 15, 16, 17, 18 or 19 nucleotides) complementary to the antisense strand, which preferably has at least 14, 15, 16, 17, 18 or 19 nucleotides from any of the nucleotide sequences from the group consisting of SEQ ID NO. 1159 - SEQ ID NO. 1234, SEQ ID NO. 1237, SEQ ID NO. 1238, SEQ ID NO. 1241, SEQ ID NO. 1242 and SEQ ID NO. 1266 - SEQ ID NO. 1286 (and preferably from the group consisting of SEQ ID NO. 1159 to SEQ ID NO. 1163, SEQ ID NO. 1172 to SEQ ID NO. 1175 and SEQ ID NO. 1177).
**21.** The RNAi oligonucleotide for use according to any of clauses 17 to 20, which further comprises an siRNA molecule targeting the TSC1 gene comprising an antisense strand having at least the first 19 nucleotides from the 5'-end of any of the sequences from the group consisting of SEQ ID NO. 961 - SEQ ID NO. 1021 and SEQ ID NO. 1243 (and preferably from SEQ ID NO. 984 or SEQ ID NO. 996), and a sense strand (of at least 14, 15, 16, 17, 18 or 19 nucleotides) complementary to the antisense strand, which preferably has at least 14, 15, 16, 17, 18 or 19 nucleotides from any of the nucleotide sequences from the group consisting of SEQ ID NO. 1098 - SEQ ID NO. 1158 and SEQ ID NO. 1244 (and preferably from SEQ ID NO. 1121 or SEQ ID NO. 1133).
**22.** An RNA interfering (RNAi) oligonucleotide which reduces the expression and/or activity of the tuberous sclerosis complex 2 (TSC2) gene and the tuberous sclerosis complex 1 (TSC1) gene for use in the treatment of a retinal dystrophy, which comprises or consists of at least an siRNA molecule targeting any of the nucleotide sequences selected from any of the group consisting of SEQ ID NO. 41, SEQ ID NO. 42, SEQ ID NO. 3 and SEQ ID NO. 8, or any combination of said siRNA molecules.
**23.** The RNAi oligonucleotide for use according to clause 22, wherein the retinal dystrophy is characterized by dysfunction or progressive loss of photoreceptors, and preferably is an inherited retinal dystrophy.
**24.** The RNAi oligonucleotide for use according to clause 22 or clause 23, wherein the retinal dystrophy is an inherited retinal dystrophy, preferably selected from retinitis pigmentosa, Leber's congenital amaurosis or Alström syndrome.
**25.** The RNAi oligonucleotide for use according to any of clauses 22 to 24, wherein the siRNA molecule is an siRNA which comprises an antisense strand having a nucleotide sequence of at least 19 nucleotides which is substantially complementary to the targeted nucleotide sequence of the gene, and a sense strand complementary to the antisense strand.
**26.** The RNAi oligonucleotide for use according to clause 25, wherein the siRNA compound targeting the TSC2 gene comprises an antisense strand having at least the first 19 nucleotides from the 5'-end of the sequence SEQ ID NO. 105 or SEQ ID NO. 106, and the siRNA molecule targeting the TSC1 gene comprises an antisense strand having at least the first 19 nucleotides from the 5'-end of the sequence SEQ ID NO. 67 or SEQ ID NO. 72.
**27.** An RNA interfering (RNAi) oligonucleotide which reduces the expression and/or activity of the tuberous sclerosis complex 2 (TSC2) gene and the tuberous sclerosis complex 1 (TSC1) gene in a cell, preferably in an ocular cell, which comprise:
   - an siRNA molecule specifically targeting any of the nucleotide sequences SEQ ID NO. 41, SEQ ID NO. 42, SEQ ID NO. 3 or SEQ ID NO. 8, or
   - a combination comprising an siRNA molecule specifically targeting any of the nucleotide sequences SEQ ID NO. 41 or SEQ ID NO. 42, and a siRNA molecule specifically targeting any of the nucleotide sequences SEQ ID NO. 2, SEQ ID NO. 1 or SEQ ID NO. 7.
**28.** An RNA interfering (RNAi) oligonucleotide according to clause 27, which comprises an siRNA molecule targeting the nucleotide sequences SEQ ID NO. 41 or SEQ ID NO. 42, or comprises a combination comprising any of them and an siRNA molecule targeting the nucleotide sequence SEQ ID NO. 2.
**29.** An RNA interfering (RNAi) oligonucleotide according to clause 27 or clause 28, wherein the siRNA molecule comprises an antisense strand having a nucleotide sequence of at least 19 nucleotides which is substantially complementary to the targeted nucleotide sequence of the gene, and a sense strand complementary to the antisense strand, and wherein the antisense strand forms a double-stranded structure with the sense strand which is blunt-ended or which has of from 1 to 6 unpaired nucleotides comprised in any or both of its 5'- and 3'-ends of the antisense strand sequence.
**30.** An RNA interfering (RNAi) oligonucleotide according to any of clauses 27 to 29, which comprises an siRNA molecule having an antisense strand comprising or consisting of sequence SEQ ID NO. 1 05 or SEQ ID NO. 106, or comprises an siRNA molecule having an antisense strand comprising or consisting of sequence SEQ ID NO. 105 or SEQ ID NO. 106 and an siRNA molecule having an antisense strand comprising or consisting of SEQ ID NO. 66.
**31.** An RNA interfering (RNAi) oligonucleotide according to any of clauses 27 to 30, wherein at least one nucleotide of the siRNA molecule or compounds comprises a chemical modification.
**32.** The RNAi oligonucleotide for use according to clause 31, wherein the chemical modification is selected from: 2'-O-methyl modification, 2'-fluoro modification, 2'-O-methyl-5'-vinylphosphonate modification, 5'-O-methyl modification, substitution of uracil with 5'-methyluridine, substitution of cytosine with 5-methylcytosine, substitution of uridine or cytosine with 2'-deoxyinosine, substitution of uracyl ribose nucleotide with deoxythymidine nucleotide, substitution of ribose nucleotide with deoxyribose nucleotide, substitution of ribose nucleotide with unlocked nucleic acid (UNA) monomer, substitution of ribose nucleotide with glycol nucleic acid (GNA) monomer, substitution of ribose nucleotide with inverted abasic deoxyribose, introduction of phosphorothioate modified nucleotides, and combinations thereof.
**33.** The RNAi oligonucleotide for use according to clause 31 or clause 32, wherein the chemical modification is on the sense strand, the antisense strand or on both strands of the siRNA molecule.
**34.** Use of an RNAi oligonucleotide according to any preceding clause in the manufacture of a medicament for the treatment of a retinal dystrophy.
**35.** Use of an RNAi oligonucleotide according to clause 34 wherein the retinal dystrophy is characterized by dysfunction or progressive loss of photoreceptors, and preferably is an inherited retinal dystrophy.
**36.** Use of an RNAi oligonucleotide according to clause 34 or clause 35, wherein the retinal dystrophy is an inherited retinal dystrophy, preferably selected from retinitis pigmentosa, Leber's congenital amaurosis or Alström syndrome.
**37.** A pharmaceutical composition wherein said composition comprises at least an RNAi oligonucleotide described in any of clauses 1 to 33, and optionally a pharmaceutically acceptable carrier or diluent.
**38.** A pharmaceutical composition according to clause 37 which is a composition for local delivery to the eye.
**39.** A pharmaceutical composition according to clause 37 or clause 38, which is an eyedrop composition or a composition in solution for intravitreal injection.
**40.** A method of treatment of a retinal dystrophy the method comprising administering to a patient in need thereof an RNAi oligonucleotide according to any of clauses 1 to 33, or a pharmaceutical composition according to any of clauses 37 to 39.
**41.** The method of treatment according to clause 40 wherein the retinal dystrophy is characterized by dysfunction or progressive loss of photoreceptors, and preferably is an inherited retinal dystrophy.
**42.** The method of treatment according to clause 40 or clause 41, wherein the retinal dystrophy is an inherited retinal dystrophy, preferably selected from retinitis pigmentosa, Leber's congenital amaurosis or Alström syndrome.

## Claims

1. An RNA interfering (RNAi) oligonucleotide which reduces the expression and/or activity of the tuberous sclerosis complex 2 (TSC2) gene for use in the treatment of a retinal dystrophy, the RNAi oligonucleotide comprising an siRNA molecule specifically targeting a nucleotide sequence selected from SEQ ID NO. 41 to SEQ ID NO. 49 of the TSC2 gene.

2. The RNAi oligonucleotide for use according to claim 1, wherein the retinal dystrophy is **characterized by** dysfunction or progressive loss of photoreceptors, and preferably is an inherited retinal dystrophy, optionally selected from retinitis pigmentosa, Leber's congenital amaurosis or Alström syndrome.

3. The RNAi oligonucleotide for use according to claim 1 or claim 2, wherein the siRNA molecule comprises an antisense strand having a nucleotide sequence of at least 19 nucleotides which is substantially complementary to the targeted nucleotide sequence of the gene, and a sense strand complementary to the antisense strand.

4. The RNAi oligonucleotide for use according to any of claims 1 to 3, which comprises an siRNA molecule specifically targeting a nucleotide sequence selected from SEQ ID NO. 41 or SEQ ID NO. 42 of the TSC2 gene.

5. The RNAi oligonucleotide for use according to any of claims 1 to 4, wherein the siRNA molecule targeting the TSC2 gene comprises an antisense strand having at least the first 19 nucleotides from the 5'-end of the sequence SEQ ID NO. 105 or SEQ ID NO. 106, and a sense strand complementary to the antisense strand.

6. The RNAi oligonucleotide for use according to claim 1 to 5, which also reduces the expression and/or activity of the tuberous sclerosis complex 1 (TSC1) gene.

7. The RNAi oligonucleotide for use according to any of claims 1 to 6, which further comprises an siRNA molecule specifically targeting a nucleotide sequence selected from SEQ ID NO. 1 to SEQ ID NO. 40, preferably a nucleotide sequence selected from SEQ ID NO. 1 to SEQ ID NO. 8, of the TSC1 gene.

8. The RNAi oligonucleotide for use according to any of claims 1 to 7, which further comprises an siRNA molecule specifically targeting the nucleotide sequence SEQ ID NO. 2, SEQ ID NO. 1 or SEQ ID NO. 7 of the TSC1 gene.

9. The RNAi oligonucleotide for use according to any of claims 1 to 8, which comprises an siRNA molecule targeting any of the nucleotide sequences SEQ ID NO. 41 or SEQ ID NO. 42 of the TSC2 gene, and an siRNA molecule targeting the nucleotide sequence SEQ ID NO. 2 or SEQ ID NO. 1 of the TSC1 gene.

10. The RNAi oligonucleotide for use according to any of claims 7 to 9, wherein the siRNA molecule targeting the TSC1 gene comprises an antisense strand having at least the first 19 nucleotides from the 5'-end of the sequence SEQ ID NO. 66 or SEQ ID NO. 65.

11. The RNAi oligonucleotide for use according to any of claims 7 to 10, wherein the siRNA molecules targeting the TSC2 gene and the TSC1 gene are connected by one or more moieties selected from a linker, a spacer or a branching point.

12. The RNAi oligonucleotide for use according to any of claims 7 to 11, wherein the siRNA molecules targeting the TSC2 gene and the TSC1 gene are administered jointly or separately.

13. The RNAi oligonucleotide for use according to any of claims 1 to 12, wherein the siRNA molecule or molecules are formulated in a pharmaceutical composition for local delivery to the eye; preferably for topical or intravitreal delivery to the eye.

14. The RNAi oligonucleotide for use according to any of claims 1 to 13, wherein the comprised siRNA molecule or molecules are siRNA comprising a double-stranded region of 15, 16, 17, 18 or 19 nucleotides, preferably being siRNA comprising a double-stranded region of 19 nucleotides which optionally is blunt-ended.

15. The RNAi oligonucleotide for use according to any of claims 1 to 14, wherein at least one nucleotide of the siRNA molecule or molecules comprises a chemical modification, preferably wherein the chemical modification is selected from: 2'-O-methyl modification, 2'-fluoro modification, 2'-O-methyl-5'-vinylphosphonate modification, 5'-O-methyl modification, substitution of uracil with 5'-methyluridine, substitution of cytosine with 5-methylcytosine, substitution of uridine or cytosine with 2'-deoxyinosine, substitution of uracyl ribose nucleotide with deoxythymidine nucleotide, substitution of ribose nucleotide with deoxyribose nucleotide, substitution of ribose nucleotide with unlocked nucleic acid (UNA) monomer, substitution of ribose nucleotide with glycol nucleic acid (GNA) monomer, substitution of ribose nucleotide with inverted abasic deoxyribose, introduction of phosphorothioate modified nucleotides, and combinations thereof; and optionally wherein the chemical modification is on the sense strand, the antisense strand or on both strands of the siRNA molecule.

16. The RNAi oligonucleotide for use according to claim 15, wherein the siRNA molecule targeting the TSC2 gene comprises an antisense strand having at least the first 19 nucleotides from the 5'-end of any of the nucleotide sequences selected from the group consisting of SEQ ID NO. 1022 to SEQ ID NO. 1026, SEQ ID NO. 1035 to SEQ ID NO. 1038 and SEQ ID NO. 1040, and a sense strand complementary to the antisense strand.

17. The RNAi oligonucleotide for use according to any of claims 15 or 16, which further comprises an siRNA molecule targeting the TSC1 gene comprising an antisense strand having at least the first 19 nucleotides from the 5'-end of any of the sequences SEQ ID NO. 984 or SEQ ID NO. 996, and a sense strand complementary to the antisense strand.

18. An RNA interfering (RNAi) oligonucleotide which reduces the expression and/or activity of the tuberous sclerosis complex 2 (TSC2) gene and the tuberous sclerosis complex 1 (TSC1) gene for use in the treatment of a retinal dystrophy, the RNAi oligonucleotide comprising or consisting of an siRNA molecule targeting any of the nucleotide sequences selected from the group consisting of SEQ ID NO. 41, SEQ ID NO. 42, SEQ ID NO. 3 and SEQ ID NO. 8, or any combination of said siRNA molecules.

19. The RNAi oligonucleotide for use according to claim 18, wherein the retinal dystrophy is **characterized by** dysfunction or progressive loss of photoreceptors, and preferably is an inherited retinal dystrophy, optionally selected from retinitis pigmentosa, Leber's congenital amaurosis or Alström syndrome.

20. The RNAi oligonucleotide for use according to any of claims 18 or 19, wherein the siRNA molecule is a siRNA which comprises an antisense strand having a nucleotide sequence of at least 19 nucleotides which is substantially complementary to the targeted nucleotide sequence of the gene, and a sense strand complementary to the antisense strand.

21. The RNAi oligonucleotide for use according to claim 20, wherein the siRNA molecule targeting the TSC2 gene comprises an antisense strand having at least the first 19 nucleotides from the 5'-end of the sequence SEQ ID NO. 105 or SEQ ID NO. 106, and the siRNA molecule targeting the TSC1 gene comprises an antisense strand having at least the first 19 nucleotides from the 5'-end of the sequence SEQ ID NO. 67 or SEQ ID NO. 72.

22. An RNA interfering (RNAi) oligonucleotide which reduces the expression and/or activity of the tuberous sclerosis complex 2 (TSC2) gene and the tuberous sclerosis complex 1 (TSC1) gene in a cell, the RNAi oligonucleotide comprising:
- an siRNA molecule specifically targeting any of the nucleotide sequences SEQ ID NO. 41, SEQ ID NO. 42, SEQ ID NO. 3 or SEQ ID NO. 8, or
- a combination comprising an siRNA molecule specifically targeting any of the nucleotide sequences SEQ ID NO. 41 or SEQ ID NO. 42, and a siRNA molecule targeting any of the nucleotide sequences SEQ ID NO. 2, SEQ ID NO. 1 or SEQ ID NO. 7.

23. An RNA interfering (RNAi) oligonucleotide according to claim 22, which comprises an siRNA molecule targeting the nucleotide sequences SEQ ID NO. 41 or SEQ ID NO. 42, and optionally further comprises an siRNA molecule targeting the nucleotide sequence SEQ ID NO. 2.

24. An RNA interfering (RNAi) oligonucleotide according to claim 22 or claim 23, wherein the siRNA molecule comprises an antisense strand having a nucleotide sequence of at least 19 nucleotides which is substantially complementary to the targeted nucleotide sequence of the gene, and a sense strand complementary to the antisense strand, and wherein the antisense strand forms a double-stranded structure with the sense strand which is blunt-ended or which has of from 1 to 6 unpaired nucleotides comprised in either or both of the 5'- and 3'-ends of the antisense strand sequence.

25. An RNA interfering (RNAi) oligonucleotide according to any of claims 22 to 24, which comprises an siRNA molecule having an antisense strand comprising or consisting of sequence SEQ ID NO. 105 or SEQ ID NO. 106, and optionally further comprises an siRNA molecule having an antisense strand comprising or consisting of SEQ ID NO. 66.

26. An RNA interfering (RNAi) oligonucleotide according to any of claims 22 to 25, wherein at least one nucleotide of the siRNA molecule or compounds comprises a chemical modification, preferably wherein the chemical modification is selected from: 2'-O-methyl modification, 2'-fluoro modification, 2'-O-methyl-5'-vinylphosphonate modification, 5'-O-methyl modification, substitution of uracil with 5'-methyluridine, substitution of cytosine with 5-methylcytosine, substitution of uridine or cytosine with 2'-deoxyinosine, substitution of uracyl ribose nucleotide with deoxythymidine nucleotide, substitution of ribose nucleotide with deoxyribose nucleotide, substitution of ribose nucleotide with unlocked nucleic acid (UNA) monomer, substitution of ribose nucleotide with glycol nucleic acid (GNA) monomer, substitution of ribose nucleotide with inverted abasic deoxyribose, introduction of phosphorothioate modified nucleotides, and combinations thereof; and optionally wherein the chemical modification is on the sense strand, the antisense strand or on both strands of the siRNA molecule.

27. A pharmaceutical composition wherein said composition comprises at least an RNAi oligonucleotide described in any of claims 1 to 26, preferably being a composition for local delivery to the eye, and preferably the composition is an eyedrop composition or a composition in solution for intravitreal injection.
